(19) Europäisches Patentamt
European Patent Office
Office européen des brevets

(11) Publication number: **0 245 950 B1**

(12) **EUROPEAN PATENT SPECIFICATION**

(45) Date of publication of patent specification: **17.06.92**

(51) Int. Cl.[5]: **A01N 37/28**, C07C 243/12

(21) Application number: **87303092.8**

(22) Date of filing: **09.04.87**

(54) **Insecticidal n-substituted-n' - substituted-n, n'-diacylhydrazines.**

(30) Priority: **01.05.86 US 858482**

(43) Date of publication of application:
**19.11.87 Bulletin 87/47**

(45) Publication of the grant of the patent:
**17.06.92 Bulletin 92/25**

(84) Designated Contracting States:
**AT BE CH DE ES FR GB GR IT LI LU NL SE**

(56) References cited:

**BERICHTE DER DEUTSCHEN CHEMISCHEN GESELLSCHAFT, vol. 1, 1923, pages 954-963, Verlag Chemie GmbH, Leipzig, DD; K.A. TAIPALE: "Die katalytische Reduktion der aliphatischen Azine, II. Mitteilung: Reduktion des Dimethylketazins und des Isobutyraldazins in Gegenwart von Eisessig"**

**JOURNAL OF THE CHEMICAL SOCIETY, CHEMICAL COMMUNICATIONS, no. 13, 9th July 1969, pages 685,706-708; J.R. FLETCHER et al.: "Conformational changes in Tetraalkylhydrazines"**

**Austr. J. Chem. 25, 523-529 (1972)**

**Help. Chim. Acta 61, 1477-1510 (1978)**

**Ann. cl. Ch. 590, 1-36 (1954)**

**J. Econ. Ent. 39, 416-417 (1946)**

(73) Proprietor: **ROHM AND HAAS COMPANY**
**Independence Mall West**
**Philadelphia Pennsylvania 19105(US)**

(72) Inventor: **Murphy, Raymond August**
**Forge Gate Apartments Apt. 38-B-1**
**Lansdale Pennsylvania 19446(US)**
Inventor: **Hsu, Adam Chi-Tung**
**1686 Heebner Way**
**Lansdale Pennsylvania 19446(US)**

(74) Representative: **Angell, David Whilton et al**
**ROHM AND HAAS (UK) LTD. European Operations Patent Department Lennig House 2**
**Mason's Avenue**
**Croydon CR9 3NB(GB)**

**EP 0 245 950 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention is concerned with certain N-substituted-N'-substituted-N,N'-diacylhydrazines which are useful as insecticides, compositions containing those compounds and methods of their use.

The search for compounds which have a combination of excellent insecticidal activity and low undesirable toxicity is a continuing one because of factors such as the desire for compounds exhibiting greater activity, better selectivity, low undesirable environmental impact, low production cost and effectiveness against insects resistant to many known insecticides.

We have now found certain novel compounds which are suitable for controlling plant-destructive insects in crops of cultivated plants, ornamentals and forestry.

Certain hydrazine derivatives have been disclosed in the literature.

In 25 Aust. J. Chem., 523-529 (1972), several N,N'-dibenzoylhydrazine derivatives are disclosed in which one or both nitrogen atoms are alkylated or phenylated.

In 61 Helv. Chim. Acta, 1477-1510 (1978), several N,N'-dibenzoylhydrazine derivatives are disclosed.

In 44 J.A.C.S., 2556-2567 (1922), isopropyl hydrazine $(CH_3)_2CH-NH-NH_2$, symmetrical diisopropyl hydrazine, dibenzoylisopropyl hydrazine and certain derivatives are disclosed.

In 44 J.A.C.S., 1557-1564 (1972), isopropyl, menthyl and bornyl semicarbazides are disclosed.

In 48 J.A.C.S., 1030-1035 (1926), symmetrical di-methylphenylmethyl hydrazine and certain related compounds including 1,2-bis-methylphenylmethyl-4-phenyl-semicarbazide are disclosed.

In 27 Bull. Chem. Soc. Japan; 624-627 (1954), certain hydrazine derivatives including alpha-betadibenzoylphenyl hydrazine are disclosed.

In J. Chem. Soc. (C), 1531-1536 (1966), N,N'-dibenzoylphenyl hydrazine and N-acetyl-N'-benzoyl-p-nitrophenyl hydrazine are disclosed.

In 56B Chem. Berichte, 954-962 (1923), symmetrical di-isopropyl hydrazines, symmetrical diisobutyl and certain derivatives including N,N'-diisobutyldibenzoyl hydrazine are disclosed.

In 590 Annalen der Chemie, 1-36 (1954), certain N,N'-dibenzoyl hydrazine derivatives are disclosed including N'-methyl- and N'-(2-phenyl)-isopropyl-N,N'-dibenzoyl hydrazine.

In J. Chem. Soc., 4191-4198 (1952), N,N'-di-n-propyl hydrazine and the bis-3,5-dinitrobenzoyl derivatives are disclosed.

In 32 Zhur. Obs. Khim., 2806-2809 (1962), N'-2,4-methyl-2,4-pentadiene-N,N'-dibenzoyl hydrazine is disclosed.

In 17 Acta. Chim. Scand., 95-102 (1963), 2-benzoyl-thiobenzhydrazide ($C_6H_5$-CS-NHNH-CO-$C_6H_5$) and certain hydrazone and hydrazine derivatives are disclosed including 1,2-dibenzoyl-benzyl hydrazine.

In 25 Zhur. Obs. Khim, 1719-1723 (1955), N,N'-bis-cyclohexyl hydrazine and N,N'-dibenzoylcyclohexyl hydrazine are disclosed.

In J. Chem. Soc., 4793-4800 (1964), certain dibenzoyl hydrazine derivatives are disclosed including tribenzoyl hydrazine and N,N'-dibenzoylcyclohexyl hydrazine.

In 36 J. Prakt. Chem., 197-201 (1967), certain dibenzoyl hydrazine derivatives including N'-ethyl-; N'-n-propyl-; N'-isobutyl-; N'-neopentyl-; N'-n-heptyl-; and N'-cyclohexylmethyl-N,N'-dibenzoyl hydrazines are disclosed.

In 26 J.O.C., 4336-4340 (1961), N'-t-butyl-N,N'-di-(t-butoxycarbonyl)hydrazide is disclosed.

In 41 J.O.C., 3763-3765 (1976), N'-t-butyl-N-(phenylmethoxycarbonyl)-N'-(chlorocarbonyl)hydrazide is disclosed.

In 94 J.A.C.S., 7406-7416 (1972), N'-t-butyl-N,N'-dimethoxycarbonylhydrazide is disclosed.

In 43 J.O.C., 808-815 (1978), N'-t-butyl-N-ethoxycarbonyl-N'-phenylaminocarbonylhydrazide and N'-t-butyl-N-ethoxycarbonyl-N'-methylaminocarbonylhydrazide are disclosed.

In none of the above references is any biological activity disclosed for the indicated compounds.

In 39 J. Econ. Ent., 416-417 (1946), certain N-phenyl-N'-acylhydrazines are disclosed and evaluated for their toxicity against codling moth larvae.

The N-substituted-N'-substituted-N,N'-diacyl hydrazines of the present invention differ from known compounds primarily by their N- and N'-substituents.

We have found compounds of the invention which are distinguished by their excellent insecticidal activity against insects of the orders Lepidoptera and Coleoptera and particularly against insects of the order Lepidoptera, without material adverse impact on beneficial insects.

The insecticidal compounds of the invention comprise substituted hydrazines of the formula:

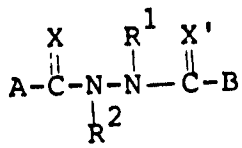

I

wherein

X and X' are the same or different O, S or NR;

$R^1$ is unsubstituted ($C_3$-$C_{10}$) branched alkyl or a ($C_1$-$C_4$) straight chain alkyl substituted with one or two of the same or different ($C_3$-$C_6$)cycloalkyl;

$R^2$ is ($C_1$-$C_6$)alkyl; alkoxyalkyl having, independently, 1 to 6 carbon atoms in each alkyl group; alkylthioalkyl having, independently, 1 to 6 carbon atoms in each alkyl group; ($C_2$-$C_6$)alkenyl; ($C_2$-$C_6$)alkynyl; or phen-($C_1$-$C_4$)alkyl where the phenyl ring is unsubstituted or substituted with one to three of the same or different halo, cyano, nitro, hydroxy, ($C_1$-$C_4$)alkyl, halo-($C_1$-$C_4$)alkyl, ($C_1$-$C_4$)alkoxy, halo-($C_1$-$C_4$)alkoxy, carboxy, ($C_1$-$C_4$)alkoxy-carbonyl, ($C_1$-$C_4$)alkanoyloxy, $NH_2$, NHZ or NZZ';

A and B are the same or different unsubstituted or substituted naphthyl where the substituents can be from one to five of the same or different halo; cyano; nitro; hydroxy; ($C_1$-$C_4$)alkoxy; ($C_1$-$C_4$)alkyl; carboxy; ($C_1$-$C_4$)alkoxy-carbonyl; ($C_1$-$C_4$)alkanoyloxy, $NH_2$; NHZ or NZZ'; unsubstituted or substituted phenyl where the substituents can be from one to five of the same or different halo; nitroso; nitro; cyano; hydroxy; ($C_1$-$C_6$)alkyl; halo-($C_1$-$C_6$)alkyl; cyano-($C_1$-$C_6$)alkyl; ($C_1$-$C_6$)alkoxy; halo-($C_1$-$C_6$)alkoxy; alkoxyalkyl having, independently, 1 to 6 carbon atoms in each alkyl group; alkoxyalkoxy having, independently, 1 to 6 carbon atoms in each alkyl group; -$OCO_2$R group; ($C_2$-$C_6$)alkenyl optionally substituted with halo, cyano, ($C_1$-$C_4$)alkyl, ($C_1$-$C_4$)alkoxy, halo-($C_1$-$C_4$)alkoxy or ($C_1$-$C_4$)alkylthio; ($C_2$-$C_6$)alkenyl-carbonyl; ($C_2$-$C_6$)alkadienyl; ($C_2$-$C_6$)alkynyl optionally substituted with halo, cyano, nitro, hydroxy, ($C_1$-$C_4$)alkyl, ($C_1$-$C_4$)alkoxy, halo-($C_1$-$C_4$)alkoxy or ($C_1$-$C_4$)-alkylthio; ($C_2$-$C_6$)alkynyl-carbonyl; carboxy; -$ZCO_2$R' group; -COR group; halo-($C_1$-$C_6$)-alkyl-carbonyl; cyano-($C_1$-$C_6$)alkyl-carbonyl; nitro-($C_1$-$C_6$)alkyl-carbonyl; ($C_1$-$C_6$)alkoxy-carbonyl; halo-($C_1$-$C_6$)alkoxy-carbonyl; -OCOR group; amino; amino substituted with hydroxy, ($C_1$-$C_4$)alkoxy or ($C_1$-$C_4$)alkylthio groups; phenylamino; diphenylamino; -CONRR' group; -OCONRR' group -C(NR)NR'R'' group; -N=NR group; phenylazo; -NRCOR' group; -$NRCO_2$R' group; -N(COR)COR' group; -OCONRCOR' group; sulfhydryl; halothio; thiocyanato; ($C_1$-$C_6$)alkylthio; ($C_1$-$C_6$)haloalkylthio; -SOR group; -$SO_2$R group; phenylsulfonyl; -$OSO_2$R group; halo-($C_1$-$C_6$)alkylsulfonyloxy; -$SO_2$NRR' group; -NRSOR' group; -$NRSOR_2$R' group; -CSR group; -$CS_2$R group; -NRCSR' group; -SCOR group; tri-($C_1$-$C_6$)alkylsilyl; unsubstituted or substituted phenyl having one to three of the same or different halo, cyano, nitro, hydroxy ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ'; phenoxy where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ'; benzoyl where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)-alkanoyloxy, $NH_2$, NHZ or NZZ'; phenoxycarbonyl where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)-alkanoyloxy, $NH_2$, NHZ or NZZ'; phenylthio where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ'; -CR=N-$R^2$ group where $R^2$ is hydroxy, ($C_1$-$C_4$)alkyl, ($C_1$-$C_4$)alkoxy, amino (-NRR'), phenylamino, -COR group, carboxy, ($C_1$-$C_4$)alkoxy-carbonyl, ($C_1$-$C_4$)alkanoyloxy, benzoyl, phenoxycarbonyl or -CONRR' group; or when two adjacent positions on the phenyl ring are substituted with alkoxy groups, these groups may be joined to form, together with the carbon atoms to which they are attached, a 5 or 6 membered dioxolano or dioxano heterocyclic ring;

where R, R' and R'' are hydrogen or ($C_1$-$C_6$)alkyl; Z and Z' are ($C_1$-$C_4$)alkyl, "amino" means NRR'; plus N-trimethylsiloxymethyl-N'-2,2 dimethylpropyl-N-(4-methoxy-3-chlorobenzoyl)-N'-benzoylhydrazine and N-phenylthiomethyl-t-butyl-N-(4-toluoyl)-N'-(3,4-dichlorobenzoyl) hydrazine, but excluding

$N(CHMe_2)COPh$
|
$N(CHMe_2)COPh$

and

$N(CH_2CHMe_2)COPh$
|
$N(CH_2CHMe_2)COPh$.

and
agronomically acceptable salts thereof.

The invention also extends to insecticidal compositions comprising an insecticidal compound of the invention together with agronomically acceptable diluent or carrier and to the use of the compounds and compositions as insecticides.

The term "halo" should be understood as including chloro, fluoro, bromo and iodo. The term "alkyl" by itself or as a part of another substituent, unless otherwise stated, includes straight or branched chain groups such as methyl, ethyl, n-propyl, isopropyl, n-butyl, t-butyl, isobutyl, neopentyl and the like and where indicated higher homologues and isomers such as n-octyl, isooctyl and the like. The term "haloalkyl" by itself or as part of another substituent is an alkyl group of the stated number of carbon atoms having one or more halo atoms bonded thereto such as chloromethyl, 1- or 2- bromoethyl, trifluoromethyl and the like. Analogously, "cyanoalkyl" by itself or as part of another group is an alkyl group of the stated number of carbon atoms having one or more cyano groups bonded thereto; "haloalkoxy" by itself or as part of another group is an alkoxy group of the stated number of carbon atoms having one or more halo atoms bonded thereto such as difluoromethoxy, trifluoromethoxy, 2-fluoroethoxy, 2,2,2-trifluoroethoxy and the like. "Alkenyl" and "alkynyl" by themselves or as part of another substituent comprise straight and branched chain groups of the stated number of carbon atoms. "Alkdienyl" is a straight or branched chain alkenyl group comprising two carbon-carbon double bonds that can be conjugated such as 1,3-butadienyl, cumulated such as 1,2-propadienyl or isolated such as 1,4-pentadienyl.

Typical compounds of interest within the scope of the present invention include, but are not limited to:

N-methyl-N'-t-butyl-N,N'-bis(4-chlorobenzoyl)hydrazine
N-methyl-N'-t-butyl-N,N'-bis(3-chlorobenzoyl)hydrazine
N-methyl-N'-t-butyl-N,N'-dibenzoyl hydrazine
N-methyl-N'-t-butyl-N,N'-bis(3,4-dichlorobenzoyl)hydrazine
N-methyl-N'-t-butyl-N,N'-bis(4-toluoyl)hydrazine
N-methyl-N'-t-butyl-N,N'-bis(4-nitrobenzoyl)hydrazine
N-methyl-N'-t-butyl-N,N'-bis(4-anisoyl)hydrazine
N-methyl-N'-t-butyl-N,N'-bis(3-nitrobenzoyl)hydrazine
N-methyl-N'-t-butyl-N,N'-bis(3-anisoyl)hydrazine
N-methyl-N'-t-butyl-N,N'-bis(2-nitrobenzoyl)hydrazine
N-methyl-N'-t-butyl-N,N'-bis(2-chlorobenzoyl)hydrazine
N-methyl-N'-t-butyl-N,N'-bis(2-anisoyl)hydrazine
N-methyl-N'-t-butyl-N-(4-toluoyl)-N'-benzoyl hydrazine
N-methyl-N'-t-butyl-N,N'-bis(4-cyanobenzoyl)hydrazine
N-methyl-N'-t-butyl-N-(4-toluoyl)-N'-(4-chlorobenzoyl)hydrazine
N-methyl-N'-t-butyl-N-benzoyl-N'-(4-chlorobenzoyl)hydrazine
N-methyl-N'-t-butyl-N-benzoyl-N'-(3-chlorobenzoyl)hydrazine
N-methyl-N'-t-butyl-N-benzoyl-N'-(2-chlorobenzoyl)hydrazine
N-methyl-N'-t-butyl-N,N'-bis(3-toluoyl)hydrazine
N-methyl-N'-t-butyl-N,N'-bis(2-toluoyl)hydrazine
N-methyl-N'-t-butyl-N-benzoyl-N'-(4-toluoyl)hydrazine
N-methyl-N'-t-butyl-N-benzoyl-N'-(3-toluoyl)hydrazine
N-methyl-N'-t-butyl-N-benzoyl-N'-(2-toluoyl)hydrazine
N-methyl-N'-t-butyl-N-benzoyl-N'-(4-anisoyl)hydrazine
N-methyl-N'-t-butyl-N-benzoyl-N'-(3-anisoyl)hydrazine

N-methyl-N'-t-butyl-N-benzoyl-N'-(2-anisoyl)hydrazine
N-methyl-N'-t-butyl-N-benzoyl-N'-(4-n-butylbenzoyl)hydrazine
N-methyl-N'-t-butyl-N-benzoyl-N'-(4-cyanobenzoyl)hydrazine
N-methyl-N'-t-butyl-N-benzoyl-N'-(4-nitrobenzoyl)hydrazine
N-methyl-N'-t-butyl-N-benzoyl-N'-(3-nitrobenzoyl)hydrazine
N-methyl-N'-t-butyl-N-benzoyl-N'-(2-nitrobenzoyl)hydrazine
N-methyl-N'-t-butyl-N,N'-bis(4-t-butylbenzoyl)hydrazine
N-methyl-N'-t-butyl-N-(4-toluoyl)-N'-(3,4-dichlorobenzoyl)hydrazine
N-methyl-N'-t-butyl-N-benzoyl-N'-(4-fluorobenzoyl)hydrazine
N-methyl-N'-t-butyl-N-benzoyl-N'-(3-fluorobenzoyl)hydrazine
N-methyl-N'-t-butyl-N-benzoyl-N'-(2-fluorobenzoyl)hydrazine
N-methyl-N'-t-butyl-N-benzoyl-N'-(2,4-dichlorobenzoyl)hydrazine
N-methyl-N'-isopropyl-N,N'-dibenzoyl hydrazine
N-methyl-N'-t-butyl-N-benzoyl-N'-(4-trifluoromethylbenzoyl)hydrazine
N-methyl-N'-t-butyl-N-benzoyl-N'-(3-trifluoromethylbenzoyl)hydrazine
N-methyl-N'-t-butyl-N-benzoyl-N'-(2-trifluoromethylbenzoyl)hydrazine
N-methyl-N'-t-butyl-N-benzoyl-N'-(2,5-difluorobenzoyl)hydrazine
N-methyl-N'-(2,2-dimethylethyl)-N,N'-dibenzoyl hydrazine
N-methyl-N'-t-butyl-N-benzoyl-N'-(3-cyanobenzoyl)hydrazine
N-methyl-N'-(1-methylpropyl)-N,N'-dibenzoyl hydrazine
N-methyl-N'-t-butyl-N-benzoyl-N'-(2,6-difluorobenzoyl)hydrazine
N-methyl-N'-t-butyl-N-(4-chlorobenzoyl)-N'-benzoyl hydrazine
N-methyl-N'-t-butyl-N-benzoyl-N'-(3,4-dichlorobenzoyl)hydrazine
N-methyl-N'-t-butyl-N-benzoyl-N'-(3,5-dichlorobenzoyl)hydrazine
N-methyl-N'-t-butyl-N-benzoyl-N'-(2,6-dichlorobenzoyl)hydrazine
N-methyl-N'-t-butyl-N-(4-t-butylbenzoyl)-N'-benzoyl hydrazine
N-methyl-N'-t-butyl-N-(2-chlorobenzoyl)-N'-benzoyl hydrazine
N-methyl-N'-t-butyl-N-(1-naphthoyl)-N'-benzoyl hydrazine
N-methyl-N'-t-butyl-N,N'-dinaphthoyl hydrazine
N-methyl-N'-t-butyl-N-(3-chlorobenzoyl)-N'-benzoyl hydrazine
N-methyl-N'-t-butyl-N-(4-chlorobenzoyl)-N'-(3,4-dichlorobenzoyl)hydrazine
N-methyl-N'-t-butyl-N-(2-chlorobenzoyl)-N'-(3,4-dichlorobenzoyl)hydrazine
N-methyl-N'-t-butyl-N-(2-toluoyl)-N'-benzoyl hydrazine
N-methyl-N'-t-butyl-N-benzoyl-N'-(2-chloro-4-nitrobenzoyl)hydrazine
N-methyl-N'-t-butyl-N-benzoyl-N'-(3,5-dinitrobenzoyl)hydrazine
N-methyl-N'-t-butyl-N-benzoyl-N'-(2,3-dichlorobenzoyl)hydrazine
N-methyl-N'-(1,2,2-trimethylethyl)-N,N'-dibenzoyl hydrazine
N-methyl-N'-t-butyl-N-benzoyl-N'-(2-chloro-5-methylbenzoyl)hydrazine
N-methyl-N'-t-butyl-N-benzoyl-N'-(3,5-dimethylbenzoyl)hydrazine
N-methyl-N'-t-butyl-N-benzoyl-N'-(2-nitro-5-methylbenzoyl)hydrazine
N-methyl-N'-t-butyl-N-benzoyl-N'-(2-methyl-3-chlorobenzoyl)hydrazine
N-methyl-N'-t-butyl-N-benzoyl-N'-(3-chloro-4-methylbenzoyl)hydrazine
N-methyl-N'-t-butyl-N-benzoyl-N'-(2-nitro-3-chlorobenzoyl)hydrazine
N-methyl-N'-t-butyl-N-benzoyl-N'-(3-methoxy-4-nitrobenzoyl)hydrazine
N-methyl-N'-t-butyl-N-benzoyl-N'-(2-nitro-3-methoxybenzoyl)hydrazine
N-methyl-N'-t-butyl-N-benzoyl-N'-(2,4-dinitrobenzoyl)hydrazine
N-methyl-N'-t-butyl-N-(4-chlorobenzoyl)-N'-(2-chlorobenzoyl)hydrazine
N-methyl-N'-t-butyl-N-(4-chlorobenzoyl)-N'-(3-chlorobenzoyl)hydrazine
N-methyl-N'-t-butyl-N-(4-chlorobenzoyl)-N'-(4-toluoyl)hydrazine
N-methyl-N'-t-butyl-N-(4-chlorobenzoyl)-N'-(3,5-dichlorobenzoyl)hydrazine
N-methyl-N'-t-butyl-N-(4-chlorobenzoyl)-N'-(2,4-dichlorobenzoyl)hydrazine
N-methyl-N'-t-butyl-N-(4-chlorobenzoyl)-N'-(4-trifluoromethylbenzoyl)hydrazine
N-methyl-N'-t-butyl-N-benzoyl-N'-(4-methanesulfonyloxybenzoyl)hydrazine
N-methyl-N'-t-butyl-N-benzoyl-N'-(4-isopropylbenzoyl)hydrazine
N-methyl-N'-t-butyl-N-benzoyl-N'-(2-acetoxybenzoyl)hydrazine
N-methyl-N'-t-butyl-N-benzoyl-N'-(4-ethylbenzoyl)hydrazine
N-methyl-N'-t-butyl-N-benzoyl-N'-(2-bromobenzoyl)hydrazine
N-methyl-N'-t-butyl-N-benzoyl-N'-(4-hydroxybenzoyl)hydrazine

N-methyl-N'-t-butyl-N-(4-toluoyl)-N'-(2-toluoyl)hydrazine
N-methyl-N'-t-butyl-N-(4-toluoyl)-N'-(3-toluoyl)hydrazine
N-methyl-N'-t-butyl-N-(4-toluoyl)-N'-(2,4-dichlorobenzoyl)hydrazine
N-methyl-N'-t-butyl-N-(4-toluoyl)-N'-(3,5-dichlorobenzoyl)hydrazine
N-methyl-N'-t-butyl-N-(4-toluoyl)-N'-(2-chlorobenzoyl)hydrazine
N-methyl-N'-t-butyl-N-(4-toluoyl)-N'-(4-fluorobenzoyl)hydrazine
N-methyl-N'-t-butyl-N-(4-toluoyl)-N'-(4-trifluoromethylbenzoyl)hydrazine
N-methyl-N'-t-butyl-N-(4-toluoyl)-N'-(3-chlorobenzoyl)hydrazine
N-methyl-N'-t-butyl-N-(4-chlorobenzoyl)-N'-(3-chlorobenzoyl)hydrazine
N-methyl-N'-t-butyl-N-(4-chlorobenzoyl)-N'-(4-chloromethylbenzoyl)hydrazine
N-methyl-N'-t-butyl-N-(4-chlorobenzoyl)-N'-(2-toluoyl)hydrazine
N-methyl-N'-t-butyl-N-(4-chlorobenzoyl)-N'-(3-anisoyl)hydrazine
N-methyl-N'-t-butyl-N-(4-chlorobenzoyl)-N'-(3-toluoyl)hydrazine
N-methyl-N'-t-butyl-N,N'-bis(4-fluorobenzoyl)hydrazine
N-methyl-N'-t-butyl-N,N'-bis(3-fluorobenzoyl)hydrazine
N-methyl-N'-t-butyl-N,N'-bis(2-fluorobenzoyl)hydrazine
N-methyl-N'-t-butyl-N,N'-bis(2-naphthoyl)hydrazine
N-methyl-N'-t-butyl-N-(4-isobutylbenzoyl)-N'-(2-nitrobenzoyl)hydrazine
N-methyl-N'-t-butyl-N-(2-bromobenzoyl)-N'-(4-ethenylbenzoyl)hydrazine
N-methyl-N'-t-butyl-N-(4-toluoyl)-N'-(4-ethynylbenzoyl)hydrazine
N-methyl-N'-t-butyl-N-[4-(1-hydroxy-2-propynyl)benzoyl]-N'-(3,4-methylenedioxybenzoyl)hydrazine
N-methyl-N'-t-butyl-N-(3-phenoxybenzoyl)-N'-(2-bromobenzoyl)hydrazine
N-methyl-N'-t-butyl-N-(2,4-dichlorobenzoyl)-N'-(4-trifluoromethoxybenzoyl)hydrazine
N-methyl-N'-t-butyl-N-(4-ethylbenzoyl)-N'-(2-difluoromethoxy-4-chlorobenzoyl)hydrazine
N-methyl-N'-isopropyl-N'-(4-chloro-2-bromobenzoyl)-N-benzoyl hydrazine
N-methyl-N'-(2,2-dimethylethyl)-N-(3-bromomethylbenzoyl)-N'-(4-isopropyloxybenzoyl)hydrazine
N-methyl-N'-t-butyl-N-(4-chloromethylbenzoyl)-N'-(2-carboxybenzoyl)hydrazine
N-methyl-N'-(1-methylpropyl)-N-(4-carboxybenzoyl)-N'-(3,4,5-trichlorobenzoyl)hydrazine
N-methyl-N'-t-butyl-N-(4-propanoylbenzoyl)-N'-[4-(4-pentenyl)benzoyl]hydrazine
N-methyl-N'-(1,2,2-trimethylpropyl)-N-[2-(ethoxy-1-ethoxyl)benzoyl]-N'-[4-(2-ethylbutanoyl)benzoyl]-hydrazine
N-methyl-N'-t-butyl-N-(6-bromo-2-naphthoyl)-N'-(4-benzoylbenzoyl)hydrazine
N-methyl-N'-isopropyl-N-(4-(2-pentynoyl)benzoyl)-N'-(3-nitrobenzoyl)hydrazine
N-methyl-N'-t-butyl-N-(4-bromo-2-cyanobenzoyl)-N'-(6-(5-oxotetrahydronaphthoyl)hydrazine
N-methyl-N'-(2,2-dimethylpropyl)-N-(4-t-butyloxycarbonylbenzoyl)-N'-(4-chloro-3-trifluoromethoxybenzoyl)hydrazine
N-methyl-N'-t-butyl-N-(2-benzyloxycarbonylbenzoyl)-N'-(2-methoxy-4-bromobenzoyl)hydrazine
N-methyl-N'-t-butyl-N-(4-(2,2,2-trifluoroethoxycarbonyl)-3-methylbenzoyl-N'-(2,4-dichloro-3-hydroxybenzoyl)hydrazine
N-methyl-N'-isopropyl-N-(3-propanoyloxybenzoyl)-N'-(2,5-dibromobenzoyl)hydrazine
N-methyl-N'-(1,2,2-trimethylpropyl)-N-(4-propylbenzoyl)-N'-(3-ethoxycarbonyloxybenzoyl)hydrazine
N-methyl-N'-t-butyl-N-(3,5-dimethylbenzoyl)-N'-(4-t-butylcarbonyloxybenzoyl)hydrazine
N-methyl-N'-(1-methylpropyl)-N-(2-aminobenzoyl)-N'-(3,4-dichlorobenzoyl)hydrazine
N-methyl-N'-t-butyl-N-(4-chloro-2-trifluoromethoxybenzoyl)-N'-(4-methylaminobenzoyl)hydrazine
N-methyl-N'-t-butyl-N-(4-dimethylaminobenzoyl)-N'-(4-acetylaminobenzoyl)hydrazine
N-methyl-N'-t-butyl-N-(3-formylbenzoyl)-N'-(2-chloro-4-(N-hydroxyformiminoyl)benzoyl)hydrazine
N-methyl-N'-t-butyl-N-(2-methanesulfonylaminobenzoyl)-N'-(2-chloro-3-(N'-phenylhydrazinoformyl)-benzoyl)hydrazine
N-methyl-N'-(1-methylpropyl)-N-(2-aminocarbonylbenzoyl)-N'-(2-chloro-4-ethylaminocarbonylbenzoyl)-hydrazine
N-methyl-N'-isopropyl-N-(4-methyl-3-dimethylaminocarbonylbenzoyl)-N'-(4-trifluoromethylbenzoyl)-hydrazine
N-methyl-N'-(1,2,2-trimethylpropyl)-N-(4-trifluoromethoxy-2-chlorobenzoyl)-N'-(4-methoxycarbonylaminobenzoyl)hydrazine
N-methyl-N'-t-butyl-N-(2-carboxymethylbenzoyl)-N'-(4-dimethylaminocarbonyloxybenzoyl)hydrazine
N-methyl-N'-t-butyl-N-(3-methylaminocarbonyloxybenzoyl)-N'-(2-chloro-4-(N-acetoxyaminocarbonyloxy)-benzoyl hydrazine
N-methyl-N'-isopropyl-N-(4-methoxy-3-bromobenzoyl)-N'-(4-sulfhydrylbenzoyl)hydrazine

N-methyl-N'-(1-methylpropyl)-N-(3-chloro-5-sulfhydrylbenzoyl)-N'-(3-phenylazobenzoyl)hydrazine
N-methyl-N'-(2,2-dimethylpropyl)-N-(2-methylthiobenzoyl)-N'-(2-chloro-4-(1,3-dioxolano-2-yl-benzoyl)-hydrazine
N-methyl-N'-t-butyl-N-(3-methanesulfinylbenzoyl)-N'-(3,4,5-trimethoxybenzoyl)hydrazine
N-methyl-N'-(1,2,2-trimethylpropyl)-N-(3-phenylsufonylbenzoyl)-N'-(3,4-dichlorobenzoyl)hydrazine
N-methyl-N'-t-butyl-N-(3-trifluoromethanesulfonyloxybenzoyl)-N'-(2-chloro-4-trichloromethylthiobenzoyl)-hydrazine
N-methyl-N'-t-butyl-N-(2-iodobenzoyl)-N'-(4-aminosulfonyloxy)hydrazine
N-methyl-N'-t-butyl-N-(2,5-dichlorobenzoyl)-N'-(4-trimethylsilylbenzoyl)hydrazine
N-methyl-N'-(1,2,2-trimethylpropyl)-N-(4-acetylthiobenzoyl)-N'-(3,4-dichlorobenzoyl)hydrazine
N-methyl-N'-t-butyl-N-(methylthiocarbonylthioxybenzoyl)-N'-(3-chloro-4-formylaminobenzoyl) hydrazine
N-methyl-N'-t-butyl-N-(3-methylthiocarbonylbenzoyl)-N'-(4-pentafluoroethoxybenzoyl)hydrazine
N-methyl-N'-(t-butyl)-N-(pentafluorobenzoyl)-N'-(4-phenylaminobenzoyl)hydrazine
N-methyl-N'-(t-butyl)-N-(6-chlorophenylbenzoyl)-N'-(3-chloro-4-acetylaminobenzoyl)hydrazine
N-methyl-N'-isopropyl-N-(3-hydroxyaminobenzoyl)-N'-(4-tribromomethylbenzoyl)hydrazine
N-methyl-N'-(1,2,2-trimethylpropyl)-N-(4-aminocarbonylaminobenzoyl)-N'-(2-bromobenzoyl)hydrazine
N-methyl-N'-(1-methylpropyl)-N-(4-fluoro-3-bromochloromethylbenzoyl)-N'-(3-cyanomethylbenzoyl)-hydrazine
N-methyl-N'-t-butyl-N-(4-propylthiobenzoyl)-N'-(3,4-dichlorobenzoyl)hydrazine
N-methyl-N'-t-butyl-N-(4-chloromethylcarbonylbenzoyl)-N'-(2-bromobenzoyl)hydrazine
N-methyl-N'-t-butyl-N-(3-trichloroethenylbenzoyl)-N'-(4-fluorobenzoyl)hydrazine
N-methyl-N'-isopropyl-N-(4-(1,3-dimethylbutyl)benzoyl)-N'-(2-nitrobenzoyl)hydrazine
N-methyl-N'-t-butyl-N-(4-nitrosobenzoyl)-N'-(2,4-dichlorobenzoyl)hydrazine
N-methyl-N'-t-butyl-N-(4-(N'-methylformamidinoylbenzoyl)-N'-(3-chloro-4-bromobenzoyl)hydrazine
N-methyl-N'-isopropyl-N-(2,6-dichlorobenzoyl)-N'-(4-trifluoromethoxybenzoyl)hydrazine
N-methyl-N'-t-butyl-(2,3,4-trichlorobenzoyl)-N'-(2-nitrobenzoyl)hydrazine
N-methyl-N'-t-butyl-N-(2-chlorobenzoyl)-N'-(4-chlorosulfenylbenzoyl)hydrazine
N-methyl-N'-t-butyl-N-(4-allenylbenzoyl)-N'-(4-chlorobenzoyl)hydrazine
N-ethyl-N'-t-butyl-N,N'-bis(4-chlorobenzoyl)hydrazine
N-ethyl-N'-t-butyl-N,N'-bis(3-chlorobenzoyl)hydrazine
N-ethyl-N'-t-butyl-N,N'-dibenzoyl hydrazine
N-ethyl-N'-t-butyl-N,N'-bis(3,4-dichlorobenzoyl)hydrazine
N-ethyl-N'-t-butyl-N,N'-bis(4-toluoyl)hydrazine
N-ethyl-N'-t-butyl-N,N'-bis(4-nitrobenzoyl)hydrazine
N-ethyl-N'-t-butyl-N,N'-bis(4-anisoyl)hydrazine
N-ethyl-N'-t-butyl-N,N'-bis(3-nitrobenzoyl)hydrazine
N-ethyl-N'-t-butyl-N,N'-bis(3-anisoyl)hydrazine
N-ethyl-N'-t-butyl-N,N'-bis(2-nitrobenzoyl)hydrazine
N-ethyl-N'-t-butyl-N,N'-bis(2-chlorobenzoyl)hydrazine
N-ethyl-N'-t-butyl-N,N'-bis(2-anisoyl)hydrazine
N-ethyl-N'-t-butyl-N-(4-toluoyl)-N'-benzoyl hydrazine
N-ethyl-N'-t-butyl-N,N'-bis(4-cyanobenzoyl)hydrazine
N-ethyl-N'-t-butyl-N-(4-toluoyl)-N'-(4-chlorobenzoyl)hydrazine
N-ethyl-N'-t-butyl-N-benzoyl-N'-(4-chlorobenzoyl)hydrazine
N-ethyl-N'-t-butyl-N-benzoyl-N'-(3-chlorobenzoyl)hydrazine
N-ethyl-N'-t-butyl-N-benzoyl-N'-(2-chlorobenzoyl)hydrazine
N-ethyl-N'-t-butyl-N,N'-bis(3-toluoyl)hydrazine
N-ethyl-N'-t-butyl-N,N'-bis(2-toluoyl)hydrazine
N-ethyl-N'-t-butyl-N-benzoyl-N'-(4-toluoyl)hydrazine
N-ethyl-N'-t-butyl-N-benzoyl-N'-(3-toluoyl)hydrazine
N-ethyl-N'-t-butyl-N-benzoyl-N'-(2-toluoyl)hydrazine
N-ethyl-N'-t-butyl-N-benzoyl-N'-(4-anisoyl)hydrazine
N-ethyl-N'-t-butyl-N-benzoyl-N'-(3-anisoyl)hydrazine
N-ethyl-N'-t-butyl-N-benzoyl-N'-(2-anisoyl)hydrazine
N-ethyl-N'-t-butyl-N-benzoyl-N'-(4-n-butylbenzoyl)hydrazine
N-ethyl-N'-t-butyl-N-benzoyl-N'-(4-cyanobenzoyl)hydrazine
N-ethyl-N'-t-butyl-N-benzoyl-N'-(4-nitrobenzoyl)hydrazine
N-ethyl-N'-t-butyl-N-benzoyl-N'-(3-nitrobenzoyl)hydrazine

N-ethyl-N'-t-butyl-N-benzoyl-N'-(2-nitrobenzoyl)hydrazine
N-ethyl-N'-t-butyl-N,N'-bis(4-t-butylbenzoyl)hydrazine
N-ethyl-N'-t-butyl-N-(4-toluoyl)-N'-(3,4-dichlorobenzoyl)hydrazine
N-ethyl-N'-t-butyl-N-benzoyl-N'-(4-fluorobenzoyl)hydrazine
N-ethyl-N'-t-butyl-N-benzoyl-N'-(3-fluorobenzoyl)hydrazine
N-ethyl-N'-t-butyl-N-benzoyl-N'-(2-fluorobenzoyl)hydrazine
N-ethyl-N'-t-butyl-N-benzoyl-N'-(2,4-dichlorobenzoyl)hydrazine
N-ethyl-N'-isopropyl-N,N'-dibenzoyl hydrazine
N-ethyl-N'-t-butyl-N-benzoyl-N'-(4-trifluoromethylbenzoyl)hydrazine
N-ethyl-N'-t-butyl-N-benzoyl-N'-(3-trifluoromethylbenzoyl)hydrazine
N-ethyl-N'-t-butyl-N-benzoyl-N'-(2-trifluoromethylbenzoyl)hydrazine
N-ethyl-N'-t-butyl-N-benzoyl-N'-(2,5-difluorobenzoyl)hydrazine
N-ethyl-N'-(2,2-dimethylethyl)-N,N'-dibenzoyl hydrazine
N-ethyl-N'-t-butyl-N-benzoyl-N'-(3-cyanobenzoyl)hydrazine
N-ethyl-N'-(1-methylpropyl)-N,N'-dibenzoyl hydrazine
N-ethyl-N'-t-butyl-N-benzoyl-N'-(2,6-difluorobenzoyl)hydrazine
N-ethyl-N'-t-butyl-N-(4-chlorobenzoyl)-N'-benzoyl hydrazine
N-ethyl-N'-t-butyl-N-benzoyl-N'-(3,4-dichlorobenzoyl)hydrazine
N-ethyl-N'-t-butyl-N-benzoyl-N'-(3,5-dichlorobenzoyl)hydrazine
N-ethyl-N'-t-butyl-N-benzoyl-N'-(2,6-dichlorobenzoyl)hydrazine
N-ethyl-N'-t-butyl-N-(4-t-butylbenzoyl)-N'-benzoyl hydrazine
N-ethyl-N'-t-butyl-N-(2-chlorobenzoyl)-N'-benzoyl hydrazine
N-ethyl-N'-t-butyl-N-(1-naphthoyl)-N'-benzoyl hydrazine
N-ethyl-N'-t-butyl-N,N'-dinaphthoyl hydrazine
N-ethyl-N'-t-butyl-N-(3-chlorobenzoyl)-N'-benzoyl hydrazine
N-ethyl-N'-t-butyl-N-(4-chlorobenzoyl)-N'-(3,4-dichlorobenzoyl)hydrazine
N-ethyl-N'-t-butyl-N-(2-chlorobenzoyl)-N'-(3,4-dichlorobenzoyl)hydrazine
N-ethyl-N'-t-butyl-N-(2-toluoyl)-N'-benzoyl hydrazine
N-ethyl-N'-t-butyl-N-benzoyl-N'-(2-chloro-4-nitrobenzoyl)hydrazine
N-ethyl-N'-t-butyl-N-benzoyl-N'-(3,5-dinitrobenzoyl)hydrazine
N-ethyl-N'-t-butyl-N-benzoyl-N'-(2,3-dichlorobenzoyl)hydrazine
N-ethyl-N'-(1,2,2-trimethylethyl)-N,N'-dibenzoyl hydrazine
N-ethyl-N'-t-butyl-N-benzoyl-N'-(2-chloro-5-methylbenzoyl)hydrazine
N-ethyl-N'-t-butyl-N-benzoyl-N'-(3,5-dimethylbenzoyl)hydrazine
N-ethyl-N'-t-butyl-N-benzoyl-N'-(2-nitro-5-methylbenzoyl)hydrazine
N-ethyl-N'-t-butyl-N-benzoyl-N'-(2-methyl-3-chlorobenzoyl)hydrazine
N-ethyl-N'-t-butyl-N-benzoyl-N'-(3-chloro-4-methylbenzoyl)hydrazine
N-ethyl-N'-t-butyl-N-benzoyl-N'-(2-nitro-3-chlorobenzoyl)hydrazine
N-ethyl-N'-t-butyl-N-benzoyl-N'-(3-methoxy-4-nitrobenzoyl)hydrazine
N-ethyl-N'-t-butyl-N-benzoyl-N'-(2-nitro-3-methoxybenzoyl)hydrazine
N-ethyl-N'-t-butyl-N-benzoyl-N'-(2,4-dinitrobenzoyl)hydrazine
N-ethyl-N'-t-butyl-N-(4-chlorobenzoyl)-N'-(2-chlorobenzoyl)hydrazine
N-ethyl-N'-t-butyl-N-(4-chlorobenzoyl)-N'-(3-chlorobenzoyl)hydrazine
N-ethyl-N'-t-butyl-N-(4-chlorobenzoyl)-N'-(4-toluoyl)hydrazine
N-ethyl-N'-t-butyl-N-(4-chlorobenzoyl)-N'-(3,5-dichlorobenzoyl)hydrazine
N-ethyl-N'-t-butyl-N-(4-chlorobenzoyl)-N'-(2,4-dichlorobenzoyl)hydrazine
N-ethyl-N'-t-butyl-N-(4-chlorobenzoyl)-N'-(4-trifluoromethylbenzoyl)hydrazine
N-ethyl-N'-t-butyl-N-benzoyl-N'-(4-methanesulfonyloxybenzoyl)hydrazine
N-ethyl-N'-t-butyl-N-benzoyl-N'-(4-isopropylbenzoyl)hydrazine
N-ethyl-N'-t-butyl-N-benzoyl-N'-(2-acetoxybenzoyl)hydrazine
N-ethyl-N'-t-butyl-N-benzoyl-N'-(4-ethylbenzoyl)hydrazine
N-ethyl-N'-t-butyl-N-benzoyl-N'-(2-bromobenzoyl)hydrazine
N-ethyl-N'-t-butyl-N-benzoyl-N'-(4-hydroxybenzoyl)hydrazine
N-ethyl-N'-t-butyl-N-(4-toluoyl)-N'-(2-toluoyl)hydrazine
N-ethyl-N'-t-butyl-N-(4-toluoyl)-N'-(3-toluoyl)hydrazine
N-ethyl-N'-t-butyl-N-(4-toluoyl)-N'-(2,4-dichlorobenzoyl)hydrazine
N-ethyl-N'-t-butyl-N-(4-toluoyl)-N'-(3,5-dichlorobenzoyl)hydrazine
N-ethyl-N'-t-butyl-N-(4-toluoyl)-N'-(2-chlorobenzoyl)hydrazine

N-ethyl-N'-t-butyl-N-(4-toluoyl)-N'-(4-fluorobenzoyl)hydrazine
N-ethyl-N'-t-butyl-N-(4-toluoyl)-N'-(4-trifluoromethylbenzoyl)hydrazine
N-ethyl-N'-t-butyl-N-(4-toluoyl)-N'-(3-chlorobenzoyl)hydrazine
N-ethyl-N'-t-butyl-N-(4-chlorobenzoyl)-N'-(3-chlorobenzoyl)hydrazine
N-ethyl-N'-t-butyl-N-(4-chlorobenzoyl)-N'-(4-chloromethylbenzoyl)hydrazine
N-ethyl-N'-t-butyl-N-(4-chlorobenzoyl)-N'-(2-toluoyl)hydrazine
N-ethyl-N'-t-butyl-N-(4-chlorobenzoyl)-N'-(3-anisoyl)hydrazine
N-ethyl-N'-t-butyl-N-(4-chlorobenzoyl)-N'-(3-toluoyl)hydrazine
N-ethyl-N'-t-butyl-N,N'-bis(4-fluorobenzoyl)hydrazine
N-ethyl-N'-t-butyl-N,N'-bis(3-fluorobenzoyl)hydrazine
N-ethyl-N'-t-butyl-N,N'-bis(2-fluorobenzoyl)hydrazine
N-ethyl-N'-t-butyl-N,N'-bis(2-naphthoyl)hydrazine
N-ethyl-N'-t-butyl-N-(4-isobutylbenzoyl)-N'-(2-nitrobenzoyl)hydrazine
N-ethyl-N'-t-butyl-N-(2-bromobenzoyl)-N'-(4-ethenylbenzoyl)hydrazine
N-ethyl-N'-t-butyl-N-(4-toluoyl)-N'-(4-ethynylbenzoyl)hydrazine
N-ethyl-N'-t-butyl-N-[4-(1-hydroxy-2-propynyl)benzoyl]-N'-(3,4-methylenedioxybenzoyl)hydrazine
N-ethyl-N'-t-butyl-N-(3-phenoxybenzoyl)-N'-(2-bromobenzoyl)hydrazine
N-ethyl-N'-t-butyl-N-(2,4-dichlorobenzoyl)-N'-(4-trifluoromethoxybenzoyl)hydrazine
N-ethyl-N'-t-butyl-N-(4-ethylbenzoyl)-N'-(2-difluoromethoxy-4-chlorobenzoyl)hydrazine
N-ethyl-N'-isopropyl-N'-(4-chloro-2-bromobenzoyl)-N-benzoylhydrazine
N-ethyl-N'-(2,2-dimethylethyl)-N-(3-bromomethylbenzoyl)-N'-(4-isopropyloxybenzoyl)hydrazine
N-ethyl-N'-t-butyl-N-(4-chloromethylbenzoyl)-N'-(2-carboxybenzoyl)hydrazine
N-ethyl-N'-(1-methylpropyl)-N-(4-carboxybenzoyl)-N'-(3,4,5-trichlorobenzoyl)hydrazine
N-ethyl-N'-t-butyl-N-(4-propanoylbenzoyl)-N'-[4-(4-pentenyl)benzoyl]hydrazine
N-ethyl-N'-(1,2,2-trimethylpropyl)-N-[2-(ethoxy-1-ethoxyl)benzoyl]-N'-[4-(2-ethylbutanoyl)benzoyl]-hydrazine
N-ethyl-N'-t-butyl-N-(6-bromo-2-naphthoyl)-N'-(4-benzoylbenzoyl)hydrazine
N-ethyl-N'-isopropyl-N-(4-(2-pentynoyl)benzoyl)-N'-(3-nitrobenzoyl)hydrazine
N-ethyl-N'-t-butyl-N-(4-bromo-2-cyanobenzoyl)-N'-(6-(5-oxotetrahydronaphthoyl)hydrazine
N-ethyl-N'-(2,2-dimethylpropyl)-N-(4-t-butyloxycarbonylbenzoyl)-N'-(4-chloro-3-trifluoromethoxybenzoyl)-hydrazine
N-ethyl-N'-t-butyl-N-(2-benzyloxycarbonylbenzoyl)-N'-(2-methoxy-4-bromobenzoyl)hydrazine
N-ethyl-N'-t-butyl-N-(4-(2,2,2-trifluoroethoxycarbonyl)-3-methylbenzoyl-N'-(2,4-dichloro-3-hydroxybenzoyl)hydrazine
N-ethyl-N'-isopropyl-N-(3-propanoyloxybenzoyl)-N'-(2,5-dibromobenzoyl)hydrazine
N-ethyl-N'-(1,2,2-trimethylpropyl)-N-(4-propylbenzoyl)-N'-(3-ethoxycarbonyloxybenzoyl)hydrazine
N-ethyl-N'-t-butyl-N-(3,5-dimethylbenzoyl)-N'-(4-t-butylcarbonyloxybenzoyl)hydrazine
N-ethyl-N'-(1-methylpropyl)-N-(2-aminobenzoyl)-N'-(3,4-dichlorobenzoyl)hydrazine
N-ethyl-N'-t-butyl-N-(4-chloro-2-trifluoromethoxybenzoyl)-N'-(4-methylaminobenzoyl)hydrazine
N-ethyl-N'-t-butyl-N-(4-dimethylaminobenzoyl)-N'-(4-acetylaminobenzoyl)hydrazine
N-ethyl-N'-t-butyl-N-(3-formylbenzoyl)-N'-(2-chloro-4-(N-hydroxyformiminoyl)benzoyl)hydrazine
N-ethyl-N'-t-butyl-N-(2-methanesulfonylaminobenzoyl)-N'-(2-chloro-3-(N'-phenylhydrazinoformyl)-benzoyl)hydrazine
N-ethyl-N'-(1-methylpropyl)-N-(2-aminocarbonylbenzoyl)-N'-(2-chloro-4-ethylaminocarbonylbenzoyl)-hydrazine
N-ethyl-N'-isopropyl-N-(4-methyl-3-dimethylaminocarbonylbenzoyl)-N'-(4-trifluoromethylbenzoyl)-hydrazine
N-ethyl-N'-(1,2,2-trimethylpropyl)-N-(4-trifluoromethoxy-2-chlorobenzoyl)-N'-(4-methoxycarbonylaminobenzoyl)hydrazine
N-ethyl-N'-t-butyl-N-(2-carboxymethylbenzoyl)-N'-(4-dimethylaminocarbonyloxybenzoyl)hydrazine
N-ethyl-N'-t-butyl-N-(3-methylaminocarbonyloxybenzoyl)-N'-(2-chloro-4-(N-acetoxyaminocarbonyloxy)-benzoyl hydrazine
N-ethyl-N'-isopropyl-N-(4-methoxy-3-bromobenzoyl)-N'-(4-sulfhydrylbenzoyl)hydrazine
N-ethyl-N'-(1-methylpropyl)-N-(3-chloro-5-sulfhydrylbenzoyl)-N'-(3-phenylazobenzoyl)hydrazine
N-ethyl-N'-(2,2-dimethylpropyl)-N-(2-methylthiobenzoyl)-N'-(2-chloro-4-(1,3-dioxolano-2-ylbenzoyl)-hydrazine
N-ethyl-N'-t-butyl-N-(3-methanesulfinylbenzoyl)-N'-(3,4,5-trimethoxybenzoyl)hydrazine
N-ethyl-N'-(1,2,2-trimethylpropyl)-N-(3-phenylsulfonylbenzoyl)-N'-(3,4-dichlorobenzoyl)hydrazine

N-ethyl-N'-t-butyl-N-(3-trifluoromethanesulfonyloxybenzoyl)-N'-(2-chloro-4-trichloromethylthiobenzoyl)-hydrazine
N-ethyl-N'-t-butyl-N-(2-iodobenzoyl)-N'-(4-aminosulfonyloxy)hydrazine
N-ethyl-N'-t-butyl-N-(2,5-dichlorobenzoyl)-N'-(4-trimethylsilylbenzoyl)hydrazine
N-ethyl-N'-(1,2,2-trimethylpropyl)-N-(4-acetylthiobenzoyl)-N'-(3,4-dichlorobenzoyl)hydrazine
N-ethyl-N'-t-butyl-N-(methylthiocarbonylthioxybenzoyl)-N'-(3-chloro-4-formylaminobenzoyl)hydrazine
N-ethyl-N'-t-butyl-N-(3-methylthiocarbonylbenzoyl)-N'-(4-pentafluoroethoxybenzoyl)hydrazine
N-ethyl-N'-(t-butyl)-N-(pentafluorobenzoyl)-N'-(4-phenylaminobenzoyl)hydrazine
N-ethyl-N'-(t-butyl)-N-(6-chlorophenylbenzoyl)-N'-(3-chloro-4-acetylaminobenzoyl)hydrazine
N-ethyl-N'-isopropyl-N-(3-hydroxyaminobenzoyl)-N'-(4-tribromomethylbenzoyl)hydrazine
N-ethyl-N'-(1,2,2-trimethylpropyl)-N-(4-aminocarbonylaminobenzoyl)-N'-(2-bromobenzoyl)hydrazine
N-ethyl-N'-(1-methylpropyl)-N-(4-fluoro-3-bromochloromethylbenzoyl)-N'-(3-cyanomethylbenzoyl)-hydrazine
N-ethyl-N'-t-butyl-N-(4-propylthiobenzoyl)-N'-(3,4-dichlorobenzoyl)hydrazine
N-ethyl-N'-t-butyl-N-(4-chloromethylcarbonylbenzoyl)-N'-(2-bromobenzoyl)hydrazine
N-ethyl-N'-t-butyl-N-(3-trichloroethenylbenzoyl)-N'-(4-fluorobenzoyl)hydrazine
N-ethyl-N'-isopropyl-N-(4-(1,3-dimethylbutyl)benzoyl)-N'-(2-nitrobenzoyl)hydrazine
N-ethyl-N'-t-butyl-N-(4-nitrosobenzoyl)-N'-(2,4-dichlorobenzoyl)hydrazine
N-ethyl-N'-t-butyl-N-(4-(N'-methylformamidinoylbenzoyl)-N'-(3-chloro-4-bromobenzoyl)hydrazine
N-ethyl-N'-isopropyl-N-(2,6-dichlorobenzoyl)-N'-(4-trifluoromethoxybenzoyl)hydrazine
N-ethyl-N'-t-butyl-(2,3,4-trichlorobenzoyl)-N'-(2-nitrobenzoyl)hydrazine
N-ethyl-N'-t-butyl-N-(2-chlorobenzoyl)-N'-(4-chlorosulfenylbenzoyl)hydrazine
N-ethyl-N'-t-butyl-N-(4-allenylbenzoyl)-N'-(4-chlorobenzoyl)hydrazine
N-benzyl-N'-t-butyl-N-(4-chlorobenzoyl)-N'-(4-ethylbenzoyl)hydrazine
N-allyl-N'-t-butyl-N-(4-ethylbenzoyl)-N'-(3-ethylbenzoyl)hydrazine
N-benzyl-N'-t-butyl-N,N'-dibenzoylhydrazine
N-allyl-N'-t-butyl-N,N'-dibenzoylhydrazine
N-methoxymethyl-N'-t-butyl-N,N'-dibenzoylhydrazine
N-methylthiomethyl-N'-t-butyl-N,N'-dibenzoylhydrazine
N-methylthiomethyl-N'-t-butyl-N,N'-bis(4-chlorobenzoyl)hydrazine
N-methoxymethyl-N'-t-butyl-N-(2-chlorobenzoyl)-N'-(2,4-dichlorobenzoyl)hydrazine
N-phenylthiomethyl-N'-t-butyl-N-(4-toluoyl)-N'-(2-nitrobenzoyl)hydrazine
N-trimethylsiloxymethyl-N'-2,2-dimethylpropyl-N-(4-methoxy-3-chlorobenzoyl)-N'-benzoylhdyrazine
N-isopropyl-N'-2,2-dimethylpropyl-N-(4-chlorobenzoyl)N'-(3-ethylbenzoyl)hydrazine
N-propargyl-N'-(2,2-dimethylpropyl)-N-(4-toluoyl)-N'-(2-bromobenzoyl)hydrazine
N-propyl-N'-t-butyl-N-(3-ethoxybenzoyl)-N'-(2-bromobenzoyl)hydrazine
N-hexyl-N'-t-butyl-N-(3,4-dichlorobenzoyl)-N'-(2-nitrobenzoyl)hydrazine
N-(3,5-dinitrobenzyl)-N'-t-butyl-N,N'-bis(2-chlorobenzoyl)hydrazine
N-(2,4-dichlorobenzyl)-N'-(1,2,2-trimethylpropyl)-N-(4-bromobenzoyl)-N'-(3-toluoyl)hydrazine
N-(4-methoxybenzyl)-N'-(2,2-trimethylpropyl)-N-(4-ethoxybenzoyl)-N'-(3-chlorobenzoyl)hydrazine
N-(3-nitrobenzyl)-N'-isopropyl-N-(4-ethylbenzoyl)-N'-(3-toluoyl)hydrazine
N-allyl-N'-isopropyl-N-(4-toluoyl)-N'-(4-chlorobenzoyl)hydrazine
N-allyl-N'-(1,2,2-trimethylpropyl)-N-(3-methoxybenzoyl)-N'-benzoylhydrazine
N-propyl-N'-isopropyl-N-(4-methylthiobenzoyl)-N'-(3,4-dichlorobenzoyl)hydrazine
N-propargyl-N'-t-butyl-N-(4-ethylbenzoyl)-N'-(3,5-dimethylbenzoyl)hydrazine
N-methoxymethyl-N'-t-butyl-N-(4-iodobenzoyl)-N'-(3,5-dichlorobenzoyl)hydrazine
N-methoxymethyl-N'-t-butyl-N-(4-propylbenzoyl)-N'-(4-chlorobenzoyl)hydrazine
N-methoxymethyl-N'-t-butyl-N-(4-vinylbenzoyl)-N'-(3,5-dichlorobenzoyl)hydrazine
N-methylthiomethyl-N'-t-butyl-N-(4-toluoyl)-N'-(2-nitrobenzoyl)hydrazine
N-methylthiomethyl-N'-t-butyl-N-(2-chlorobenzoyl)-N'-(3-toluoyl)hydrazine
N-methylthiomethyl-N'-isopropyl-N-(anisoyl)-N'-benzoylhydrazine
N-phenylthiomethyl-t-butyl-N-(4-toluoyl)-N'-(3,4-dichlorobenzoyl)hydrazine
N-(trimethylsilyloxymethyl)-N'-(4-ethylbenzoyl)-N'-(2-chloro-5-methylbenzoyl)hydrazine

Because of their good insecticidal activity, preferred compounds of the present invention for use in the insecticidal compositions and formulations include those where, independently

| | |
|---|---|
| X and X' are | O or S; and/or |
| $R^1$ is | unsubstituted ($C_3$-$C_8$) branched alkyl; |
| $R^2$ is | ($C_1$-$C_4$)alkyl; ($C_1$-$C_4$)alkoxy-($C_1$-$C_4$)alkyl having independently the stated number of |

carbon atoms in each alkyl group; methylthiomethyl; $(C_2-C_5)$alkenyl; $(C_2-C_5)$alkynyl; or benzyl where the phenyl ring is unsubstituted or substituted with halo, nitro, $(C_1-C_4)$alkyl or $(C_1-C_4)$alkoxy;
and/or

A and B are the same or different
unsubstituted naphthyl; or
unsubstituted or substituted phenyl where the substituents can be from one to three of the same or different halo; nitro; cyano; $(C_1-C_4)$alkyl; $(C_1-C_4)$haloalkyl; $(C_1-C_4)$-cyanoalkyl; $(C_1-C_4)$alkoxy; $(C_1-C_4)$haloalkoxy; -COD group; carboxy; $(C_1-C_4)$alkoxy-carbonyl; $(C_1-C_4)$alkanoyloxy; $(C_2-C_6)$alkenyl; $(C_2-C_6)$alkadienyl; $(C_2-C_6)$alkynyl; -NDD' group; thiocyanato; $(C_1-C_4)$alkylthio; -CSD group, $-CS_2D$ group; -SCOD group; tri$(C_1-C_4)$alkylsilyl having independently the stated number of carbon atoms in each alkyl group; unsubstituted or substituted phenyl having one to two of the same or different halo, nitro, $(C_1-C_4)$alkyl, $(C_1-C_4)$alkoxy, carboxy, $(C_1-C_4)$alkoxy-carbonyl, $(C_1-C_4)$-alkanoyloxy, $NH_2$, NHZ or NZZ'; phenoxy where the phenyl ring is unsubstituted or substituted with one or two of the same or different halo, nitro, $(C_1-C_4)$alkyl, $(C_1-C_4)$-alkoxy, carboxy, $(C_1-C_4)$alkoxy-carbonyl, $(C_1-C_4)$alkanoyloxy, $NH_2$, NHZ or NZZ'; or when two adjacent positions on the phenyl ring are substituted with alkoxy groups, these groups may be joined to form, together with the carbon atoms to which they are attached, a 5- or 6-membered dioxolano or dioxano heterocyclic ring;

where D and D' are hydrogen or $(C_1-C_4)$alkyl; Z and Z' are $(C_1-C_4)$alkyl;
and agronomically acceptable salts thereof.

More preferred insecticidal compounds of the present invention having very good activity for use in the insecticidal compositions and formulations of the present invention include those where, independently,

X and X' are O or S; and/or
$R^1$ is $(C_4-C_7)$ branched alkyl;
$R^2$ is methyl; ethyl; $(C_1-C_2)$alkoxy-$(C_1-C_2)$alkyl having independently the stated number of carbon atoms in each alkyl group; $(C_2-C_5)$alkenyl; $(C_2-C_5)$alkynyl; or benzyl where the phenyl ring is unsubstituted or substituted with halo; and/or
A and B are the same or different
unsubstituted naphthyl;
unsubstituted or substituted phenyl having one to three of the same or different halo; nitro; cyano; $(C_1-C_4)$alkyl; $(C_1-C_4)$haloalkyl; $(C_1-C_4)$cyanoalkyl; $(C_1-C_4)$alkoxy; $(C_1-C_4)$-alkoxy-$(C_1-C_4)$alkyl having independently the stated number of carbon atoms in each alkyl group; -COD group; $(C_1-C_4)$alkoxy-carbonyl; $(C_1-C_4)$alkanoyloxy; thiocyanato; unsubstituted or substituted phenyl having one or two of the same or different halo, nitro, $(C_1-C_4)$alkyl, $(C_1-C_4)$alkoxy, carboxy, $(C_1-C_4)$alkoxy-carbonyl, $(C_1-C_4)$alkanoyloxy, $NH_2$, NHZ or NZZ'; or phenoxy where the phenyl ring is unsubstituted or substituted with one or two of the same or different halo, nitro, $(C_1-C_4)$alkyl, $(C_1-C_4)$alkoxy, carboxy, $(C_1-C_4)$-alkoxy-carbonyl, $(C_1-C_4)$alkanoyloxy, $NH_2$, NHZ or NZZ';

where D is hydrogen or $(C_1-C_4)$alkyl; Z and Z' are $(C_1-C_4)$alkyl; and
agronomically acceptable salts thereof.

Because of their excellent insecticidal activity, very preferred compounds of the present invention include those where, independently,

X and X' are O; and/or
$R^1$ is t-butyl; neopentyl (2,2-dimethylpropyl) or 1,2,2-trimethylpropyl; and/or
$R^2$ is methyl; methoxymethyl; $(C_2-C_4)$alkenyl; $(C_2-C_5)$alkynyl; benzyl or 4-halobenzyl; and/or
A and B are the same or different phenyl or substituted phenyl where the substituents can be from one to three of the same or different halo, nitro, $(C_1-C_4)$alkyl, $(C_1-C_4)$alkoxy, or $(C_1-C_4)$-haloalkyl; and

agronomically acceptable salts thereof.

Because of their outstanding insecticidal activity, particularly preferred compounds of the present invention include those where, independently,

X and X' are O; and/or
$R^1$ is t-butyl; and/or
$R^2$ is $(C_2-C_5)$alkynyl; and/or
A and B are unsubstituted or substituted phenyl where the substituents can be one or two of the same or different chloro, fluoro, bromo, iodo, methyl, ethyl, methoxy or trifluoromethyl;

and

agronomically acceptable salts thereof.

Those N-substituted-N'-substituted-N,N'-diacyl hydrazines of Formula I which possess acidic or basic functional groups may be further reacted to form novel salts with appropriate bases or acids. These salts also exhibit insecticidal activity. Typical salts are the agronomically acceptable metal salts, ammonium salts and acid addition salts. Among the metal salts are those in which the metal cation is an alkali metal cation such as sodium, potassium, lithium or the like; alkaline earth metal cation such as calcium, magnesium, barium, strontium, or the like; or heavy metal cation such as zinc, manganese, cupric, cuprous, ferric, ferrous, titanium, aluminum or the like. The ammonium salts include those in which the ammonium cation has the formula $NR^5R^6R^7R^8$ wherein each of $R^5$, $R^6$, $R^7$ and $R^8$ is independently hydrogen, hydroxy, ($C_1$-$C_4$)alkoxy, ($C_1$-$C_{20}$)alkyl, ($C_3$-$C_8$)alkenyl, ($C_3$-$C_8$)alkynyl, ($C_2$-$C_8$)hydroxyalkyl, ($C_2$-$C_8$)alkoxyalkyl, ($C_2$-$C_6$)-aminoalkyl, ($C_2$-$C_6$)haloalkyl, amino, ($C_1$-$C_4$)alkyl- or di-($C_1$-$C_4$)alkylamino, substituted or unsubstituted phenyl, substituted or unsubstituted phenylalkyl, having up to four carbon atoms in the alkyl moiety, or any two of $R^5$, $R^6$, $R^7$ or $R^8$ can be taken together to form with the nitrogen atom a 5- or 6-membered heterocyclic ring, optionally having up to one additional hetero atom (e.g., oxygen, nitrogen, or sulfur) in the ring, and preferably saturated, such as piperidino, morpholino, pyrrolidino, piperazino or the like, or any three of $R^5$, $R^6$, $R^7$ or $R^8$ can be taken together to form with the nitrogen atom a 5- or 6-membered aromatic heterocyclic ring, such as piperazole or pyridine. When $R^5$, $R^6$, $R^7$ or $R^8$ substituent in the ammonium group is a substituted phenyl or substituted phenylalkyl, the substituents on the phenyl and phenalkyl will generally be selected from halo, ($C_1$-$C_8$)alkyl, ($C_1$-$C_4$)alkoxy, hydroxy, nitro, trifluoromethyl, cyano, amino, ($C_1$-$C_4$)alkylthio and the like. Such substituted phenyl groups preferably have up to two such substituents. Representative ammonium cations include ammonium, dimethylammonium, 2-ethylhexylammonium, bis(2-hydroxyethyl)ammonium, tris(2-hydroxyethyl)ammonium, dicyclohexylammonium, t-octylammonium, 2-hydroxyethylammonium, morpholinium, piperidinium, 2-phenethylammonium, 2-methylbenzylammonium, n-hexylammonium, triethylammonium, trimethylammonium, tri(n-butyl)ammonium, methoxyethylammonium, diisopropylammonium, pyridinium, dialkylammonium, pyrazolium, propargylammonium, dimethylhydrazinium, octadecylammonium, 4-dichlorophenylammonium, 4-nitrobenzylammonium, benzyltrimethylammonium, 2-hydroxy-ethyldimethyloctadecylammonium, 2-hydroxyethyldiethyloctylammonium, decyltrimethylammonium, hexyltriethylammonium, 4-methylbenzyltrimethylammonium, and the like. Among the acid addition salts are those in which the anion is an agronomically acceptable anion such as hydrochloride, hydrobromide sulfate, nitrate, perchlorate, acetate, oxalate and the like.

The compounds of this invention or their precursors can be prepared by reacting a suitably substituted hydrazine (Formula II) with an alkyl halide, allyl halide or phenylmethylhalide in the presence of a base in an inert or substantially inert solvent or mixture of solvents according to the following process:

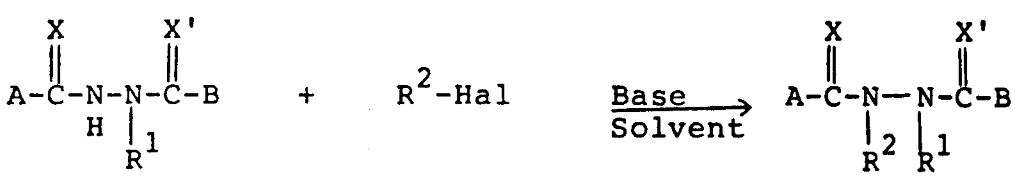


II III I

where X, X', $R^1$ $R^2$, A and B are as defined above for Formula I and Hal is halogen (chloro, fluoro or bromo).

Suitable bases for use in the above process include metal hydrides such as sodium hydride or potassium hydride; metal alkoxides such as sodium alkoxides or potassium alkoxides; sodium hydroxide; potassium hydroxide; or lithium diisopropyl amide. If desired, mixtures of these bases may be used. The preferred base is potassium t-butoxide.

Suitable solvents for use in the above process include ethers such as tetrahydrofuran (THF), glyme and the like; dimethylformamide (DMF); dimethylsulfoxide (DMSO); acetonitrile; or a mixture of water and benzene or toluene. If desired, mixtures of these solvents may be used. The preferred solvent is dimethylformamide.

The above process can be carried out at temperatures between about -20 °C and about 100 °C. Preferably, this reaction is carried out between about -5 °C and about 50 °C.

Preparation of the compounds of the present invention by the above process is preferably carried out at about atmospheric pressure although higher or lower pressures can be used if desired.

Substantially equimolar amounts of reactants are preferably used although higher or lower amounts can

be used if desired.

Generally one equivalent of base is used per equivalent of starting material of Formula III.

The compounds of Formula III are generally commercially available or can be prepared by known procedures.

Modifications to the above process may be necessary to accommodate reactive functionalities of particular substituents. Such modifications would be apparent and known to those skilled in the art.

The compounds of Formula II or their precursors can be prepared according to the following processes. Process A can be used when preparing compounds according to Formula II where X and X' are both oxygen and A and B are the same (for example, both A and B are phenyl or 4-chlorophenyl) or different (for example, A is 4-methylphenyl and B is 4-bromophenyl).

Process A:

Step 1

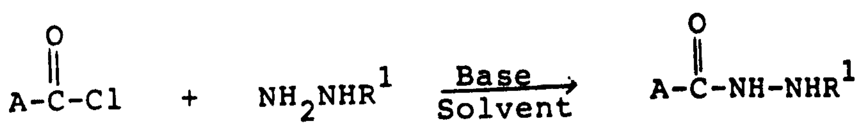


IV V VI

Step 2

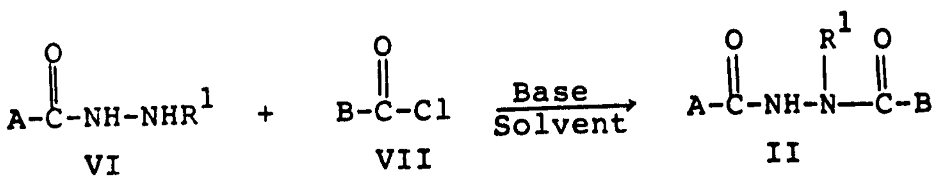


VI VII II

where R[1], A and B are as defined above for Formula I and X and X' are oxygen.

Process B can be used when preparing compounds according to Formula II where X and X' are oxygen, and R[1], A and B are as defined above for Formula I.

Process B:

Step 1

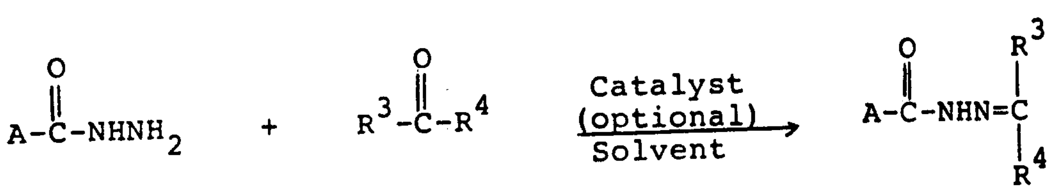


VIII IX X

Step 2

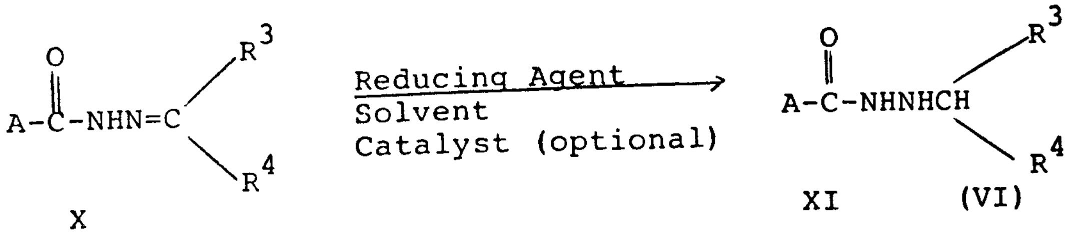


X XI (VI)

Step 3

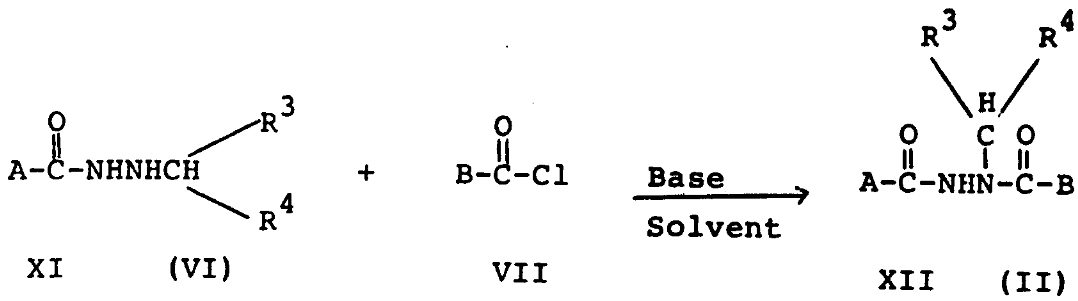


XI (VI) VII XII (II)

where X and X' are oxygen, A and B are as defined above for Formula I, and $R^3$ and $R^4$ are the same or different hydrogen or ($C_2$-$C_9$) straight or branched chain unsubstituted or substituted alkyl have one or two of the same or different ($C_3$-$C_6$)cycloalkyl provided that $R^3$ and $R^4$ are not both H or $R^3$ or $R^4$ is not a straight chain alkyl group when the other ($R^3$ or $R^4$) is hydrogen. As can be seen above, the intermediate product of Step 2, the compounds of Formula XI, corresponds to the compounds of Formula VI. In addition, the compound of Formula XII corresponds to the compounds of Formula II where X and X' are oxygen.

Process C can be used when preparing compounds according to Formula I where A, B and $R^1$ are as defined for Formula I and one or both X and X' are sulfur.

Process C:

Step 1:

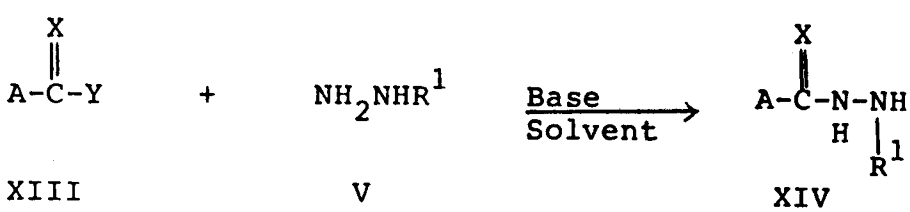


XIII V XIV

Step 2:

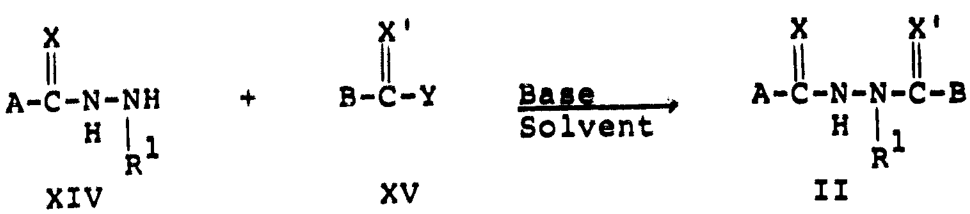


XIV XV II

where A, B and $R^1$ are as defined above for Formula I and one or both X and X' are sulfur, and Y is a good

leaving group such as carboxyalkylthio (for example, carboxymethylthio, $-SCH_2CO_2H$); alkylthio (for example, methylthio); or halo (for example, chloro).

In Process A, a compound of Formula IV is reacted with a monosubstituted hydrazine of Formula V or a corresponding acid addition salt such as the hydrochloride salt or the like in the presence of a base in an inert or substantially inert solvent or mixture of solvents to afford an intermediate product of Formula VI which can be isolated or further reacted with a compound of Formula VII in the presence of a base in an inert or substantially inert solvent or mixture of solvents to afford the compounds of Formula II.

When A and B are the same, for example, both A and B are 4-chlorophenyl, two equivalents of a compound of Formula IV or VII are reacted with a monosubstituted hydrazine of Formula V in the presence of a base in an inert or substantially inert solvent or mixture of solvents to afford the compounds of Formula II.

Examples of the compounds of Formula IV and/or Formula VII which can be used in the above processes include benzoyl chloride, 4-chlorobenzoyl chloride, 4-methylbenzoyl chloride, 3,5-dichlorobenzoyl chloride, 2-bromobenzoyl chloride, 3-cyanobenzoyl chloride and the like. The compounds of Formula IV and/or Formula VII are generally commercially available or can be prepared by known procedures.

Examples of the compounds of Formula V which can be used in the above processes include isopropylhydrazine, t-butylhydrazine, neopentylhydrazine, alphamethylneopentylhydrazine, isobutylhydrazine, isopentylhydrazine, isooctylhydrazine, and the like. The compounds of Formula V are generally commercially available or can be prepared by known procedures.

Suitable solvents for use in the above processes include water; alcohols such as methanol, ethanol, isopropanol and the like; hydrocarbons such as toluene, xylene, hexane, heptane and the like; glyme; tetrahydrofuran; acetonitrile; pyridine; or haloalkanes such as methylene chloride or mixtures of these solvents.

Preferred solvents are water, toluene, methylene chloride or a mixture of these solvents.

Examples of bases for use in the above processes include tertiary amines such as triethylamine; pyridine; potassium carbonate; sodium carbonate; sodium bicarbonate; sodium hydroxide; or potassium hydroxide. Preferred bases are sodium hydroxide, potassium hydroxide or triethylamine.

In process B, a compound of Formula VIII is reacted with a ketone or aldehyde of Formula IX in an inert or substantially inert solvent or mixture of solvents and optionally in the presence of a catalyst to afford an intermediate product of Formula X. The intermediate product of Formula X is then further reacted with a reducing agent in an inert or substantially inert solvent or mixture of solvents to afford a second intermediate product of Formula XI (VI) which can be isolated or further reacted with a compound of Formula VII in the presence of a base in an inert or substantially inert solvent or mixture of solvents to afford the compound of Formula II.

Examples of the compounds of Formula VIII which can be used in the above Process B include benzoylhydrazine, 4-chlorobenzoylhydrazine, 2-methylbenzoylhydrazine, 4-methylbenzoylhydrazine, 3,5-dichlorobenzoylhydrazine and the like. The compounds of Formula VIII are generally commercially available or can be prepared by known procedures.

Examples of the compounds of Formula IX which can be used in the above Process B include 1,1,1-trimethylacetaldehyde, methylethylketone, diethylketone and the like. The compounds of Formula IX are generally commercially available or can be prepared by known procedures.

Optionally, a catalyst may be used in Step 1 of Process B. Suitable catalysts generally include organic acids such as acetic acid, trifluoroacetic acid, oxalic acid and the like; mineral acids such as hydrochloric acid, sulfuric acid, nitric acid and the like; arylsulfonic acids such as toluenesulfonic acid; or pyridinium toluenesulfonate. Preferred catalysts are organic acids or arylsulfonic acids. Most preferred catalysts are acetic acid or trifluoroacetic acid.

Suitable solvents for use in the above Process B, Step 1, include alcohols such as methanol, ethanol, isopropanol and the like; hydrocarbons such as toluene, benzene; ethers such as tetrahydrofuran (THF), glyme and the like; or dimethylformamide. Preferred solvents are alcohols and hydrocarbons. Most preferred solvents are alcohols such as methanol or ethanol.

Examples of suitable reducing agents for use in the above Process B, Step 2, include hydrides such as sodium borohydride and derivatives thereof such as sodium cyanoborohydride, lithium aluminum hydride and derivatives thereof and the like; or diborane. Preferred reducing agents are sodium borohydride and derivatives thereof or lithium aluminum hydride and derivatives thereof. Most preferred as a reducing agent is sodium cyanoborohydride.

Optionally, in Process B, Step 2, a catalyst may be included. Examples of suitable catalysts include organic acids such as acetic acid, trifluoroacetic acid; or mineral acids such as hydrochloric acid, sulfuric

acid and the like. Preferred catalysts are organic acids or hydrochloric acid. Most preferred catalysts are acetic acid, trifluoroacetic acid or hydrochloric acid.

Suitable solvents for use in the above Process B, Step 2, include alcohols such as methanol, ethanol, isopropanol and the like; ethers such as tetrahydrofuran (THF), diethylether, glyme and the like; or halohydrocarbons such as methylene chloride, chloroform and the like. Preferred solvents are alcohols and most preferred are methanol or ethanol.

Step 3 of Process B corresponds to Step 2 of Process A. Consequently, those bases and solvents suitable for use in Step 2 of Process A are suitable for use in Step 3 of Process B including the preferred bases and solvents described above.

In Process C, a compound of Formula XIII is reacted with a monosubstituted hydrazine of Formula V or a corresponding acid addition salt such as the hydrochloride salt or the like in the presence of a base in an inert or substantially inert solvent or mixture of solvents to afford an intermediate compound of Formula XIV which can be isolated or further reacted with a compound of Formula XV in the presence of a base in an inert or substantially inert solvent or mixture of solvents to afford the desired product of Formula II.

When A and B are the same, for example, both A and B are unsubstituted phenyl, two equivalents of a compound Formula XIII or XV are reacted with a monosubstituted hydrazine of Formula III in the presence of a base in an inert or substantially inert solvent or mixture of solvents to afford the desired product of Formula II.

Examples of the compounds of Formula XIII and/or Formula XV which can be used in the above Process C include 3-methyl-methylthio-thiobenzoate, 4-chloromethylthio-thiobenzoate, 4-methyl-methylthio-thiobenzoate, carboxymethylthio-thiobenzoate and the like. The compounds of Formula XIII and/or Formula XV are generally commercially available or can be prepared by known procedures.

Suitable solvents for use in the above Process C are generally polar high-boiling solvents such as dimethylformamide (DMF); glyme; tetrahydrofuran (THF); and pyridine. The preferred solvent is pyridine.

Suitable bases for use in the above Process C include tertiary amines such as triethylamine; and pyridine. The preferred base is pyridine.

The above Processes A and B can be carried out at temperatures between about -20 ° C and about 100 ° C. Preferably, these reactions are carried out between about -5 ° C and about 50 ° C.

Process C can be carried out at temperatures between about 10 ° C and 200 ° C. Preferably, this reaction is carried out between about 70 ° C and about 100 ° C.

Preparation of the compounds of the present invention by Processes A, B and C is generally carried out at about atmospheric pressure, although higher or lower amounts can be used if desired.

Substantially equimolar amounts of reactants are preferably used in Processes A, B and C, although higher or lower amounts can be used if desired.

Generally, when preparing the compounds of Formula II, about one equivalent of base is used per equivalent of starting material of Formula IV, VII, XIII and/or XV. Where the acid addition salt of the monosubstituted hydrazine of Formula V is used, one additional equivalent of base is used. For example, in Process A, when substituents A and B are the same and a monosubstituted hydrazine is used, about two equivalents of base are used since about two equivalents of a suitably substituted benzoyl chloride of Formula IV or VII are employed. In Process A, when substituents A and B are different and an acid addition salt of the monosubstituted hydrazine of Formula V is used, about two equivalents of base are used in Step 1 and about one equivalent of base is used in Step 2.

Modifications to the above processes may be necessary to accommodate reactive functionalities of particular A and/or B substituents. Such modifications would be apparent and known to those skilled in the art.

Alternatively, the compounds of the invention or their precursors can be prepared by substantially following the procedures described above for Process A except a disubstituted hydrazine $NH(R^2)NH(R^1)$ (Formula XVI) where $R^1$ and $R^2$ are as defined above for Formula I is employed rather than the monosubstituted hydrazine of Formula V.

The disubstituted hydrazines of Formula XVI are generally commercially available or can be prepared by known procedures. Those bases and solvents suitable for use in Process A are suitable for use in this alternative process including the preferred bases and solvents described. Substantially the same temperatures, pressures and amounts of materials are used, as described above for Process A.

The agronomically acceptable salts embraced by Formula I of the invention can be prepared by reacting a metal hydroxide, a metal hydride or an amine or ammonium salt, such as a halide, hydroxide or alkoxide with a compound of Formula I having one or more hydroxy or carboxy groups or reacting a quaternary ammonium salt, such as chloride, bromide, nitrate or the like with a metal salt or a compound of Formula I in a suitable solvent. When metal hydroxides are used as reagents, useful solvents include water;

ethers such as glyme and the like; dioxane; tetrahydrofuran; alcohols such as methanol, ethanol, isopropanol and the like. When metal hydrides are used as reagents, useful solvents include nonhydroxylic solvents, for example, ethers such as dioxane, glyme, diethylether and the like; tetrahydrofuran; hydrocarbons such as toluene, xylene, hexane, pentane, heptane, octane and the like; dimethylformamide, and the like. When amines are used as reagents, useful solvents include alcohols, such as methanol or ethanol; hydrocarbons, such as toluene, xylene, hexane and the like; tetrahydrofuran; glyme; dioxane; or water. When ammonium salts are used as reagents, useful solvents include water; alcohols, such as methanol or ethanol; glyme, tetrahydrofuran; or the like. When the ammonium salt is other than a hydroxide or alkoxide, an additional base, such as potassium or sodium hydroxide, hydride, or alkoxide is generally used. The particular choice of solvent will depend on the relative solubilities of the starting materials and the resultant salts, and slurries rather than solutions of certain reagents may be used to obtain the salts. Generally, equivalent amounts of the starting reagents are used and the salt-forming reaction is carried out at about 0 ° C to about 100 ° C, preferably at about room temperature.

The acid addition salts of the present invention can be prepared by reacting hydrochloric, hydrobromic, sulfuric, nitric, phosphoric, acetic, propionic, benzoic or other suitable acid with a compound of Formula I having a base functional group in a suitable solvent. Useful solvents include water, alcohols, ethers, esters, ketones, haloalkanes and the like. The particular choice of solvent will depend on the relative solubilities of the starting materials and the resulting salts and slurries rather than solutions of certain reagents may be used to obtain the salts. Generally, equivalent molar amounts of starting materials are used and the salt-forming reaction is carried out at from about -10 ° C to about 100 ° C, preferably at about room temperature.

The following Examples will further illustrate this invention but are not intended to limit it in any way. In Table I, some N-substituted-N'-substituted-N,N'-diacyl-hydrazinesof the present invention that have been made are listed. Structures were confirmed by NMR and in some cases by IR and/or elemental analysis. Specific illustrative preparation of the compounds of Examples 1, 2, 3, 4, 5 and 8 are described after Table I.

## TABLE I

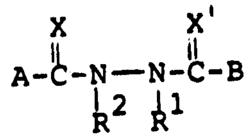

| Ex. No. | X | X' | $R^1$ | $R^2$ | A | B |
|---|---|---|---|---|---|---|
| 1 | O | O | $-C(CH_3)_3$ | $CH_3$ | $C_6H_5$ | $C_6H_5$ |
| 2 | O | O | $-C(CH_3)_3$ | $-CH_2C_6H_5$ | $C_6H_5$ | $C_6H_5$ |
| 3 | O | O | $-C(CH_3)_3$ | $-CH_2CH{=}CH_2$ | $C_6H_5$ | $C_6H_5$ |
| 4 | O | O | $-C(CH_3)_3$ | $-CH_2OCH_3$ | $C_6H_5$ | $C_6H_5$ |
| 5 | O | O | $-C(CH_3)_3$ | $-CH_2SCH_3$ | $C_6H_5$ | $C_6H_5$ |
| 6 | O | O | $-C(CH_3)_3$ | $-CH_2C{\equiv}CH$ | $C_6H_5$ | $C_6H_5$ |
| 7 | O | O | $-C(CH_3)_3$ | $-CH_2C{\equiv}CH$ | $C_6H_4CH_3$-4 | $C_6H_4CH_3$-3 |
| 8 | O | O | $-C(CH_3)_3$ | $-CH_2C{\equiv}CH$ | $C_6H_5$ | $C_6H_3(CH_3)_2$-3,5 |
| 9 | O | O | $-C(CH_3)_3$ | $-CH_2C{\equiv}CH$ | $C_6H_5$ | $C_6H_3Cl_2$-2,4 |
| 10 | O | O | $-C(CH_3)_3$ | $-CH_2C{\equiv}CH$ | $C_6H_5$ | $C_6H_3Cl_2$-3,4 |
| 11 | O | O | $-C(CH_3)_3$ | $-CH_2C_6H_4Br$-4 | $C_6H_5$ | $C_6H_5$ |

**EXAMPLE No. 1 - Preparation of N-methyl-N'-t-butyl-N,N'-dibenzoylhydrazine**

To a stirred solution of N'-t-butyl-N,N'-dibenzoylhydrazine (2.5 g, 0.008 M) in dimethylformamide (DMF) (30 ml) at room temperature under nitrogen was added portionwise sodium hydride (60% oil dispersion) (0.4 g, 0.009 M). The mixture was stirred at room temperature for 0.5 hours, and then methyl iodide (1.0 g, 0.008 M) was added dropwise. The reaction mixture was allowed to stir for 1 hour. The mixture was then diluted with water (50 ml), neutralized with 10% HCl, and the product extracted into methylene chloride (50 ml). The methylene chloride layer was washed with water (5 x 20 ml), dried over anhydrous magnesium sulfate, and the methylene chloride removed under vacuum to afford N-methyl-N'-t-butyl-N,N'-dibenzoylhydrazine as an oil.

**EXAMPLE No. 2 - Preparation of N-benzyl-N'-t-butyl-N,N'-dibenzoylhydrazine**

To a stirred solution of N'-t-butyl-N,N'-dibenzoylhydrazine (2 g, 0.006 M) in DMF (25 ml) at room temperature under nitrogen was added portionwise sodium hydride (60% oil dispersion) (0.3 g, 0.007 M). The mixture was stirred at room temperature for 0.5 hours, and then benzyl bromide (1.2 g, 0.007 M) was added dropwise. The reaction mixture was warmed to 60 °C and allowed to stir for 2 hours. The mixture was then diluted with water (50 ml), neutralized with 1% HCl, and the product extracted into methylene chloride (50 ml). The methylene chloride layer was washed with water (5 x 20 ml), dried over anhydrous magnesium sulfate, and the methylene chloride removed under vacuum to afford N-benzyl-N'-t-butyl-N,N'-dibenzoylhydrazine as an oil.

**EXAMPLE No.3 - Preparation of N-allyl-N'-t-butyl-N,N'-dibenzoylhydrazine**

To a stirred solution of N'-t-butyl-N,N'-dibenzoylhydrazine (3 g, 0.011 M) in DMF (30 ml) at room temperature under nitrogen was added portionwise sodium hydride (50% oil dispersion) (0.5 g, 0.012 M). The mixture was stirred at room temperature for 0.5 hours, and then allyl iodide (1.8 g, 0.01 M) was added dropwise. The reaction mixture was warmed to 60 °C and stirred for 2 hours. The mixture was then diluted with water (50 ml), neutralized with 10% HCl, and the product extracted into methylene chloride (50 ml). The methylene chloride layer was washed with water (5 x 20 ml), dried over anhydrous magnesium sulfate, and the methylene chloride removed under vacuum to afford N-allyl-N'-t-butyl-N,N'-dibenzoylhydrazine as an oil

**EXAMPLE No. 4 - Preparation of N-methylthiomethyl-N'-t-butyl-N-N'-dibenzoylhydrazine**

N'-t-butyl-N,N'-dibenzoylhydrazine (2 g, 0.007 M) was stirred at room temperature in a two phase system of toluene-50% sodium hydroxide with 100 mg of phase transfer catalyst (tetra-n-butylammonium hydrogen sulfate). Methoxymethyl chloride (1.2 g, 0.015 M) was added dropwise and the mixture was stirred 3 hours. The layers were separated and the toluene layer was washed several times with water (until the water washes were neutral). The toluene solution was dried over anhydrous magnesium sulfate and the toluene removed under vacuum to afford N-methoxymethyl-N'-t-butyl-N,N'-dibenzoylhydrazine as a thick oil.

**EXAMPLE No. 5 - Preparation of N-methylthiomethyl-N'-t-butyl-N-N'-dibenzoylhydrazine**

To a stirred suspension of sodium hydride (a 50% oil dispersion washed 2 times with 20 ml pentane) (0.21 g, 0.0043 M) in dry DMF (20 ml) under nitrogen at room temperature, was added N'-t-butyl-N,N'-dibenzoylhydrazine (1 g, 0.0034 M) portionwise as a solid. The mixture was stirred at room temperature for 1/2 hour and methylthiomethylchloride (0.34 g, 0.0035 M) was added dropwise. The resulting mixture was heated at 50 °C overnight, cooled, diluted with methylene chloride and washed repeatedly with water. The organic layer was dried over anhydrous magnesium sulfate and the methylene chloride removed under vacuum. The oily residue was chromatographed on silica gel using methylene chloride to afford N-methylthiomethyl-N'-t-butyl-N,N'-dibenzoylhydrazine as an oil (60% yield).

EXAMPLE NO. 8 - Preparation of N-(2-propynyl)-N'-t-butyl-N-benzoyl-N'-(3,5-dimethylbenzoyl)hydrazine

To a stirred suspension of N'-t-butyl-N-benzoyl-N'-(3,5-dimethylbenzoyl)hydrazine (1.5 g) in dimethylformamide (DMF) (20 ml) was added sodium hydride (200 mg of 60% oil dispersion) portionwise. After 15 min., propargyl bromide (0.6 g) was added to the reaction mixture dropwise and the reaction stirred for 1 hour. The reaction mixture was diluted with ethyl acetate (50 ml) and washed with water (5 x 20 ml). The organic layer was then dried over magnesium sulfate and the solvent removed under vacuum to afford N-(2-propynyl)-N'-t-butyl-N-benzoyl-N'-(3,5-dimethylbenzoyl)hydrazine as a yellow amorphous solid. The product, N-(2-propynyl)-N'-t-butyl-N-benzoyl-N'-(3,5-dimethylbenzoyl)hydrazine, was purified by column chromatography on silica gel (solvent system : methylene chloride) to afford a 70% yield as a white solid.

By following substantially the processes for preparing the compounds of the present invention as described above and as exemplified by the illustrative preparation of the compounds of Examples 1, 2, 3, 4, 5 and 8, the compounds of Formula I are prepared.

As previously noted, compounds of the present invention exhibit excellent insecticidal activity and are selective against insects of the orders Lepidoptera and Coleoptera.

In general, for the control of insects in agriculture, horticulture and forestry, a dosage corresponding to from about 10 grams to about 10 kilograms of the active substance per hectare may be used and from about 100 grams to about 5 kilograms per hectare of the active substance is preferred. The exact amount of dosage for a given situation can be routinely determined and depends on a variety of factors, for example, the substance used, the kind of pest, the formulation used, the state of the crop infested with the pest and the prevailing weather conditions. The term "insecticidal" as employed in the specification and claims of this application is to be construed as any means which adversely affects the existence or growth of the target insects. Such means can comprise a complete killing action, eradication, arresting in growth, inhibition, reducing in number or any combination thereof. The term "control" or "combat" as employed in the specification and claims of this application is to be construed as extending to "insecticidal" use and to the protection of plants from insect damage. By "insecticidally active amount" is meant that dosage of active substance sufficient to exert insect "control".

The compounds of the present invention, for practical applications, can be utilized in the form of compositions or formulations. Examples of the preparation of compositions and formulations can be found in the American Chemical Society publication "Pesticidal Formulation Research," (1969), Advances in Chemistry Series No. 86, written by Wade Van Valkenburg; and the Marcel Dekker, Inc. publication "Pesticide Formulations," (1973), edited by Wade Van Valkenburg. In these compositions and formulations, the active substance is mixed with conventional inert agronomically acceptable (i.e., plant compatible and/or pesticidally inert) diluents or extenders such as solid carrier material or liquid carrier material, of the type usable in conventional compositions or formulations. By agronomically acceptable carrier is meant any substance which can be used to dissolve, disperse or diffuse the active ingredient in the composition without impairing the active ingredient's effectiveness and which by itself has no significant detrimental effect on the soil, equipment, desirable plants or agronomic environment. If desired, adjuvants such as surfactants, stabilizers, antifoam agents and antidrift agents may also be added.

Examples of compositions and formulations according to the invention are aqueous solutions and dispersions, oily solutions and oil dispersions, pastes, dusting powders, wettable powders, emulsifiable concentrates, flowables, granules, baits, invert emulsions, aerosol compositions and fumigating candles.

Wettable powders, pastes, flowables and emulsifiable concentrates are concentrated preparations which are diluted with water before or during use.

Baits are preparations generally comprising a food or other substance attractive to the target pest, that includes at least one lethal or non-lethal toxicant. Lethal toxicants kill the pest upon ingesting the bait while non-lethal toxicants change the behavior, feeding habits and physiology of the pest for the purpose of control.

The invert emulsions are mainly used for aerial application, where large areas are treated with a comparatively small amount of preparation. The invert emulsion may be prepared in the spraying apparatus shortly before, or even during, the spraying operation by emulsifying water in an oil solution or an oil dispersion of the active substance.

Compositions and formulations are prepared in a known manner, for instance by extending the active compounds with conventional dispersible liquid diluent carriers and/or dispersible solid carriers optionally with the use of carrier vehicle assistants, e.g., conventional surface-active agents, including emulsifying agents and/or dispersing agents, whereby, for example, in the case where water is used as diluent, organic solvents may be added as auxiliary solvents. The following may be chiefly considered for use as conventional carrier vehicles for this purpose: aerosol propellants which are gaseous at normal temperatures and pressures, such as halogenated hydrocarbons, e.g., dichlorodifluoromethane and trifluorochloromethane, as well as butane, propane, nitrogen and carbon dioxide; inert dispersible liquid diluent carriers, including inert organic solvents, such as aromatic hydrocarbons (e.g., benzene, toluene, xylene, alkyl naphthalenes, etc.), halogenated, especially chlorinated, aromatic hydrocarbons (e.g., chlorobenzenes, etc.), cycloalkanes (e.g., cyclohexane, etc.), paraffins (e.g., petroleum or mineral oil fractions), chlorinated aliphatic hydrocarbons (e.g., methylene chloride, chloroethylenes, etc.), vegetable oils (e.g., soybean oil, cottonseed oil, corn oil, etc.), alcohols (e.g., methanol, ethanol, propanol, butanol, glycol, etc.) as well as

ethers and esters thereof (e.g., glycol monomethyl ether, etc.), amines (e.g., ethanolamine, etc.), amides (e.g., dimethyl formamide, etc.), sulfoxides (e.g., dimethyl sulfoxide, etc.), acetonitrile, ketones (e.g., acetone, methyl ethyl ketone, methyl isobutyl ketone, cyclohexanone, isophorone, etc.), and/or water; solid carriers including ground natural minerals, such as kaolins, clays, talc, chalk, quartz, attapulgite, montmorillonite or diatomaceous earth, and ground synthetic minerals, such as highly-dispersed silicic acid, alumina and silicates; solid carriers for granules include crushed and fractionated natural rocks such as calcite, marble, pumice, sepiolite and dolomite, as well as synthetic granules of inorganic and organic meals, and granules of organic material such as sawdust, coconut shells, corn cobs and tobacco stalks. The following may be chiefly considered for use as conventional carrier vehicle assistants: emulsifying agents, such as cationic and/or nonionic and/or anionic emulsifying agents (e.g., polyethylene oxide esters of fatty acids, polyethylene oxide ethers of fatty alcohols, alkyl sulfates, alkyl sulfonates, aryl sulfonates, albumin hydrolysates, etc., and especially alkyl arylpolyglycol ethers, magnesium stearate, sodium oleate, etc.); and/or dispersing agents, such as lignin, sulfite waste liquors, methyl cellulose, etc.

Adhesives such as carboxymethylcellulose and natural and synthetic polymers in the form of powders, granules or latices, such as gum arabic, polyvinyl alcohol and polyvinyl acetate, can be used in the formulations.

If desired, it is possible to use colorants in compositions and formulations containing compounds of the present invention such as inorganic pigments, for example, iron oxide, titanium oxide and Prussion Blue, and organic dyestuffs, such as alizarin dyestuffs, azo dyestuffs and metal phthalocyanine dyestuffs, and trace nutrients such as salts of iron, manganese, boron, copper, cobalt, molybdenum and zinc.

The active compounds of the present invention may be employed alone or in the form of mixtures with one another and/or with such solid and/or liquid dispersible carrier vehicles and/or with other known compatible active agents, especially plant protection agents, such as other insecticides, arthropodicides, nematicides, fungicides, bactericides, rodenticides, herbicides, fertilizers, growth-regulating agents, synergists, etc., if desired, or in the form of particular dosage preparations for specific application made therefrom, such as solutions, emulsions, suspensions, powders, pastes, and granules which are thus ready for use.

As concerns commercially marketed preparations, these generally contemplate carrier composition mixtures in which the active compound is present in an amount substantially between about 0.1% and 99% by weight, and preferably between about 1% and 75% by weight, of the mixture. Carrier composition mixtures suitable for direct application or field application generally contemplate those in which the active compound is present in an amount substantially between about 0.0001% and 5%, preferably between about 0.001% and 3%, by weight of the mixture. Thus the present invention contemplates overall compositions which comprise mixtures of a conventional dispersible carrier such as (1) a dispersible inert finely divided carrier solid, and/or (2) a dispersible carrier liquid such as an inert organic solvent and/or water, preferably including a surface-active effective amount of a carrier vehicle assistant (e.g., a surface-active agent, such as an emulsifying agent and/or a dispersing agent), and an amount of the active compound, generally between about 0.0001% and about 99% by weight of the composition, preferably between about 0.001% and about 90% by weight of the composition, and more preferably between about 0.01% and about 75% by weight of the composition, which is effective for the purpose in question.

The active compounds can be applied as sprays by methods commonly employed, such as conventional high-gallonage hydraulic sprays, low gallonage sprays, ultra-low-volume sprays, airblast spray, aerial sprays and dusts. If low volume applications are desired, a solution of the compound is usually used. In ultra-low-volume applications, a liquid composition containing the active compound is usually applied as a spray (e.g., mist) by means of atomizing equipment in finely divided form (average particle size of from about 50 to about 100 microns or less) using airplane crop spraying techniques. Typically only a few liters per hectare are needed and often amounts up to about 15 to 1000 g/hectare, preferably about 40 to 600 g/hectare of active ingredient are sufficient. With ultra-low-volume, it is possible to use highly concentrated liquid compositions with said liquid carrier vehicles containing from about 20 to about 95% by weight of the active compound.

Furthermore, the present invention contemplates methods of selectively killing, combatting or controlling pests, which comprises contacting insects with a correspondingly combative or toxic amount (i.e., an insecticidally effective amount) of at least one active compound of the invention alone or together with a carrier vehicle (composition or formulation) as noted above. The term "contacting" as employed in the specification and claims of this application is to be construed as applying to at least one of (a) such insects and (b) the corresponding habitat thereof (i.e., the locus to be protected, for example, to a growing crop or other plant or an area where a crop or other plant is to be grown), the active compound of this invention alone or as a constituent of a composition or formulation. The instant formulations or compositions are

applied in the usual manner, for instance by spraying, atomizing, vaporizing, scattering, dusting, watering, squirting, sprinkling, pouring, fumigating, dry dressing, moist dressing, wet dressing, slurry dressing, encrusting and the like.

It will be realized, of course, that the concentration of the particular active compound utilized in admixture with the carrier vehicle will depend upon such factors as the type of equipment employed, method of application, area to be treated, types of pests to be controlled and degree of infestation. Therefore, in special cases it is possible to go above or below the aforementioned concentration ranges.

Granular preparations are produced for example, by taking up the active substance in a solvent and by using the resulting solution, as the case may be in the presence of a binder, to impregnate a granular carrier material, such as porous granules (for example, pumice and attaclay), or chopped tobacco stems or the like.

A granular preparation (frequently termed a "pellet") may alternatively be produced by compressing the active substance together with powdered minerals in the presence of lubricants and binders and by disintegrating and straining the composite to the desired grain size.

Dusts are obtainable by intimately mixing the active substance with an inert solid carrier material in a concentration of from about 1 to about 50% by weight. Examples of suitable solid carrier materials are talc, kaolin, pipe clay, diatomaceous earth, dolomite, gypsum, chalk, bentonite, attapulgite and colloidal $SiO_2$ or mixtures of these and similar substances. Alternatively organic carrier materials such as, for example, ground walnut shells may be used.

Wettable powders and flowables are produced by mixing from about 10 to about 99 parts by weight of a solid inert carrier such, for example, as the aforementioned carrier materials with from about 1 to about 80 parts by weight of the active substance, optionally dissolved in a volatile solvent such as acetone, from about 1 to about 5 parts by weight of a dispersing agent such, for example, as the lignosulfonates or alkylnaphthalene sulfonates known for this purpose and preferably also from about 0.5 to about 5 parts by weight of a wetting agent, such as fatty alcohol sulfates, or alkylarylsulfonates of fatty acid condensation products. In the case of flowables, a liquid inert carrier such as water is also included.

To produce emulsifiable concentrates the active compound is dissolved or finely divided in a suitable solvent which preferably is poorly miscible with water, an emulsifier being added to the resulting solution. Examples of suitable solvents are xylene, toluene, high-boiling aromatic petroleum distillates, for example solvent naphtha, distilled tar oil and mixtures of these liquids. Examples of suitable emulsifiers are alkylphenoxypolyglycol ethers, polyoxyethylene sorbitan esters of fatty acids or polyoxyethylene sorbitol esters of fatty acids. The concentration of the active compound in these emulsifiable concentrates is not restricted within narrow limits and may vary between about 2% and about 50% by weight depending upon toxicant solubility. A suitable liquid highly concentrated primary composition other than an emulsifiable concentrate is a solution of the active substance in a liquid which is readily miscible with water, for example, acetone, to which solution a dispersant and, as the case may be, a wetting agent are added. When such a primary composition is diluted with water shortly before or during the spraying operation an aqueous dispersion of the active substance is obtained.

An aerosol preparation according to the invention is obtained in the usual manner by incorporating the active substance or a solution thereof in a suitable solvent in a volatile liquid suitable for use as a propellant such, for example, as a mixture of chlorine and fluorine derivatives of methane and ethane.

Fumigating candles or fumigating powders, i.e., preparations which when burning are capable of emitting a pesticidal smoke, are obtained by taking up the active substance in a combustible mixture which may, for example, comprise a sugar or a wood, preferably in the ground form, as a fuel, a substance to sustain combustion such, for example, as ammonium nitrate or potassium chlorate, and furthermore a substance for retarding combustion, for example kaolin, bentonite and/or colloidal silicic acid.

A bait preparation comprises a food or other substance attractive to pests, a carrier, the toxicant and may optionally include other substances commonly used in preparations of this kind, such as, a preservative to inhibit bacterial and fungal growth, a waterproofing agent to prevent disintegration under wet conditions and dyes or colorants as described above.

In addition to the aforementioned ingredients, the preparations according to the invention may also contain other substances commonly used in preparations of this kind.

For example, a lubricant, such as calcium stearate or magnesium stearate, may be added to a wettable powder or to a mixture to be granulated. Furthermore, there may, for example, be added "adhesives" such as polyvinylalcohol cellulose derivatives or other colloidal materials, such as casein, to improve the adherence of this pesticide to the surface to be protected.

Representative preparation of compositions and formulations including the compounds of the present invention are set forth below as Examples A through I by way of illustration but not limitation.

Example A

Granular

| Ingredient | %/wt. |
|---|---|
| Toxicant and toxicant impurities | 0.25 |
| Triton X-305 (binder) (Octylphenyl-30-ethylene oxide ethanol) | 0.25 |
| Agsorb 24/48 (diluent) (Montmorillonite clay) | 99.50 |

Preparation: The toxicant and Triton X-305 are dissolved into methylene chloride and the mixture is added to the Agsorb with continuous mixing. The methylene chloride is then allowed to evaporate.

Example B

Dust

| Ingredient | %/wt. |
|---|---|
| Toxicant and toxicant impurities | 1.0 |
| Talc | 99.0 |

Preparation: Toxicant is dissolved in excess acetone and the mixture is impregnated onto the talc. The acetone is then permitted to evaporate.

Example C

Wettable Powder

| Ingredient | %/wt. |
|---|---|
| Toxicant and toxicant impurities | 31.3 |
| Duponal WA Dry (wetter) (Sodium lauryl sulfate) | 2.0 |
| Reax 45A (dispersant) (Sodium lignin sulfonate) | 5.0 |
| Barden clay (diluent) | 31.7 |
| HilSil 233 (diluent) (Sodium silica) | 30.0 |

Preparation: The toxicant optionally dissolved in a volatile solvent is absorbed onto the Barden clay and HiSil carriers. The Duponal and Reax are then added and the entire dry mixture blended until homogeneous. The composition is then micronized to a fine particle size.

Example D

Emulsifiable Concentrate

| Ingredient | %/wt. |
|---|---|
| Toxicant and toxicant impurities | 15.0 |
| Sponto 232T (emulsifier) (Anionic and nonionic blend of the following surfactants: calcium dodecyl benzene sulfonate; and ethoxylated alkylphenol) | 6.0 |
| Sponto 234T (emulsifier) (Anionic and nonionic blend of the following surfactants: calcium dodecyl benzene sulfonate; and ethoxylated alkylphenol) | 4.0 |
| Cyclohexanone (solvent) | 22.5 |
| Tenneco 500-100 (solvent) (Aromatic solvent mixture principally comprising xylene, cumene and ethyl benzene having a boiling point range of 290-345°F) | 52.5 |

Preparation: All ingredients are mixed together with continuous agitation until a homogeneous clear solution is obtained.

Example E

Aerosol

| Ingredient | %/wt. |
|---|---|
| Toxicant and toxicant impurities | 0.5 |
| Freon 12 | 99.5 |

Preparation: The components are mixed and packaged under pressure in a suitable container equipped with a release spray valve.

Example F

Fumigating Candle or Fumigating Powder

| Ingredient | %/wt. |
|---|---|
| Toxicant and toxicant impurities | 1.0 |
| Wood dust | 96.0 |
| Starch | 3.0 |

Preparation: Toxicant, wood dust, and starch are blended together and then molded into a candle using a small amount of water to activate the starch.

Example G

Bait

Method A

| Ingredient | %/wt. |
|---|---|
| Toxicant and toxicant impurities | 1.00 |
| Wheat Bran (carrier and attractant) | 89.95 |
| Corn Syrup (attractant) | 7.00 |
| Corn Oil (attractant) | 2.00 |
| Kathon 4200 (preservative) (3-isothiazolone) | 0.05 |

Preparation: The corn oil and corn syrup are added to the wheat bran with adequate mixing. The toxicant and Kathon are premixed with excess acetone and this solution is added to the wheat bran base with continued mixing. The acetone is then permitted to evaporate.

Method B

| Ingredient | %/wt. |
|---|---|
| Toxicant and toxicant impurities | 0.06 |
| Granulated Sugar (carrier and attractant) | 99.94 |

Example H

Pellet

Same as Example G, Method A, with this addition: the bait composition is formed into 1/4" diameter by 3/8" long pellets using a suitable die and press apparatus.

Example I

Flowable

| Ingredient | %/wt. |
|---|---|
| Toxicant and toxicant impurities | 31.3 |
| Duponal WA Dry (wetter) (Sodium lauryl sulfate) | 2.0 |
| Reax 45A (dispersant) (Sodium lignin sulfonate) | 5.0 |
| HiSil 233 (diluent) (Sodium silica) | 30.0 |
| Kelzan (thickener) (Xanthan gum) | 0.5 |
| Water | 31.2 |

Preparation: The toxicant is absorbed onto the HiSil carrier. The Duponal and Reax are then added and the entire dry mixture blended until homogeneous. The composition is then micronized to a fine particle size. The resulting powder is suspended in water and the Kelzan added.

Compositions and formulations according to the present invention may also include known pesticidal compounds. This expands the spectrum of activity of the preparations and may give rise to synergism.

The following known insecticidal, fungicidal and acaricidal compounds are suitable for use in such a combined preparation.

Insecticides such as:

Chlorinated hydrocarbons, for example, 2,2-bis(p-chlorophenyl)-1,1,1-trichloroethane and hexachloroepoxyoctahydrodimethanonaphthalene;

Carbamates, for example, N-methyl-1-naphthylcarbamate;

Dinitrophenols, for example, 2-methyl-4,6-dinitrophenol and 2-(2-butyl)-4,6-dinitrophenyl-3,3-dimethylacrylate;

Organic phosphorus compounds, such as dimethyl-2-methoxy-3-carbonyl-1-methylvinyl phosphate, O,O-diethyl-O-p-nitrophenylphosphorothioate; N-monomethylamide of O,O-dimethyldithiophosphorylacetic acid;

Diphenylsulfides, for example, p-chlorobenzyl or p-chlorophenyl sulfide and 2,4,4',5-tetrachlorodiphenylsulfide;

Diphenylsulfonates, for example, p-chlorophenylbenzenesulfonate;

Methylcarbinols, for example, 4,4-dichloro-1-trichloromethylbenzhydrol;

Quinoxaline compounds, such as methylquinoxaline dithiocarbonate;

Amidines such as N'-(4-chloro-2-methylphenyl) N,N-dimethylformamidine;

Pyrethroids such as Allethrin;

Biologicals such as Bacillus thuringiensis preparations;
Organic tin compounds such as tricyclohexyltin hydroxide;
Synergists such as piperonyl butoxide.
Fungicides such as:
Organic mercury compounds, for example, phenylmercuryacetate and methylmercurycyanoguanide;
Organic tin compounds, for example, triphenyltin hydroxide and triphenyltin acetate;
Alkylenebisdithiocarbamates, for example, zinc ethylenebisthiocarbamate and manganese ethylenebisdithiocarbamate; and
2,4-dinitro-6-(2-octyl-phenylcrotonate), 1-bis(dimethylamino)phosphoryl-3-phenyl-5-amino-1,2,4-triazole, 6-methylquinoxaline-2,3-dithiocarbonate, 1,4-dithioanthraquinone-2,3-dicarbonitrile, N-trichloromethylthiophthalimide, N-trichloromethylthiotetrahydrophthalimide, N-(1,1,2,2-tetrachloroethylthio)-tetrahydrophthalimide, N-dichlorofluoromethylthio-N-phenyl-N'-dimethylsulfonyldiamide and tetrachloroisophthalonitrile.

Biological Activity

It has been found by biological evaluation that compounds according to the present invention have pesticidal activity and are capable of controlling larvae and adult forms of pests, especially insects from the orders Lepidoptera and Coleoptera and most particularly insects from the order Lepidoptera. One skilled in the art will know how to determine the activity of a given compound against a given insect and the dosage required to obtain general or selective insecticidal effects.

As previously noted, the compounds of the present invention are particularly suitable for controlling plant-destructive insects in crops of cultivated plants, such as, but not limited to, cotton, vegetables, corn and other cereals and the like; forestry, such as, but not limited to, birch, spruce, pine, fir and the like; and ornamental plants, flowers and trees. Compounds of the present invention are also particularly suitable for controlling insects destructive to stored commodities such as seeds and the like; fruit crops, such as, but not limited to, fruit and/or citrus trees, raspberry bushes and the like; and turf, such as, but not limited to, lawns, sod and the like.

In evaluating the pesticidal activity of the compounds of this invention, the following test procedures were employed.

A test solution containing 600 parts per million (ppm) was made by dissolving the test compound in a solvent (acetone:methanol, 1:1), adding water to give an acetone:methanol:water system of 5:5:90 and then a surfactant. A 1:1 mixture of an alkylarylpolyetheralcohol (sold under the trademark Triton X-155) and a modified phthalic glycerol alkyl resin (sold under the trademark Triton B-1956) was utilized at the equivalent of 1 ounce per 100 gal. of test solution as a surfactant.

Initial evaluations were made on one or more of the following pests:

| Code Symbol | Common Name | Latin Name |
|---|---|---|
| SAW | Southern Armyworm | Spodoptera eridania |
| MBB | Mexican Bean Beetle | Epilachna varivestis |

For the foliar bean beetle and armyworm tests, individual bean (Phaseolus limensis var. Woods' Prolific) leaves are placed on moistened pieces of filter paper in Petri dishes. The leaves are then sprayed with the test solution using a rotating turntable and allowed to dry. The dishes are infested with 10 third instar larvae of Southern armyworm or Mexican bean beetle. The dishes are then covered.

The percent mortality for the bean beetle and armyworm evaluations are determined 96 hours after treatment. Evaluations are based on a scale of 0-100% in which 0 equals no activity and 100 equals total kill.

The rotating turntable consists of a fixed, continuously operating spray nozzle under which targets are rotated at a fixed speed and distance. When the target is a Petri dish (such as for the armyworm), the distance from the nozzle is 15 inches (38 cm). The nozzle is located 8 inches (20 cm) from the rotating shaft. The targets on individual platforms revolve around the shaft at 1 revolution per 20 seconds but only a brief portion of this time occurs in the spray path. Targets pass only once under the nozzle and then are removed to drying hoods.

The nozzle used is a 1/4 JCO Spraying Systems (Wheaton, Illinois) air atomizing nozzle equipped with a No. 2850 fluid cap and No. 70 air cap. At the 10 psig (0,7 $kg/cm^2$) air pressure used and with liquid siphon feed 0.5 GPH (gallons per hour) (2,27 litres/hr) are delivered in a round spray pattern with a 21° spray angle. Targets are misted with spray droplets to the point that the droplets coalesce to form a uniform thin

film insufficient to drown test organisms.

All treatments are maintained at 75-80°F (24°-26°C) under continuous fluorescent light in a well-ventilated room.

The results of the initial insecticidal evaluations are given in Table II.

Armyworm and bean beetle spray (foliar) results are 96 hours observation unless otherwise noted.

TABLE II

| Initial Biological Evaluations | | |
|---|---|---|
| Example No. | Foliar Application Test Species | |
| | SAW | MBB |
| 1 | 0 | 100 |
| 2 | 0 | 100 |
| 3 | 100 | 100 |
| 4 | 100 | 10 |
| 5 | 10 | 20 |
| 6 | 100 | 80 |
| 7 | 100 | 30 |
| 8 | 100 | 100 |
| 9 | 100 | 20 |
| 10 | 100 | 40 |
| 11 | 100 | 20 |

In its mechanical aspects therefore a process of the invention for improving the commercial value and/or profitability of vendible crops from plants whose growth is affected or likely to be affected by insects comprises (1) charging to a container, fumigation device or mechanical dissemination device an insecticidal composition of the invention as hereinbefore described (2) using the container, fumigator or mechanical dissemination device to apply the insecticidal composition, in the form of granules, dust, smoke, vapour or surfactant-containing liquid preparation to growing plants or to a growth medium where the plants are growing or are to be grown, or to the insects themselves, (3) controlling the dose of the active ingredient during this application step so that the rate of application of active insecticidal compound is sufficient to combat the insects but is insufficient to cause an unacceptably adverse effect on the crop plants growing or to be grown in the treated area.

The following words are trademarks which may be registered in some or all of the designated states: Triton, Agsorb, Duponal, Reax, Hisil, Sponto, Tenneco, Kathon and Kelzan.

## Claims

**1.** An insecticidally active compound which is a substituted hydrazine having the formula:

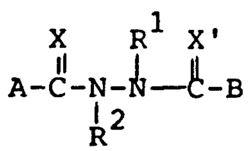

I

wherein

X and X' are the same or different O, S or NR;

$R^1$ is unsubstituted ($C_3$-$C_{10}$) branched alkyl or a ($C_1$-$C_4$) straight chain alkyl substituted with one or two of the same or different ($C_3$-$C_6$)cycloalkyl;

$R^2$ is ($C_1$-$C_6$)alkyl; alkoxyalkyl having, independently, 1 to 6 carbon atoms in each alkyl group; ($C_1$-$C_6$)alkylthioalkyl having, independently, 1 to 6 carbon atoms in each alkyl group; ($C_2$-$C_6$)alkenyl; ($C_2$-$C_6$)alkynyl; or phen-($C_1$-$C_4$)alkyl where the phenyl ring is unsubstituted or substituted with one to three of the same or different halo, cyano, nitro, hydroxy,

$(C_1-C_4)$alkyl, halo-$(C_1-C_4)$alkyl, $(C_1-C_4)$alkoxy, halo-$(C_1-C_4)$alkoxy, carboxy, $(C_1-C_4)$-alkoxy-carbonyl, $(C_1-C_4)$alkanoyloxy, $NH_2$, NHZ or NZZ';

A and B are the same or different

unsubstituted or substituted naphthyl where the substituents can be from one to five of the same or different halo; cyano; nitro; hydroxy; $(C_1-C_4)$alkoxy; $(C_1-C_4)$alkyl; carboxy; $(C_1-C_4)$alkoxy-carbonyl; $(C_1-C_4)$alkanoyloxy; $NH_2$; NHZ or NZZ';

unsubstituted or substituted phenyl where the substituents can be from one to five of the same or different halo; nitroso; nitro; cyano; hydroxy; $(C_1-C_6)$alkyl; halo-$(C_1-C_6)$alkyl; cyano-$(C_1-C_6)$alkyl; $(C_1-C_6)$alkoxy; halo-$(C_1-C_6)$alkoxy; alkoxyalkyl having independently 1 to 6 carbon atoms in each alkyl group; alkoxyalkoxy having independently 1 to 6 carbon atoms in each alkyl group; $-OCO_2R$ group; $(C_2-C_6)$-alkenyl optionally substituted with halo, cyano, $(C_1-C_4)$alkyl, $(C_1-C_4)$alkoxy, halo-$(C_1-C_4)$alkoxy or $(C_1-C_4)$alkylthio; $(C_2-C_6)$alkenyl-carbonyl; $(C_2-C_6)$alkadienyl; $(C_2-C_6)$alkynyl optionally substituted with halo, cyano, nitro, hydroxy, $(C_1-C_4)$alkyl, $(C_1-C_4)$alkoxy, halo-$(C_1-C_4)$alkoxy or $(C_1-C_4)$alkylthio; $(C_2-C_6)$alkynyl-carbonyl; carboxy; $-ZCO_2R'$ group; -COR group; halo-$(C_1-C_6)$alkyl-carbonyl; cyano-$(C_1-C_6)$alkyl-carbonyl; nitro-$(C_1-C_6)$alkyl-carbonyl; $(C_1-C_6)$alkoxy-carbonyl; halo-$(C_1-C_6)$alkoxy-carbonyl; -OCOR group; -NRR' group; amino substituted with hydroxy, $(C_1-C_4)$alkoxy or $(C_1-C_4)$alkylthio groups; phenylamino; diphenylamino; -CONRR' group; -OCONRR' group; -C(NR)NR'R'' group; -N=NR group; phenylazo; -NRCOR' group; $-NRCO_2R'$ group; -N(COR)COR' group; -OCONRCOR' group; sulfhydryl; halothio; thiocyanato; $(C_1-C_6)$alkylthio; halo-$(C_1-C_6)$alkylthio; -SOR group; $-SO_2R$ group; phenylsulfonyl; $-OSO_2R$ group; $(C_1-C_6)$haloalkylsulfonyloxy; $-SO_2NRR'$ group; -NRSOR' group; $-NRSO_2R'$ group; -CSR group; $-CS_2R$ group; -NRCSR' group; -SCOR group; tri-$(C_1-C_6)$alkylsilyl having independently the stated number of carbon atoms in each alkyl group; unsubstituted or substituted phenyl having one to three of the same or different halo, cyano, nitro, hydroxy $(C_1-C_4)$alkyl, $(C_1-C_4)$-alkoxy, carboxy, $(C_1-C_4)$alkoxy-carbonyl, $(C_1-C_4)$alkanoyloxy, $NH_2$, NHZ or NZZ'; phenoxy where the phenyl ring is unsubstituted or substituted with one to three of the same or different halo, cyano, nitro, hydroxy, $(C_1-C_4)$alkyl, $(C_1-C_4)$alkoxy, carboxy, $(C_1-C_4)$alkoxy-carbonyl, $(C_1-C_4)$alkanoyloxy, $NH_2$, NHZ or NZZ'; benzoyl where the phenyl ring is unsubstituted or substituted with one to three of the same or different halo, cyano, nitro, hydroxy, $(C_1-C_4)$alkyl, $(C_1-C_4)$alkoxy, carboxy, $(C_1-C_4)$alkoxy-carbonyl, $(C_1-C_4)$alkanoyloxy, $NH_2$, NHZ or NZZ'; phenoxycarbonyl where the phenyl ring is unsubstituted or substituted with one to three of the same or different halo, cyano, nitro, hydroxy, $(C_1-C_4)$alkyl, $(C_1-C_4)$alkoxy, carboxy, $(C_1-C_4)$alkoxy-carbonyl, $(C_1-C_4)$alkanoyloxy, $NH_2$, NHZ or NZZ'; phenylthio where the phenyl ring is unsubstituted or substituted with one to three of the same or different halo, cyano, nitro, hydroxy, $(C_1-C_4)$alkyl, $(C_1-C_4)$alkoxy, carboxy, $(C_1-C_4)$alkoxy-carbonyl, $(C_1-C_4)$alkanoyloxy, $NH_2$, NHZ or NZZ'; $-CR=N-R^2$ group where $R^2$ is hydroxy, $(C_1-C_4)$alkyl, $(C_1-C_4)$alkoxy, -NRR', phenylamino, -COR, carboxy, $(C_1-C_4)$-alkoxy-carbonyl, $(C_1-C_4)$alkanoyloxy, benzoyl, phenoxycarbonyl or -CONRR'; or when two adjacent positions on the phenyl ring are substituted with alkoxy groups, these groups may be joined to form, together with the carbon atoms to which they are attached, a 5 or 6 membered dioxolano or dioxano heterocyclic ring;

where R, R' and R'' are hydrogen or $(C_1-C_6)$alkyl; Z and Z' are $(C_1-C_6)$alkyl; plus N-trimethylsiloxymethyl-N'-2,2 dimethylpropyl-N-(4-methoxy-3-chlorobenzoyl)-N'-benzoylhydrazine and N-phenylthiomethyl-t-butyl-N-(4-toluoyl)-N'-(3,4-dichlorobenzoyl) hydrazine, but excluding

$N(CHMe_2)COPh$
|
$N(CHMe_2)COPh$

and

$N(CH_2CHMe_2)COPh$
|
$N(CH_2CHMe_2)COPh$.

or an agronomically acceptable salt thereof.

**2.** A compound according to claim 1 wherein

X and X' are O or S;

$R^1$ is unsubstituted ($C_3$-$C_8$) branched alkyl;

$R^2$ is ($C_1$-$C_4$)alkyl; ($C_1$-$C_4$)alkoxy-($C_1$-$C_4$)alkyl having independently the stated number of carbon atoms in each alkyl group; methylthiomethyl; ($C_2$-$C_5$)alkenyl; ($C_2$-$C_5$)alkynyl; or benzyl where the phenyl ring is unsubstituted or substituted with halo, nitro, ($C_1$-$C_4$)alkyl or ($C_1$-$C_4$)alkoxy; and

A and B are the same or different unsubstituted naphthyl; or unsubstituted or substituted phenyl where the substituents can be from one to three of the same or different halo; nitro; cyano; ($C_1$-$C_4$)alkyl; halo-($C_1$-$C_4$)alkyl; cyano-($C_1$-$C_4$)alkyl; ($C_1$-$C_4$)alkoxy, halo-($C_1$-$C_4$)alkoxy; -COD group; carboxy; ($C_1$-$C_4$)alkoxy-carbonyl; ($C_1$-$C_4$)alkanoyloxy; ($C_2$-$C_6$)-alkenyl; ($C_2$-$C_6$)alkadienyl; ($C_2$-$C_6$)alkynyl; -NDD' group; thiocyanato; ($C_1$-$C_4$)-alkylthio; -CSD group; -$CS_2$D group; -SCOD group; tri-($C_1$-$C_4$)alkylsilyl having independently the stated number of carbon atoms in each alkyl group; unsubstituted or substituted phenyl having one to two of the same or different halo, nitro, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ'; phenoxy where the phenyl ring is unsubstituted or substituted with one or two of the same or different halo, nitro, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ'; or when two adjacent positions on the phenyl ring are substituted with alkoxy groups, these groups may be joined to form a 5- or 6-membered dioxolano or dioxano heterocyclic ring;

where D and D' are hydrogen or ($C_1$-$C_4$)alkyl; and Z and Z' are ($C_1$-$C_4$)alkyl.

**3.** A compound according to claim 2 wherein

$R^1$ is ($C_4$-$C_7$) branched alkyl;

$R^2$ is methyl; ethyl; ($C_1$-$C_2$)alkoxyalkyl having independently the stated number of carbon atoms in each alkyl group; ($C_2$-$C_5$)alkenyl; ($C_2$-$C_5$)alkynyl; or benzyl where the phenyl ring is unsubstituted or substituted with halo; and

A and B are the same or different unsubstituted naphthyl;

unsubstituted or substituted phenyl having one to three of the same of different halo; nitro; cyano; ($C_1$-$C_4$)alkyl; halo-($C_1$-$C_4$)alkyl; cyano-($C_1$-$C_4$)alkyl; ($C_1$-$C_4$)alkoxy; ($C_1$-$C_4$)alkoxy-($C_1$-$C_4$)alkyl having independently the stated number of carbon atoms in each alkyl group; -COD; ($C_1$-$C_4$)alkoxy-carbonyl; ($C_1$-$C_4$)alkanoyloxy; thiocyanato; unsubstituted or substituted phenyl having one or two of the same or different halo, nitro, ($C_1$-$C_4$)alkyl, ($C_1$-$C_4$)alkoxy, carboxy, ($C_1$-$C_4$)alkoxy-carbonyl, ($C_1$-$C_4$)-alkanoyloxy, $NH_2$, NHZ or NZZ'; or phenoxy where the phenyl ring is unsubstituted or substituted with one or two of the same or different halo, nitro, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ'; where D is hydrogen or ($C_1$-$C_4$)alkyl; and Z and Z' are ($C_1$-$C_4$)alkyl.

**4.** A compound according to claim 3 wherein

X and X' are O;

$R^1$ is t-butyl; neopentyl (2,2-dimethylpropyl) or 1,2,2-trimethylpropyl;

$R^2$ is methyl; methoxymethyl; ($C_2$-$C_4$)alkenyl; ($C_2$-$C_5$)alkynyl; benzyl or 4-halobenzyl; and

A and B are the same or different phenyl or substituted phenyl where the substituents can be

from one to three of the same or different halo, nitro, ($C_1$-$C_4$)alkyl, ($C_1$-$C_4$)alkoxy, or halo-($C_1$-$C_4$)alkyl.

**5.** A compound according to claim 4 wherein
$R^1$ is t-butyl;
$R^2$ is ($C_2$-$C_5$)alkynyl; and
A and B are unsubstituted or substituted phenyl where the substituents can be one or two of the same or different chloro, fluoro, bromo, iodo, methyl, ethyl, methoxy or trifluoromethyl.

**6.** A compound according to claim 5 wherein
X and X' are O;
$R^1$ is t-butyl; and
$R^2$ is 2-propynyl; and (a)
A is 4-methylphenyl and
B is 3-methylphenyl; or (b)
A is phenyl and
B is phenyl,
2,4-dichlorophenyl or 3,4-dichlorophenyl.

**7.** An insecticidal composition comprising agronomically acceptable diluent or carrier and an insecticidal compound according to any one of the preceding claims.

**8.** A composition according to claim 7 containing from 0.001 to 90%, preferably 0.01 to 75%, by weight of the insecticidal compound.

**9.** A composition according to claim 7 or 8 but including,

$N(CHMe_2)COPh$ and $N(CH_2CHMe_2)COPh$
$N(CHMe_2)COPh$ $\quad$ $N(CH_2CHMe_2)COPh$

(a) additionally containing an emulsifying agent, said composition being in the form of an emulsifiable concentrate; (b) additionally containing a dispersing agent, said composition being in the form of a wettable powder; (c) containing a liquid agronomically acceptable carrier and a dispering agent, said composition being in the form of a flowable; (d) in the form of a dust or granules or (e) additionally containing an attractant agent, said composition being in the form of a bait.

**10.** A method of controlling insects which comprises contacting said insects with an insecticidally effective amount of insecticidally active compound according to any of claims 1 to 6, but including

$N(CHMe_2)COPh$ and $N(CH_2CHMe_2)COPh$
$N(CHMe_2)COPh$ $\quad$ $N(CH_2CHMe_2)COPh$.

optionally in a composition according to claim 7, 8 or 9.

**11.** A method according to claim 10 wherein the insecticidally active compound is applied to growing plants or an area wherein plants are to be grown at a rate from 10 grams to about 10 kilograms per hectare of said active compound, preferably 100 grams to 5 kilograms per hectare.

**12.** A method according to claim 10 or 11 for controlling insects from the order Lepidoptera or Coleoptera.

**Claims for the following Contracting State : AT**

**1.** An insecticidal composition comprising (a) an insecticidally active compound which is a substituted

hydrazine having the formula:

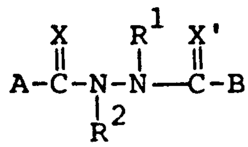

I

wherein

X and X' are the same or different O, S or NR;

$R^1$ is unsubstituted ($C_3$-$C_{10}$) branched alkyl or a ($C_1$-$C_4$) straight chain alkyl substituted with one or two of the same or different ($C_3$-$C_6$)cycloalkyl;

$R^2$ is ($C_1$-$C_6$)alkyl; alkoxyalkyl having, independently, 1 to 6 carbon atoms in each alkyl group; ($C_1$-$C_6$)alkylthioalkyl having, independently, 1 to 6 carbon atoms in each alkyl group; ($C_2$-$C_6$)alkenyl; ($C_2$-$C_6$)alkynyl; or phen-($C_1$-$C_4$)alkyl where the phenyl ring is unsubstituted or substituted with one to three of the same or different halo, cyano, nitro, hydroxy, ($C_1$-$C_4$)alkyl, halo-($C_1$-$C_4$)alkyl, ($C_1$-$C_4$)alkoxy, halo-($C_1$-$C_4$)alkoxy, carboxy, ($C_1$-$C_4$)-alkoxy-carbonyl, ($C_1$-$C_4$)alkanoyloxy, $NH_2$, NHZ or NZZ';

A and B are the same or different

unsubstituted or substituted naphthyl where the substituents can be from one to five of the same or different halo; cyano; nitro; hydroxy; ($C_1$-$C_4$)alkoxy; ($C_1$-$C_4$)alkyl; carboxy; ($C_1$-$C_4$)alkoxy-carbonyl; ($C_1$-$C_4$)alkanoyloxy; $NH_2$; NHZ or NZZ';

unsubstituted or substituted phenyl where the substituents can be from one to five of the same or different halo; nitroso; nitro; cyano; hydroxy; ($C_1$-$C_6$)alkyl; halo-($C_1$-$C_6$)alkyl; cyano-($C_1$-$C_6$)alkyl; ($C_1$-$C_6$)alkoxy; halo-($C_1$-$C_6$)alkoxy; alkoxyalkyl having independently 1 to 6 carbon atoms in each alkyl group; alkoxyalkoxy having independently 1 to 6 carbon atoms in each alkyl group; $-OCO_2R$ group; ($C_2$-$C_6$)-alkenyl optionally substituted with halo, cyano, ($C_1$-$C_4$)alkyl, ($C_1$-$C_4$)alkoxy, halo-($C_1$-$C_4$)alkoxy or ($C_1$-$C_4$)alkylthio; ($C_2$-$C_6$)alkenyl-carbonyl; ($C_2$-$C_6$)alkadienyl; ($C_2$-$C_6$)alkynyl optionally substituted with halo, cyano, nitro, hydroxy, ($C_1$-$C_4$)alkyl, ($C_1$-$C_4$)alkoxy, halo-($C_1$-$C_4$)alkoxy or ($C_1$-$C_4$)alkylthio; ($C_2$-$C_6$)alkynyl-carbonyl; carboxy; $-ZCO_2R'$ group; -COR group; halo-($C_1$-$C_6$)alkyl-carbonyl; cyano-($C_1$-$C_6$)alkyl-carbonyl; nitro-($C_1$-$C_6$)alkyl-carbonyl; ($C_1$-$C_6$)alkoxy-carbonyl; halo-($C_1$-$C_6$)alkoxy-carbonyl; -OCOR group; -NRR' group; amino substituted with hydroxy, ($C_1$-$C_4$)alkoxy or ($C_1$-$C_4$)alkylthio groups; phenylamino; diphenylamino; -CONRR' group; -OCONRR' group; -C(NR)NR'R'' group; -N=NR group; phenylazo; -NRCOR' group; $-NRCO_2R'$ group; -N(COR)COR' group; -OCONRCOR' group; sulfhydryl; halothio; thiocyanato; ($C_1$-$C_6$)alkylthio; halo-($C_1$-$C_6$)alkylthio; -SOR group; $-SO_2R$ group; phenylsulfonyl; $-OSO_2R$ group; ($C_1$-$C_6$)haloalkylsulfonyloxy; $-SO_2NRR'$ group; -NRSOR' group; $-NRSO_2R'$ group; -CSR group; $-CS_2R$ group; -NRCSR' group; -SCOR group; tri-($C_1$-$C_6$)alkylsilyl having independently the stated number of carbon atoms in each alkyl group; unsubstituted or substituted phenyl having one to three of the same or different halo, cyano, nitro, hydroxy ($C_1$-$C_4$)alkyl, ($C_1$-$C_4$)-alkoxy, carboxy, ($C_1$-$C_4$)alkoxy-carbonyl, ($C_1$-$C_4$)alkanoyloxy, $NH_2$, NHZ or NZZ'; phenoxy where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ'; benzoyl where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ'; phenoxycarbonyl where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ'; phenylthio where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-

carbonyl, ($C_1$-$C_4$)alkanoyloxy, $NH_2$, NHZ or NZZ'; -CR=N-$R^2$ group where $R^2$ is hydroxy, ($C_1$-$C_4$)alkyl, ($C_1$-$C_4$)alkoxy, -NRR', phenylamino, -COR, carboxy, ($C_1$-$C_4$)-alkoxy-carbonyl, ($C_1$-$C_4$)alkanoyloxy, benzoyl, phenoxycarbonyl or -CONRR'; or when two adjacent positions on the phenyl ring are substituted with alkoxy groups, these groups may be joined to form, together with the carbon atoms to which they are attached, a 5 or 6 membered dioxolano or dioxano heterocyclic ring;

where R, R' and R'' are hydrogen or ($C_1$-$C_6$)alkyl; Z and Z' are ($C_1$-$C_6$)alkyl; plus N-trimethylsiloxymethyl-N'-2,2 dimethylpropyl-N-(4-methoxy-3-chlorobenzoyl)-N'-benzoylhydrazine and N-phenylthiomethyl-t-butyl-N-(4-toluoyl)-N'-(3,4-dichlorobenzoyl) hydrazine, but excluding

$N(CHMe_2)COPh$
|
$N(CHMe_2)COPh$

and

$N(CH_2CHMe_2)COPh$
|
$N(CH_2CHMe_2)COPh$.

or an agronomically acceptable salt thereof, and (b) an agronomically acceptable diluent or carrier.

**2.** A composition according to claim 1 wherein

X and X' are O or S;

$R^1$ is unsubstituted ($C_3$-$C_8$) branched alkyl;

$R^2$ is ($C_1$-$C_4$)alkyl; ($C_1$-$C_4$)alkoxy-($C_1$-$C_4$)alkyl having independently the stated number of carbon atoms in each alkyl group; methylthiomethyl; ($C_2$-$C_5$)alkenyl; ($C_2$-$C_5$)alkynyl; or benzyl where the phenyl ring is unsubstituted or substituted with halo, nitro, ($C_1$-$C_4$)alkyl or ($C_1$-$C_4$)alkoxy; and

A and B are the same or different unsubstituted naphthyl; or

unsubstituted or substituted phenyl where the substituents can be from one to three of the same or different halo; nitro; cyano; ($C_1$-$C_4$)alkyl; halo-($C_1$-$C_4$)alkyl; cyano-($C_1$-$C_4$)alkyl; ($C_1$-$C_4$)alkoxy, halo-($C_1$-$C_4$)alkoxy; -COD group; carboxy; ($C_1$-$C_4$)-alkoxy-carbonyl; ($C_1$-$C_4$)alkanoyloxy; ($C_2$-$C_6$)-alkenyl; ($C_2$-$C_6$)alkadienyl; ($C_2$-$C_6$)-alkynyl; -NDD' group; thiocyanato; ($C_1$-$C_4$)alkylthio; -CSD group; -$CS_2$D group; -SCOD group; tri-($C_1$-$C_4$)alkylsilyl having independently the stated number of carbon atoms in each alkyl group; unsubstituted or substituted phenyl having one to two of the same or different halo, nitro, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ'; phenoxy where the phenyl ring is unsubstituted or substituted with one or two of the same or different halo, nitro, ($C_1$-$C_4$)alkyl, ($C_1$-$C_4$)alkoxy, carboxy, ($C_1$-$C_4$)alkoxy-carbonyl, ($C_1$-$C_4$)-alkanoyloxy, $NH_2$, NHZ or NZZ'; or when two adjacent positions on the phenyl ring are substituted with alkoxy groups, these groups may be joined to form a 5- or 6-membered dioxolano or dioxano heterocyclic ring;

where D and D' are hydrogen or ($C_1$-$C_4$)alkyl; and Z and Z' are ($C_1$-$C_4$)alkyl.

**3.** A composition according to claim 2 wherein

$R^1$ is ($C_4$-$C_7$) branched alkyl;

$R^2$ is methyl; ethyl; ($C_1$-$C_2$)alkoxyalkyl having independently the stated number of carbon atoms in each alkyl group; ($C_2$-$C_5$)alkenyl; ($C_2$-$C_5$)alkynyl; or benzyl where the phenyl ring is unsubstituted or substituted with halo; and

A and B are the same or different unsubstituted naphthyl;

unsubstituted or substituted phenyl having one to three of the same of different halo; nitro; cyano; ($C_1$-$C_4$)alkyl; halo-($C_1$-$C_4$)alkyl; cyano-($C_1$-$C_4$)alkyl; ($C_1$-$C_4$)alkoxy; ($C_1$-$C_4$)alkoxy-($C_1$-$C_4$)alkyl having independently the stated number of carbon atoms in

each alkyl group; -COD; $(C_1-C_4)$alkoxy-carbonyl; $(C_1-C_4)$alkanoyloxy; thiocyanato; unsubstituted or substituted phenyl having one or two of the same or different halo, nitro, $(C_1-C_4)$alkyl, $(C_1-C_4)$alkoxy, carboxy, $(C_1-C_4)$alkoxy-carbonyl, $(C_1-C_4)$-alkanoyloxy, $NH_2$, NHZ or NZZ'; or phenoxy where the phenyl ring is unsubstituted or substituted with one or two of the same or different halo, nitro, $(C_1-C_4)$alkyl, $(C_1-C_4)$alkoxy, carboxy, $(C_1-C_4)$alkoxy-carbonyl, $(C_1-C_4)$alkanoyloxy, $NH_2$, NHZ or NZZ'; where D is hydrogen or $(C_1-C_4)$alkyl; and Z and Z' are $(C_1-C_4)$alkyl.

**4.** A composition according to claim 3 wherein

| | |
|---|---|
| X and X' are | O; |
| $R^1$ is | t-butyl; neopentyl (2,2-dimethylpropyl) or 1,2,2-trimethylpropyl; |
| $R^2$ is | methyl; methoxymethyl; $(C_2-C_4)$alkenyl; $(C_2-C_5)$alkynyl; benzyl or 4-halobenzyl; and |
| A and B | are the same or different phenyl or substituted phenyl where the substituents can be from one to three of the same or different halo, nitro, $(C_1-C_4)$alkyl, $(C_1-C_4)$-alkoxy, or halo-$(C_1-C_4)$alkyl. |

**5.** A composition according to claim 4 wherein

| | |
|---|---|
| $R^1$ is | t-butyl; |
| $R^2$ is | $(C_2-C_5)$alkynyl; and |
| A and B are | unsubstituted or substituted phenyl where the substituents can be one or two of the same or different chloro, fluoro, bromo, iodo, methyl, ethyl, methoxy or trifluoromethyl. |

**6.** A composition according to claim 5 wherein

| | |
|---|---|
| X and X' are | O; |
| $R^1$ is | t-butyl; and |
| $R^2$ is | 2-propynyl; and (a) |
| A is | 4-methylphenyl and |
| B is | 3-methylphenyl; or (b) |
| A is | phenyl and |
| B is | phenyl, |
| | 2,4-dichlorophenyl or 3,4-dichlorophenyl. |

**7.** A composition according to any preceding claim containing from 0.001 to 90%, preferably 0.01 to 75%, by weight of the insecticidal compound.

**8.** A composition according to any preceding claim also including,

N$(CHMe_2)$COPh and N$(CH_2CHMe_2)$COPh
N$(CHMe_2)$COPh      N$(CH_2CHMe_2)$COPh

(a) additionally containing an emulsifying agent, said composition being in the form of an emulsifiable concentrate; (b) additionally containing a dispersing agent, said composition being in the form of a wettable powder; (c) containing a liquid agronomically acceptable carrier and a dispering agent, said composition being in the form of a flowable; (d) in the form of a dust or granules or (e) additionally containing an attractant agent, said composition being in the form of a bait.

**9.** A method of controlling insects which comprises contacting said insects with an insecticidally effective amount of insecticidally active composition as defined in any of claims 1 to 8.

**10.** A method according to claim 9 wherein the insecticidally active composition is applied to growing plants or an area wherein plants are to be grown at a rate from 10 grams to about 10 kilograms per hectare of said active compound, preferably 100 grams to 5 kilograms per hectare.

**11.** A method according to claim 9 or 10 for controlling insects from the order Lepidoptera or Coleoptera.

**Claims for the following Contracting State : ES**

**1.** The use of an insecticidally active compound which is a substituted hydrazine having the formula:

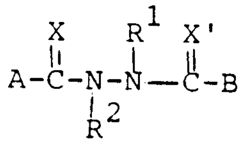

I

wherein

X and X' are the same or different O, S or NR;

$R^1$ is unsubstituted ($C_3$-$C_{10}$) branched alkyl or a ($C_1$-$C_4$) straight chain alkyl substituted with one or two of the same or different ($C_3$-$C_6$)cycloalkyl;

$R^2$ is ($C_1$-$C_6$)alkyl; alkoxyalkyl having, independently, 1 to 6 carbon atoms in each alkyl group; ($C_1$-$C_6$)alkylthioalkyl having, independently, 1 to 6 carbon atoms in each alkyl group; ($C_2$-$C_6$)alkenyl; ($C_2$-$C_6$)alkynyl; or phen-($C_1$-$C_4$)alkyl where the phenyl ring is unsubstituted or substituted with one to three of the same or different halo, cyano, nitro, hydroxy, ($C_1$-$C_4$)alkyl, halo-($C_1$-$C_4$)alkyl, ($C_1$-$C_4$)alkoxy, halo-($C_1$-$C_4$)alkoxy, carboxy, ($C_1$-$C_4$)-alkoxy-carbonyl, ($C_1$-$C_4$)alkanoyloxy, $NH_2$, NHZ or NZZ';

A and B are the same or different

unsubstituted or substituted naphthyl where the substituents can be from one to five of the same or different halo; cyano; nitro; hydroxy; ($C_1$-$C_4$)alkoxy; ($C_1$-$C_4$)alkyl; carboxy; ($C_1$-$C_4$)alkoxy-carbonyl; ($C_1$-$C_4$)alkanoyloxy; $NH_2$; NHZ or NZZ';

unsubstituted or substituted phenyl where the substituents can be from one to five of the same or different halo; nitroso; nitro; cyano; hydroxy; ($C_1$-$C_6$)alkyl; halo-($C_1$-$C_6$)alkyl; cyano-($C_1$-$C_6$)alkyl; ($C_1$-$C_6$)alkoxy; halo-($C_1$-$C_6$)alkoxy; alkoxyalkyl having independently 1 to 6 carbon atoms in each alkyl group; alkoxyalkoxy having independently 1 to 6 carbon atoms in each alkyl group; -$OCO_2R$ group; ($C_2$-$C_6$)-alkenyl optionally substituted with halo, cyano, ($C_1$-$C_4$)alkyl, ($C_1$-$C_4$)alkoxy, halo-($C_1$-$C_4$)alkoxy or ($C_1$-$C_4$)alkylthio; ($C_2$-$C_6$)alkenyl-carbonyl; ($C_2$-$C_6$)alkadienyl; ($C_2$-$C_6$)alkynyl optionally substituted with halo, cyano, nitro, hydroxy, ($C_1$-$C_4$)alkyl, ($C_1$-$C_4$)alkoxy, halo-($C_1$-$C_4$)alkoxy or ($C_1$-$C_4$)alkylthio; ($C_2$-$C_6$)alkynyl-carbonyl; carboxy; -$ZCO_2R'$ group; -COR group; halo-($C_1$-$C_6$)alkyl-carbonyl; cyano-($C_1$-$C_6$)alkyl-carbonyl; nitro-($C_1$-$C_6$)alkyl-carbonyl; ($C_1$-$C_6$)alkoxy-carbonyl; halo-($C_1$-$C_6$)alkoxy-carbonyl; -OCOR group; -NRR' group; amino substituted with hydroxy, ($C_1$-$C_4$)alkoxy or ($C_1$-$C_4$)alkylthio groups; phenylamino; diphenylamino; -CONRR' group; -OCONRR' group; -C(NR)NR'R'' group; -N=NR group; phenylazo; -NRCOR' group; -$NRCO_2R'$ group; -N(COR)COR' group; -OCONRCOR' group; sulfhydryl; halothio; thiocyanato; ($C_1$-$C_6$)alkylthio; halo-($C_1$-$C_6$)alkylthio; -SOR group; -$SO_2R$ group; phenylsulfonyl; $OSO_2R$ group; ($C_1$-$C_6$)haloalkylsulfonyloxy; -$SO_2NRR'$ group; -NRSOR' group; $NRSO_2R'$ group; -CSR group; -$CS_2R$ group; -NRCSR' group; -SCOR group; tri-($C_1$-$C_6$)alkylsilyl having independently the stated number of carbon atoms in each alkyl group; unsubstituted or substituted phenyl having one to three of the same or different halo, cyano, nitro, hydroxy ($C_1$-$C_4$)alkyl, ($C_1$-$C_4$)-alkoxy, carboxy, ($C_1$-$C_4$)alkoxy-carbonyl, ($C_1$-$C_4$)alkanoyloxy, $NH_2$, NHZ or NZZ'; phenoxy where the phenyl ring is unsubstituted or substituted with one to three of the same or different halo, cyano, nitro, hydroxy, ($C_1$-$C_4$)alkyl, ($C_1$-$C_4$)- alkoxy, carboxy, ($C_1$-$C_4$)alkoxy-carbonyl, ($C_1$-$C_4$)alkanoyloxy, $NH_2$, NHZ or NZZ'; benzoyl where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ'; phenoxycarbonyl where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)- alkoxy-carbonyl, ($C_1$-$C_4$)alkanoyloxy, $NH_2$, NHZ or NZZ'; phenylthio where the

phenyl ring is unsubstituted or substituted with one to three of the same or different halo, cyano, nitro, hydroxy, $(C_1-C_4)$alkyl, $(C_1-C_4)$alkoxy, carboxy, $(C_1-C_4)$alkoxy-carbonyl, $(C_1-C_4)$alkanoyloxy, $NH_2$, NHZ or NZZ'; -CR=N-$R^2$ group where $R^2$ is hydroxy, $(C_1-C_4)$alkyl, $(C_1-C_4)$alkoxy, -NRR', phenylamino, -COR, carboxy, $(C_1-C_4)$-alkoxy-carbonyl, $(C_1-C_4)$alkanoyloxy, benzoyl, phenoxycarbonyl or -CONRR'; or when two adjacent positions on the phenyl ring are substituted with alkoxy groups, these groups may be joined to form, together with the carbon atoms to which they are attached, a 5 or 6 membered dioxolano or dioxano heterocyclic ring;

where R, R' and R'' are hydrogen or $(C_1-C_6)$alkyl; Z and Z' are $(C_1-C_6)$alkyl;
plus N-trimethylsiloxymethyl-N'-2,2-dimethylpropyl-N-(4-methoxy-3-chlorobenzoyl)-N'-benzoylhydrazine
and N-phenylthiomethyl-t-butyl-N-(4-toluoyl)-N'-(3,4-dichlorobenzoyl)hydrazine;

or an agronomically acceptable salt thereof; together with agronomically acceptable diluent or carrier in making an insecticidal composition.

**2.** The use according to claim 1 wherein

X and X' are O or S;
$R^1$ is unsubstituted $(C_3-C_8)$ branched alkyl;
$R^2$ is $(C_1-C_4)$alkyl; $(C_1-C_4)$alkoxy-$(C_1-C_4)$alkyl having independently the stated number of carbon atoms in each alkyl group; methylthiomethyl; $(C_2-C_5)$alkenyl; $(C_2-C_5)$alkynyl; or benzyl where the phenyl ring is unsubstituted or substituted with halo, nitro, $(C_1-C_4)$alkyl or $(C_1-C_4)$alkoxy; and
A and B are the same or different
unsubstituted naphthyl; or

unsubstituted or substituted phenyl where the substituents can be from one to three of the same or different halo; nitro; cyano; $(C_1-C_4)$alkyl; halo-$(C_1-C_4)$alkyl; cyano-$(C_1-C_4)$alkyl; $(C_1-C_4)$alkoxy, halo-$(C_1-C_4)$alkoxy; -COD group; carboxy; $(C_1-C_4)$-alkoxy-carbonyl; $(C_1-C_4)$alkanoyloxy; $(C_2-C_6)$-alkenyl; $(C_2-C_6)$alkadienyl; $(C_2-C_6)$-alkynyl; -NDD' group; thiocyanato; $(C_1-C_4)$alkylthio; -CSD group; -$CS_2$D group; -SCOD group; tri-$(C_1-C_4)$alkylsilyl having independently the stated number of carbon atoms in each alkyl group; unsubstituted or substituted phenyl having one to two of the same or different halo, nitro, $(C_1-C_4)$alkyl, $(C_1-C_4)$alkoxy, carboxy, $(C_1-C_4)$alkoxy-carbonyl, $(C_1-C_4)$alkanoyloxy, $NH_2$, NHZ or NZZ'; phenoxy where the phenyl ring is unsubstituted or substituted with one or two of the same or different halo, nitro, $(C_1-C_4)$alkyl, $(C_1-C_4)$alkoxy, carboxy, $(C_1-C_4)$alkoxy-carbonyl, $(C_1-C_4)$-alkanoyloxy, $NH_2$, NHZ or NZZ'; or when two adjacent positions on the phenyl ring are substituted with alkoxy groups, these groups may be joined to form a 5- or 6-membered dioxolano or dioxano heterocyclic ring;

where D and D' are hydrogen or $(C_1-C_4)$alkyl; and Z and Z' are $(C_1-C_4)$alkyl.

**3.** The use according to claim 2 wherein

$R^1$ is $(C_4-C_7)$ branched alkyl;
$R^2$ is methyl; ethyl; $(C_1-C_2)$alkoxyalkyl having independently the stated number of carbon atoms in each alkyl group; $(C_2-C_5)$alkenyl; $(C_2-C_5)$alkynyl; or benzyl where the phenyl ring is unsubstituted or substituted with halo; and
A and B are the same or different unsubstituted naphthyl;

unsubstituted or substituted phenyl having one to three of the same of different halo; nitro; cyano; $(C_1-C_4)$alkyl; halo-$(C_1-C_4)$alkyl; cyano-$(C_1-C_4)$alkyl; $(C_1-C_4)$alkoxy; $(C_1-C_4)$alkoxy-$(C_1-C_4)$alkyl having independently the stated number of carbon atoms in each alkyl group; -COD; $(C_1-C_4)$alkoxy-carbonyl; $(C_1-C_4)$alkanoyloxy; thiocyanato; unsubstituted or substituted phenyl having one or two of the same or different halo, nitro, $(C_1-C_4)$alkyl, $(C_1-C_4)$alkoxy, carboxy, $(C_1-C_4)$alkoxy-carbonyl, $(C_1-C_4)$-alkanoyloxy, $NH_2$, NHZ or NZZ'; or phenoxy where the phenyl ring is unsubstituted or substituted with one or two of the same or different halo, nitro, $(C_1-C_4)$alkyl, $(C_1-C_4)$alkoxy, carboxy, $(C_1-C_4)$alkoxy-carbonyl, $(C_1-C_4)$alkanoyloxy, $NH_2$, NHZ or NZZ';

where D is hydrogen or $(C_1\text{-}C_4)$alkyl; and Z and Z' are $(C_1\text{-}C_4)$alkyl.

**4.** The use according to claim 3 wherein

| | |
|---|---|
| X and X' are | O; |
| $R^1$ is | t-butyl; neopentyl (2,2-dimethylpropyl) or 1,2,2-trimethylpropyl; |
| $R^2$ is | methyl; methoxymethyl; $(C_2\text{-}C_4)$alkenyl; $(C_2\text{-}C_5)$alkynyl; benzyl or 4-halobenzyl; and |
| A and B are | the same or different phenyl or substituted phenyl where the substituents can be from one to three of the same or different halo, nitro, $(C_1\text{-}C_4)$alkyl, $(C_1\text{-}C_4)$alkoxy, or halo-$(C_1\text{-}C_4)$alkyl. |

**5.** The use according to claim 4 wherein

| | |
|---|---|
| $R^1$ is | t-butyl; |
| $R^2$ is | $(C_2\text{-}C_5)$alkynyl; and |
| A and B are | unsubstituted or substituted phenyl where the substituents can be one or two of the same or different chloro, fluoro, bromo, iodo, methyl, ethyl, methoxy or trifluoromethyl. |

**6.** The use according to claim 5 wherein

| | |
|---|---|
| X and X' are | O; |
| $R^1$ is | t-butyl; and |
| $R^2$ is | 2-propynyl; and (a) |
| A is | 4-methylphenyl and |
| B is | 3-methylphenyl; or (b) |
| A is | phenyl and |
| B is | phenyl, 2,4-dichlorophenyl or 3,4-dichlorophenyl. |

**7.** The use of an insecticidally active compound for controlling insects by contacting said insects with an insecticidally effective amount of insecticidally active compound as defined in any of claims 1 to 6 optionally in a composition also containing agronomically acceptable diluent or carrier.

**8.** A mechanical process for improving the commercial value and/or profitability of vendible crops from plants whose growth is affected or likely to be affected by insects which comprises (1) charging to a container, fumigation device or mechanical dissemination device an insecticidal compound as defined in any of claims 1 to 6 optionally in a mixture with agronomically acceptable diluent or carrier (2) using the container, fumigator or mechanical dissemination device to apply the insecticidal compound or composition, in the form of granules, dust, smoke, vapour or surfactant-containing liquid preparation to growing plants or to a growth medium where the plants are growing or are to be grown, or to the insects themselves, (3) controlling the dose of the active ingredient during this application step so that the rate of application of active insecticidal compound is sufficient to combat the insects but is insufficient to cause an unacceptably adverse effect on the crop plants growing or to be grown in the treated area.

**9.** The use or process according to any preceding claim wherein the composition contains from 0.001 to 90%, preferably 0.01 to 75%, by weight of the insecticidal compound.

**10.** The use or process according to any preceding claim wherein the composition (a) additionally contains an emulsifying agent, said composition being in the form of an emulsifiable concentrate; (b) additionally contains a dispersing agent, said composition being in the form of a wettable powder; (c) contains a liquid agronomically acceptable carrier and a dispering agent, said composition being in the form of a flowable; (d) is in the form of a dust or granules or (e) additionally containing an attractant agent, said composition being in the form of a bait.

**11.** The use or process according to any of claims 7 to 10 wherein the insecticidally active compound is applied to growing plants or an area wherein plants are to be grown at a rate from 10 grams to about 10 kilograms per hectare of said active compound, preferably 100 grams to 5 kilograms per hectare.

**12.** The use or process according to any of claims 7 to 11 for controlling insects from the order

Lepidoptera or Coleoptera.

**Revendications**

**1.** Composé actif insecticide, qui est une hydrazine substituée ayant la formule suivante :

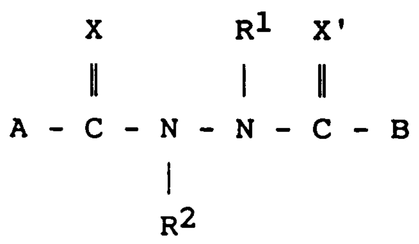

I

dans laquelle :

X et X' sont identiques ou différents et représentent chacun O, S ou NR ;

$R^1$ est un radical alkyle ramifié non substitué en $C_3$-$C_{10}$ ou un radical alkyle à chaîne droite en $C_1$-$C_4$ substitué par un ou deux radicaux cycloalkyle en $C_3$-$C_6$, qui sont identiques ou différents ;

$R^2$ est un radical alkyle en $C_1$-$C_6$ ; alcoxyalkyle ayant 1 à 6 atomes de carbone dans chaque groupe alkyle, indépendamment l'un de l'autre ; (alkyl en $C_1$-$C_6$)thioalkyle ayant 1 à 6 atomes de carbone dans chaque groupe alkyle, indépendamment l'un de l'autre ; alcényle en $C_2$-$C_6$ ; alcynyle en $C_2$-$C_6$ ; ou phén-(alkyle en $C_1$-$C_4$) où le noyau phényle est non substitué, ou substitué par 1 à 3 radicaux, identiques ou différents, choisis parmi les radicaux halogéno, cyano, nitro, hydroxy, alkyle en $C_1$-$C_4$, halogènoalkyle en $C_1$-$C_4$, alcoxy en $C_1$-$C_4$, halogéncalcoxy en $C_1$-$C_4$, carboxy, (alcoxy en $C_1$-$C_4$)-carbonyle, alcanoyloxy en $C_1$-$C_4$, $NH_2$, NHZ ou NZZ' ;

A et B sont identiques ou différents et représentent chacun un radical naphtyle non substitué, ou substitué dont les substituants peuvent être constitués d'un à cinq radicaux halogéno ; cyano ; nitro ; hydroxy ; alcoxy en $C_1$-$C_4$ ; alkyle en $C_1$-$C_4$ ; carboxy ; (alcoxy en $C_1$-$C_4$)carbonyle ; alcanoyloxy en $C_1$-$C_4$ ; $NH_2$ ; NHZ ou NZZ' ;

un radical phényle non substitué, ou substitué dont les substituants peuvent être constitués d'un à cinq radicaux identiques ou différents choisis parmi les radicaux halogéno ; nitroso ; nitro ; cyano ; hydroxy ; alkyle en $C_1$-$C_6$, halogénoalkyle en $C_1$-$C_6$ ; cyano-(alkyle en $C_1$-$C_6$) ; alcoxy en $C_1$-$C_6$ ; halogénoalcoxy en $C_1$-$C_6$ ; alcoxyalkyle ayant, indépendamment l'un de l'autre, 1 à 6 atomes de carbone dans chaque groupe alkyle ; alcoxyalcoxy ayant, indépendamment l'un de l'autre, 1 à 6 atomes de carbone dans chaque groupe alkyle ; un groupe $-OCO_2R$ ; un radical alcényle en $C_2$-$C_6$ éventuellement substitué par des radicaux halogéno, cyano, alkyle en $C_1$-$C_4$, alcoxy en $C_1$-$C_4$, halogénoalcoxy en $C_1$-$C_4$ ou alkylthio en $C_1$-$C_4$ ; (alcényl en $C_2$-$C_6$)-carbonyle ; alcadiényle en $C_2$-$C_6$ ; alcynyle en $C_2$-$C_6$ éventuellement substitué par des radicaux halogéno, cyano, nitro, hydroxy, alkyle en $C_1$-$C_4$, alcoxy en $C_1$-$C_4$, halogénoalcoxy en $C_1$-$C_4$ ou alkylthio en $C_1$-$C_4$ ; (alcynyl en $C_2$-$C_6$)-carbonyle ; carboxy ; un groupe $-ZCO_2R'$; un groupe -COR ; un radical halogéno-(alkyl en $C_1$-$C_6$)-carbonyle ; cyano-(alkyl en $C_1$-$C_6$)-carbonyle ; nitro-(alkyl en $C_1$-$C_6$)-carbonyle ; (alcoxy en $C_1$-$C_6$)-carbonyle ; halogéno-(alcoxy en $C_1$-$C_6$)-carbonyle ; un groupe -OCOR ; un groupe -NRR' ; un radical amino substitué par des groupes hydroxy, alcoxy en $C_1$-$C_4$ ou alkylthio en $C_1$-$C_4$ ; phénylamino ; diphénylamino ; un groupe -CONRR' ; un groupe -OCONRR' ; un groupe -C(NR)NR'R'' ; un groupe -N=NR ; le radical phénylazo ; un groupe -NRCOR' ; un groupe $-NRCO_2R'$ ; un groupe -N(COR)COR' ; un groupe -OCONRCOR' ; un radical sulfhydryle ; halogénothio ; thiocyanato ; alkylthio en $C_1$-$C_6$ ; halogéno-(alkyl en $C_1$-$C_6$)-thio ; un groupe -SOR ; un groupe - $SO_2R$ ; un radical phénylsulfonyle ; un groupe $-OSO_2R$ ; un radical halogéno-(alkyl en $C_1$-$C_6$)-sulfonyloxy ; un groupe $-SO_2NRR'$ ; un groupe -NRSOR' ; un groupe $-NRSO_2R'$; un groupe -CSR ; un groupe $-CS_2R$ ; un groupe -NRCSR' ; un groupe -SCOR ; un radical tri-(alkyl en $C_1$-$C_6$)-silyleayant, indépendamment les uns des autres, le nombre indiqué d'atomes de carbone dans chaque groupe alkyle ; phényle non substitué, ou phényle ayant un à trois substituants, identiques ou différents, choisis parmi les radicaux halogéno, cyano, nitro, hydroxy, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; phénoxy, où le noyau phényle est non substitué, ou substitué par un à trois substituants, identiques ou différents, choisis parmi les radicaux halogéno, cyano, nitro, hydroxy, alkyle en $C_1$-$C_4$, alcoxy en $C_1$-$C_4$, carboxy, (alcoxy en $C_1$-$C_4$)-carbonyle, alcanoyloxy en $c_1$-$C_4$, $NH_2$, NHZ ou NZZ' ;

benzoyle, où le noyau phényle est non substitué ou substitué par un à trois substituants, identiques ou différents, choisis parmi les radicaux halogéno, cyano, nitro, hydroxy, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; phénoxycarbonyle, où le noyau phényle est non substitué, ou substitué par un à trois substituants, identiques ou différents, choisis parmi les radicaux halogéno, cyano, nitro, hydroxy, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; phénylthio, où le noyau phényle est non substitué, ou substitué par un à trois substituants, identiques ou différents, choisis parmi les radicaux halogéno, cyano, nitro, hydroxy, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; un groupe -CR=N-$R^2$ où $R^2$ est un radical hydroxy, alkyle en $C_1$-$C_4$, alcoxy en $C_1$-$C_4$, -NRR', phénylamino, -COR, carboxy, (alcoxy en $C_1$-$C_4$)-carbonyle, alcanoyloxy en $C_1$-$C_4$, benzoyle, phénoxycarbonyle ou -CONRR' ; ou encore, quand deux positions contiguës du noyau phényle sont substituées par des groupes alcoxy, ces groupes peuvent être réunis pour former avec les atomes de carbone auxquels ils sont fixés des noyaux hétérocycliques dioxalanno ou dioxanno ayant 5 ou 6 chaînons ;

où R, R' et R" sont chacun un hydrogène ou un radical alkyle en $C_1$-$C_6$ ; Z et Z' représentent chacun un radical alkyle en $C_1$-$C_6$ ; plus la N-triméthylsiloxyméthyl-N'-2,2-diméthylpropyl-N-(4-méthoxy-3-chlorobenzoyl)-N'-benzoyl-hydrazine et la N-phénylthiométhyl-t-butyl-N-(4-toluoyl)-N'-(3,4-dichlorobenzoyl)-hydrazine, mais à l'exclusion de

$N(CHMe_2)COPH$
|
$N(CHMe_2)COPh$

et de

$N(CH_2CHMe_2)COPh$
|
$N(CH_2CHMe_2)COPh$

ou l'un de ses sels agronomiquement acceptables.

**2.** Composé selon la revendication 1, dans lequel :

X et X' représentent chacun O ou S ;

$R^1$ est un radical alkyle ramifié non substitué en $C_3$-$C_8$ ;

$R^2$ est un radical alkyle en $C_1$-$C_4$, (alcoxy en $C_1$-$C_4$)-(alkyle en $C_1$-$C_4$) ayant indépendamment l'un de l'autre le nombre d'atomes de carbone indiqué dans chaque groupe alkyle ; méthylthiométhyle ; alcényle en $C_2$-$C_5$ ; alcynyle en $C_2$-$C_5$ ; ou benzyle où le noyau phényle est non substitué, ou substitué par des substituants choisis parmi les radicaux halogéno, nitro, alkyle en $C_1$-$C_4$ ou alcoxy en $C_1$-$C_4$ ; et

A et B, qui sont identiques ou différents, sont choisis parmi le radical naphtyle non substitué ; ou le radical phényle non substitué, ou substitué par un à trois substituants, identiques ou différents, choisis parmi les radicaux halogéno ; nitro ; cyano ; alkyle en $C_1$-$C_4$ ; halogéno-(alkyle en $C_1$-$C_4$) ; cyano-(alkyle en $C_1$-$C_4$) ; alcoxy en $C_1$-$C_4$ ; halogéno-(alcoxy en $C_1$-$C_4$) ; un groupe -COD ; un radical carboxy ; (alcoxy en $C_1$-$C_4$)-carbonyle ; alcanoyloxy en $C_1$-$C_4$ ; alcényle en $C_2$-$C_6$ ; alcadiényle en $C_2$-$C_6$ ; alcynyle en $C_2$-$C_6$ ; un groupe -NDD' ; thiocyanato ; alkylthio en $C_1$-$C_4$ ; un groupe -CSD ; un groupe -$CS_2$D ; un groupe -SCOD ; tri-(alkyle en $C_1$-$C_4$)-silyle ayant, indépendamment les uns des autres, le nombre indiqué d'atomes de carbone dans chaque groupe alkyle ; phényle non substitué, ou phényle substitué ayant un ou deux substituants, identiques ou différents, choisis parmi les radicaux halogéno, nitro, alkyle en $C_1$-$C_4$,alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; phénoxy, où le noyau phényle est non substitué, ou substitué par un ou deux substituants, identiques ou différents, choisis parmi les radicaux halogéno, nitro, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; ou encore, quand deux positions contiguës du noyau phényle sont substituées par des groupes alcoxy, ces groupes peuvent être réunis pour former un noyau hétérocyclique dioxalanno ou dioxanno à 5 ou 6 chaînons ;

où D et D' représentent chacun un hydrogène ou un radical alkyle en $C_1$-$C_4$ ; et Z et Z' représentent chacun un radical alkyle en $C_1$-$C_4$.

3. Composé selon la revendication 2, dans lequel :
   $R^1$ est un radical alkyle ramifié en $C_4$-$C_7$ ;
   $R^2$ est un radical méthyle ; éthyle ; (alcoxy en $C_1$-$C_2$)-alkyle ayant, indépendamment l'un de l'autre, le nombre indiqué d'atomes de carbone dans chaque groupe alkyle ; alcényle en $C_2$-$C_5$ ; alcynyle en $C_2$-$C_5$ ; ou benzyle où le noyau phényle est non substitué, ou substitué par des radicaux halogéno ; et
   A et B, qui sont identiques ou différents, sont choisis parmi le radical naphtyle non substitué ; ou le radical phényle non substitué, ou substitué par un à trois substituants, identiques ou différents, choisis parmi les radicaux halogéno ; nitro ; cyano ; alkyle en $C_1$-$C_4$ ; halogéno-(alkyle en $C_1$-$C_4$) ; cyano-(alkyle en $C_1$-$C_4$) ; alcoxy en $C_1$-$C_4$ ; (alcoxy en $C_1$-$C_4$)-(alkyle en $C_1$-$C_4$) ayant, indépendamment l'un de l'autre, le nombre indiqué d'atomes de carbone dans chaque groupe alkyle ; -COD ; (alcoxy en $C_1$-$C_4$)-carbonyle ; alcanoyloxy en $C_1$-$C_4$) ; thiocyanato ; phényle non substitué ou phényle substitué ayant un ou deux substituants, identiques ou différents, choisis parmi les radicaux halogéno, nitro, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; ou phénoxy, où le noyau phényle est non substitué, ou substitué par un ou deux substituants, identiques ou différents, choisis parmi les radicaux halogéno, nitro, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; où D représente un hydrogène ou un radical alkyle en $C_1$-$C_4$ ; et Z et Z' représentent chacun un radical alkyle en $C_1$-$C_4$.

4. Composé selon la revendication 3, dans lequel X et X' sont chacun O ;
   $R^1$ est le radical tert-butyle ; néopentyl (2,2-diméthylpropyle) ou 1,2,2-triméthylpropyle ;
   $R^2$ est le radical méthyle ; méthoxyméthyle ; un radical alcényle en $C_2$-$C_4$ ; alcynyle en $C_2$-$C_5$ ; benzyle ou 4-halogénobenzyle ; et
   A et B, qui sont identiques ou différents, représentent chacun un radical phényle non substitué, ou substitué par un à trois substituants, identiques ou différents, pouvant être choisis parmi les radicaux halogéno, nitro, alkyle en $C_1$-$C_4$,alcoxy en $C_1$-$C_4$ ou halogéno-(alkyle en $C_1$-$C_4$).

5. Composé selon la revendication 4, dans lequel
   $R^1$ est le radical tert-butyle ;
   $R^2$ est un radical alcynyle en $C_2$-$C_5$ ; et
   A et B représentent chacun un radical phényle non substitué, ou substitué par un ou deux substituants, identiques ou différents, choisis parmi les radicaux chloro, fluoro, bromo, iodo, méthyle, éthyle, méthoxy ou trifluorométhyle.

6. Composé selon la revendication 5, dans lequel X et X' sont chacun O ;
   $R^1$ est le radical tert-butyle ; et
   $R^2$ est le radical 2-propynyle ; et (a)
   A est le radical 4-méthylphényle et
   B est le radical 3-méthylphényle ; ou (b)
   A est le radical phényle et
   B est le radical phényle,
   2,4-dichlorophényl ou 3,4-dichlorophényle.

7. Composition insecticide comprenant un diluant ou support agronomiquement acceptables et un composé insecticide selon l'une quelconque des revendications précédentes.

8. Composition selon la revendication 7, contenant de 0,001 à 90 et de préférence de 0,01 à 75 % en poids du composé insecticide.

9. Composition selon les revendications 7 ou 8, mais incluant

$N(CHMe_2)COPH$ | $N(CHMe_2)COPh$    $N(CH_2CHMe_2)COPh$ | $N(CH_2CHMe_2)COPh$

(a) contenant en outre un émulsifiant, ladite composition se présentant sous la forme d'un concentré émulsionnable ; (b) contenant en plus un dispersant, ladite composition se présentant sous la forme d'une poudre mouillable ; (c) contenant un support liquide agronomiquement acceptable et un dispersant, ladite composition se présentant sous la forme d'une bouillie ; (d) sous la forme d'une poudre ou de granulés ; ou (e) contenant en outre un agent attractif, ladite composition se présentant sous la forme d'un appât.

**10.** Procédé de maîtrise des insectes, qui consiste à mettre en contact lesdits insectes avec une quantité à effet insecticide d'une matière active insecticide selon l'une quelconque des revendications 1 à 6, mais incluant

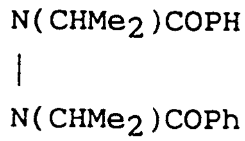
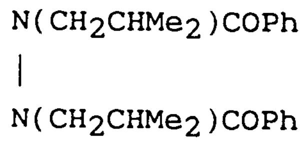

éventuellement dans une composition selon les revendications 7, 8 ou 9.

**11.** Procédé selon la revendication 10, dans lequel la matière active insecticide est appliquée sur des plantes en croissance ou sur une zone dans laquelle les plantes doivent croître, à une dose de 10 grammes à environ 10 kilogrammes par hectare de ladite matière active, de préférence de 100 grammes à 5 kilogrammes par hectare.

**12.** Procédé selon les revendications 10 ou 11, pour la maîtrise des insectes appartenant à l'ordre des lépidoptères ou des coléoptères.

**Revendications pour l'Etat contractant suivant : AT**

**1.** Composition insecticide, comprenant (a) une matière active insecticide, qui est une hydrazine substituée ayant la formule suivante :

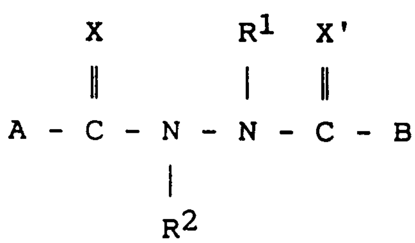

I

dans laquelle :

X et X' sont identiques ou différents et représentent chacun O, S ou NR ;

$R^1$ est un radical alkyle ramifié non substitué en $C_3$-$C_{10}$ ou un radical alkyle à chaîne droite en $C_1$-$C_4$ substitué par un ou deux radicaux cycloalkyle en $C_3$-$C_6$, qui sont identiques ou différents ;

$R^2$ est un radical alkyle en $C_1$-$C_6$ ; alcoxyalkyle ayant 1 à 6 atomes de carbone dans chaque groupe alkyle, indépendamment l'un de l'autre ; (alkyl en $C_1$-$C_6$)thioalkyle ayant 1 à 6 atomes de carbone dans chaque groupe alkyle, indépendamment l'un de l'autre ; alcényle en $C_2$-$C_6$ ; alcynyle en $C_2$-$C_6$ ; ou phén-(alkyle en $C_1$-$C_4$) où le noyau phényle est non substitué, ou substitué par 1 à 3 radicaux, identiques ou différents, choisis parmi les radicaux halogéno, cyano, nitro, hydroxy, alkyle en $C_1$-$C_4$, halogénoalkyle en $C_1$-$C_4$, alcoxy en $C_1$-$C_4$, halogénoalcoxy en $C_1$-$C_4$, carboxy, (alcoxy en $C_1$-$C_4$)-carbonyle, alcanoyloxy en $C_1$-$C_4$, $NH_2$, NHZ ou NZZ' ;

A et B sont identiques ou différents et représentent chacun un radical naphtyle non substitué, ou substitué dont les substituants peuvent être constitués d'un à cinq radicaux halogéno ; cyano ; nitro ; hydroxy ; alcoxy en $C_1$-$C_4$ ; alkyle en $C_1$-$C_4$ ; carboxy ; (alcoxy en $C_1$-$C_4$)carbonyle ; alcanoyloxy en $C_1$-$C_4$ ; $NH_2$ ; NHZ ou NZZ' ;

un radical phényle non substitué, ou substitué dont les substituants peuvent être constitués d'un à cinq radicaux identiques ou différents choisis parmi les radicaux halogéno ; nitroso ; nitro ; cyano ;

hydroxy ; alkyle en $C_1$-$C_6$, halogènalkyle en $C_1$-$C_6$ ; cyano-(alkyle en $C_1$-$C_6$) ; alcoxy en $C_1$-$C_6$ ; halogènalcoxy en $C_1$-$C_6$ ; alcoxyalkyle ayant, indépendamment l'un de l'autre, 1 à 6 atomes de carbone dans chaque groupe alkyle ; alcoxyalcoxy ayant, indépendamment l'un de l'autre, 1 à 6 atomes de carbone dans chaque groupe alkyle ; un groupe $-OCO_2R$ ; un radical alcényle en $C_2$-$C_6$ éventuellement substitué par des radicaux halogéno, cyano, alkyle en $C_1$-$C_4$, alcoxy en $C_1$-$C_4$, halogénoalcoxy en $C_1$-$C_4$ ou alkylthio en $C_1$-$C_4$ ; (alcényle en $C_2$-$C_6$)-carbonyle ; alcadiényle en $C_2$-$C_6$ ; alcynyle en $C_2$-$C_6$ éventuellement substitué par des radicaux halogéno, cyano, nitro, hydroxy, alkyle en $C_1$-$C_4$, alcoxy en $C_1$-$C_4$, halogénoalcoxy en $C_1$-$C_4$ ou alkylthio en $C_1$-$C_4$ ; (alcynyle en $C_2$-$C_6$)-carbonyle ; carboxy ; un groupe $-ZCO_2R'$ ; un groupe -COR ; un radical halogéno-(alkyl en $C_1$-$C_6$)-carbonyle ; cyano-(alkyl en $C_1$-$C_6$)-carbonyle ; nitro-(alkyl en $C_1$-$C_6$)-carbonyle ; (alcoxy en $C_1$-$C_6$)-carbonyle ; halogéno-(alcoxy en $C_1$-$C_6$)-carbonyle ; un groupe -OCOR ; un groupe -NRR' ; un radical amino substitué par des groupes hydroxy, alcoxy en $C_1$-$C_4$ ou alkylthio en $C_1$-$C_4$ ; phénylamino ; diphénylamino ; un groupe -CONRR' ; un groupe -OCONRR' ; un groupe -C(NR)NR'R" ; un groupe -N=NR ; le radical phénylazo ; un groupe -NRCOR' ; un groupe $-NRCO_2R'$ ; un groupe -N(COR)COR' ; un groupe -OCONRCOR' ; un radical sulfhydryle ; halogénothio ; thiocyanato ; alkylthio en $C_1$-$C_6$ ; halogéno-(alkyl en $C_1$-$C_6$)-thio ; un groupe -SOR; un groupe $-SO_2R$ ; un radical phénylsulfonyle ; un groupe $-OSO_2R$ ; un radical halogéno-(alkyl en $C_1$-$C_6$)-sulfonyloxy ; un groupe $-SO_2NRR'$ ; un groupe -NRSOR' ; un groupe $-NRSO_2R'$; un groupe -CSR ; un groupe $-CS_2R$ ; un groupe -NRCSR' ; un groupe -SCOR ; un radical tri-(alkyl en $C_1$-$C_6$)-silyleayant, indépendamment l'un de l'autre, le nombre indiqué d'atomes de carbone dans chaque groupe alkyle ; phényle non substitué, ou phényle ayant un à trois substituants, identiques ou différents, choisis parmi les radicaux halogéno, cyano, nitro, hydroxy, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; phénoxy, où le noyau phényle est non substitué, ou substitué par un à trois substituants, identiques ou différents, choisis parmi les radicaux halogéno, cyano, nitro, hydroxy, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; benzoyle, où le noyau phényle est non substitué ou substitué par un à trois substituants, identiques ou différents, choisis parmi les radicaux halogéno, cyano, nitro, hydroxy, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; phénoxycarbonyle, où le noyau phényle est non substitué, ou substitué par un à trois substituants, identiques ou différents, choisis parmi les radicaux halogéno, cyano, nitro, hydroxy, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; phénylthio, où le noyau phényle est non substitué, ou substitué par un à trois substituants, identiques ou différents, choisis parmi les radicaux halogéno, cyano, nitro, hydroxy, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; un groupe $-CR=N-R^2$ où $R^2$ est un radical hydroxy, alkyle en $C_1$-$C_4$, alcoxy en $C_1$-$C_4$, -NRR', phénylamino, -COR, carboxy, (alcoxy en $C_1$-$C_4$)-carbonyle, alcanoyloxy en $C_1$-$C_4$, benzoyle, phénoxycarbonyle ou -CONRR' ; ou encore, quand deux positions contiguës du noyau phényle sont substituées par des groupes alcoxy, ces groupes peuvent être réunis pour former avec les atomes de carbone auxquels ils sont fixés des noyaux hétérocycliques dioxalanno ou dioxanno ayant 5 ou 6 chaînons ;

où R, R' et R" sont chacun un hydrogène ou un radical alkyle en $C_1$-$C_6$ ; Z et Z' représentent chacun un radical alkyle en $C_1$-$C_6$ ; plus la N-triméthylsiloxyméthyl-N'-2,2-diméthylpropyl-N-(4-méthoxy-3-chlorobenzoyl)-N'-benzoyl-hydrazine et la N-phénylthiométhyl-t-butyl-N-(4-toluoyl)-N'-(3,4-dichlorobenzoyl)-hydrazine, mais à l'exclusion de

$N(CHMe_2)COPH$
|
$N(CHMe_2)COPh$

et de

$N(CH_2CHMe_2)COPh$
|
$N(CH_2CHMe_2)COPh$

ou l'un de ses sels agronomiquement acceptables, et (b) un diluant ou un support agronomiquement acceptables.

**2.** Composition selon la revendication 1, dans laquelle

X et X' représentent chacun O ou S ;
$R^1$ est un radical alkyle ramifié non substitué en $C_3$-$C_8$

$R^2$ est un radical alkyle en $C_1$-$C_4$, (alcoxy en $C_1$-$C_4$)-(alkyle en $C_1$-$C_4$) ayant indépendamment l'un de l'autre le nombre d'atomes de carbone indiqué dans chaque groupe alkyle ; méthylthiométhyle ; alcényle en $C_2$-$C_5$ ; alcynyle en $C_2$-$C_5$ ; ou benzyle où le noyau phényle est non substitué, ou substitué par des substituants choisis parmi les radicaux halogéno, nitro, alkyle en $C_1$-$C_4$ ou alcoxy en $C_1$-$C_4$ ; et

A et B, qui sont identiques ou différents, sont choisis parmi le radical naphtyle non substitué ; ou le radical phényle non substitué, ou substitué par un à trois substituants, identiques ou différents, choisis parmi les radicaux halogéno ; nitro ; cyano ; alkyle en $C_1$-$C_4$ ; halogéno-(alkyle en $C_1$-$C_4$) ; cyano-(alkyle en $C_1$-$C_4$) ; alcoxy en $C_1$-$C_4$ ; halogéno-(alcoxy en $C_1$-$C_4$) ; un groupe -COD ; un radical carboxy ; (alcoxy en $C_1$-$C_4$)-carbonyle ; alcanoyloxy en $C_1$-$C_4$ ; alcényle en $C_2$-$C_6$ ; alcadiényle en $C_2$-$C_6$ ; alcynyle en $C_2$-$C_6$ ; un groupe -NDD' ; thiocyanato ; alkylthio en $C_1$-$C_4$ ; un groupe -CSD ; un groupe -$CS_2$D ; un groupe -SCOD ; tri-(alkyl en $C_1$-$C_4$)-silyle ayant, indépendamment les uns des autres, le nombre indiqué d'atomes de carbone dans chaque groupe alkyle ; phényle non substitué, ou phényle substitué ayant un ou deux substituants, identiques ou différents, choisis parmi les radicaux halogéno, nitro, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; phénoxy, où le noyau phényle est non substitué, ou substitué par un ou deux substituants, identiques ou différents, choisis parmi les radicaux halogéno, nitro, alkyle en $C_1$-$C_4$, alcoxy en $C_1$-$C_4$, carboxy, (alcoxy en $C_1$-$C_4$)-carbonyle, alcanoyloxy en $C_1$-$C_{4\,1}$ $NH_2$, NHZ ou NZZ' ; ou encore, quand deux positions contiguës du noyau phényle sont substituées par des groupes alcoxy, ces groupes peuvent être réunis pour former un noyau hétérocyclique dioxalanno ou dioxanno à 5 ou 6 chaînons ;

où D et D' représentent chacun un hydrogène ou un radical alkyle en $C_1$-$C_4$ ; et Z et Z' représentent chacun un radical alkyle en $C_1$-$C_4$.

**3.** Composition selon la revendication 2, dans laquelle:

$R^1$ est un radical alkyle ramifié en $C_4$-$C_7$ ;

$R^2$ est un radical méthyle ; éthyle ; (alcoxy en $C_1$-$C_2$)-alkyle ayant, indépendamment l'un de l'autre, le nombre indiqué d'atomes de carbone dans chaque groupe alkyle ; alcényle en $C_2$-$C_5$ ; alcynyle en $C_2$-$C_5$ ; ou benzyle où le noyau phényle est non substitué, ou substitué par des radicaux halogéno ; et

A et B, qui sont identiques ou différents, sont choisis parmi le radical naphtyle non substitué ; ou le radical phényle non substitué,ou substitué par un à trois substituants, identiques ou différents, choisis parmi les radicaux halogéno ; nitro ; cyano ; alkyle en $C_1$-$C_4$ ; halogéno-(alkyle en $C_1$-$C_4$) ; cyano-(alkyle en $C_1$-$C_4$) ; alcoxy en $C_1$-$C_4$ ; (alcoxy en $C_1$-$C_4$)-(alkyle en $C_1$-$C_4$) ayant, indépendamment l'un de l'autre, le nombre indiqué d'atomes de carbone dans chaque groupe alkyle ; -COD ; (alcoxy en $C_1$-$C_4$)-carbonyle ; alcanoyloxy en $C_1$-$C_4$) ; thiocyanato ; phényle non substituè ou phényle substitué ayant un ou deux substituants, identiques ou différents, choisis parmi les radicaux halogéno, nitro, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; ou phénoxy, où le noyau phényle est non substitué, ou substitué par un ou deux substituants, identiques ou différents, choisis parmi les radicaux halogéno, nitro, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; où D représente un hydrogène ou un radical alkyle en $C_1$-$C_4$ ; et Z et Z' représentent chacun un radical alkyle en $C_1$-$C_4$.

**4.** Composition selon la revendication 3, dans laquelle X et X' sont chacun O ;

$R^1$ est le radical tert-butyle ; néopentyle (2,2-diméthylpropyle) ou 1,2,2-triméthylpropyle ;

$R^2$ est le radical méthyle ; méthoxyméthyle ; un radical alcényle en $C_2$-$C_4$ ; alcynyle en $C_2$-$C_5$ ; benzyle ou 4-halogénobenzyle ; et

A et B, qui sont identiques ou différents, représentent chacun un radical phényle non substitué, ou substitué par un à trois substituants, identiques ou différents, pouvant être choisis parmi les radicaux halogéno, nitro, alkyle en $C_1$-$C_4$,alcoxy en $C_1$-$C_4$ ou halogéno-(alkyle en $C_1$-$C_4$).

**5.** Composition selon la revendication 4, dans laquelle

$R^1$ est le radical tert-butyle ;

$R^2$ est un radical alcynyle en $C_2$-$C_5$ ; et

A et B représentent chacun un radical phényle non substitué, ou substitué par un ou deux

substituants, identiques ou différents, choisis parmi les radicaux chloro, fluoro, bromo, iodo, méthyle, éthyle, méthoxy ou trifluorométhyle.

**6.** Composition selon la revendication 5, dans laquelle X et X' sont chacun O ;
   $R^1$ est le radical tert-butyle ; et
   $R^2$ est le radical 2-propynyle ; et (a)
   A est le radical 4-méthylphényle et
   B est le radical 3-méthylphényle ; ou (b)
   A est le radical phényle et
   B est le radical phényle,
   2,4-dichlorophényl ou 3,4-dichlorophényle.

**7.** Composition selon l'une quelconque des revendications précédentes, contenant de 0,001 à 90 et de préférence de 0,01 à 75 % en poids du composé insecticide.

**8.** Composition selon l'une quelconque des revendications précédentes, mais incluant

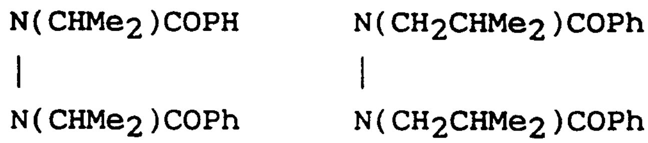

(a) contenant en outre un émulsifiant, ladite composition se présentant sous la forme d'un concentré émulsionnable ; (b) contenant en plus un dispersant, ladite composition se présentant sous la forme d'une poudre mouillable ; (c) contenant un support liquide agronomiquement acceptable et un dispersant, ladite composition se présentant sous la forme d'une bouillie ; (d) sous la forme d'une poudre ou de granulés ; ou (e) contenant en outre un agent attractif, ladite composition se présentant sous la forme d'un appât.

**9.** Procédé de maîtrise des insectes, qui consiste à mettre en contact lesdits insectes avec une quantité à effet insecticide d'une composition active insecticide selon l'une quelconque des revendications à 8.

**10.** Procédé selon la revendication 9, dans lequel la composition active insecticide est appliquée sur des plantes en croissance ou sur une zone dans laquelle les plantes doivent croître, à une dose de 10 grammes à environ 10 kilogrammes par hectare de ladite matière active, de préférence de 100 grammes à 5 kilogrammes par hectare.

**11.** Procédé selon les revendications 9 ou 10, pour la maîtrise des insectes appartenant à l'ordre des lépidoptères ou des coléoptères.

**Revendications pour l'Etat contractant suivant : ES**

**1.** Utilisation d'une matière active insecticide, qui est une hydrazine substituée ayant la formule suivante :

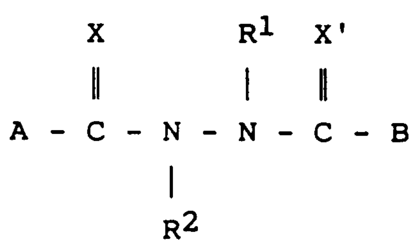

I

dans laquelle :

X et X' sont identiques ou différents et représentent chacun O, S ou NR ;

$R^1$ est un radical alkyle ramifié non substitué en $C_3$-$C_{10}$ ou un radical alkyle à chaîne droite en $C_1$-$C_4$ substitué par un ou deux radicaux cycloalkyle en $C_3$-$C_6$, qui sont identiques ou différents ;

$R^2$ est un radical alkyle en $C_1$-$C_6$ ; alcoxyalkyle ayant 1 à 6 atomes de carbone dans chaque groupe alkyle, indépendamment l'un de l'autre ; (alkyl en $C_1$-$C_6$)thioalkyle ayant 1 à 6 atomes de carbone dans chaque groupe alkyle, indépendamment l'un de l'autre ; alcényle en $C_2$-$C_6$ ; alcynyle en $C_2$-$C_6$ ; ou phén-(alkyle en $C_1$-$C_4$) où le noyau phényle est non substitué, ou substitué par 1 à 3 radicaux, identiques ou différents, choisis parmi les radicaux halogéno, cyano, nitro, hydroxy, alkyle en $C_1$-$C_4$, halogènalkyle en $C_1$-$C_4$, alcoxy en $C_1$-$C_4$, halogénoalcoxy en $C_1$-$C_4$, carboxy, (alcoxy en $C_1$-$C_4$)-carbonyle, alcanoyloxy en $C_1$-$C_4$, $NH_2$, NHZ ou NZZ' ;

A et B sont identiques ou différents et représentent chacun un radical naphtyle non substitué, ou substitué dont les substituants peuvent être constitués d'un à cinq radicaux halogéno ; cyano ; nitro ; hydroxy ; alcoxy en $C_1$-$C_4$ ; alkyle en $C_1$-$C_4$ ; carboxy ; (alcoxy en $C_1$-$C_4$)carbonyle ; alcanoyloxy en $C_1$-$C_4$ ; $NH_2$ ; NHZ ou NZZ' ;

un radical phényle non substitué, ou substitué dont les substituants peuvent être constitués d'un à cinq radicaux identiques ou différents choisis parmi les radicaux halogéno ; nitroso ; nitro ; cyano ; hydroxy ; alkyle en $C_1$-$C_6$, halogènalkyle en $C_1$-$C_6$ ; cyano-(alkyle en $C_1$-$C_6$) ; alcoxy en $C_1$-$C_6$ ; halogènalcoxy en $C_1$-$C_6$ ; alcoxyalkyle ayant, indépendamment l'un de l'autre, 1 à 6 atomes de carbone dans chaque groupe alkyle ; alcoxyalcoxy ayant, indépendamment l'un de l'autre, 1 à 6 atomes de carbone dans chaque groupe alkyle ; un groupe $-OCO_2R$ ; un radical alcényle en $C_2$-$C_6$ éventuellement substitué par des radicaux halogéno, cyano, alkyle en $C_1$-$C_4$, alcoxy en $C_1$-$C_4$, halogénoalcoxy en $C_1$-$C_4$ ou alkylthio en $C_1$-$C_4$ ; (alcényle en $C_2$-$C_6$)-carbonyle ; alcadiényle en $C_2$-$C_6$ ; alcynyle en $C_2$-$C_6$ éventuellement substitué par des radicaux halogéno, cyano, nitro, hydroxy, alkyle en $C_1$-$C_4$, alcoxy en $C_1$-$C_4$, halogénoalcoxy en $C_1$-$C_4$ ou alkylthio en $C_1$-$C_4$ ; (alcynyle en $C_2$-$C_6$)-carbonyle ; carboxy ; un groupe $-ZCO_2R'$ ; un groupe -COR ; un radical halogéno-(alkyl en $C_1$-$C_6$)-carbonyle ; cyano-(alkyl en $C_1$-$C_6$)-carbonyle ; nitro-(alkyle en $C_1$-$C_6$)-carbonyle ; (alcoxy en $C_1$-$C_6$)-carbonyle ; halogéno-(alcoxy en $C_1$-$C_6$)-carbonyle ; un groupe -OCOR ; un groupe -NRR' ; un radical amino substitué par des groupes hydroxy, alcoxy en $C_1$-$C_4$ ou alkylthio en $C_1$-$C_4$ ; phénylamino ; diphénylamino ; un groupe -CONRR' ; un groupe -OCONRR' ; un groupe -C(NR)NR'R'' ; un groupe -N=NR ; le radical phénylazo ; un groupe -NRCOR' ; un groupe $-NRCO_2R'$ ; un groupe -N(COR)COR' ; un groupe -OCONRCOR' ; un radical sulfhydryle ; halogénothio ; thiocyanato ; alkylthio en $C_1$-$C_6$ ; halogéno-(alkyle en $C_1$-$C_6$)-thio ; un groupe -SOR; un groupe $-SO_2R$ ; un radical phénylsulfonyle ; un groupe $-OSO_2R$ ; un radical halogéno-(alkyl en $C_1$-$C_6$)-sulfonyloxy ; un groupe $-SO_2NRR'$ ; un groupe -NRSOR' ; un groupe $-NRSO_2R'$; un groupe -CSR ; un groupe $-CS_2R$ ; un groupe -NRCSR' ; un groupe -SCOR ; un radical tri-(alkyl en $C_1$-$C_6$)-silyleayant, indépendamment l'un de l'autre, le nombre indiqué d'atomes de carbone dans chaque groupe alkyle ; phényle non substitué, ou phényle ayant un à trois substituants, identiques ou différents, choisis parmi les radicaux halogéno, cyano, nitro, hydroxy, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; phénoxy, où le noyau phényle est non substitué, ou substitué par un à trois substituants, identiques ou différents, choisis parmi les radicaux halogéno, cyano, nitro, hydroxy, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; benzoyle, où le noyau phényle est non substitué ou substitué par un à trois substituants, identiques ou différents, choisis parmi les radicaux halogéno, cyano, nitro, hydroxy, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; phénoxycarbonyle, où le noyau phényle est non substitué, ou substitué par un à trois substituants, identiques ou différents, choisis parmi les radicaux halogéno, cyano, nitro, hydroxy, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; phénylthio, où le noyau phényle est non substitué, ou substitué par un à trois substituants, identiques ou différents, choisis parmi les radicaux halogéno, cyano, nitro, hydroxy, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; un groupe $-CR=N-R^2$ où $R^2$ est un radical hydroxy, alkyle en $C_1$-$C_4$, alcoxy en $C_1$-$C_4$, -NRR', phénylamino, -COR, carboxy, (alcoxy en $C_1$-$C_4$)-carbonyle, alcanoyloxy en $C_1$-$C_4$, benzoyle, phénoxycarbonyle ou -CONRR' ; ou encore, quand deux positions contiguës du noyau phényle sont substituées par des groupes alcoxy, ces groupes peuvent être réunis pour former avec les atomes de carbone auxquels ils sont fixés des noyaux hétérocycliques dioxalanno ou dioxanno ayant 5 ou 6 chaînons ;

où R, R' et R'' sont chacun un hydrogène ou un radical alkyle en $C_1$-$C_6$ ; Z et Z' représentent chacun un radical alkyle en $C_1$-$C_6$ ; plus la N-triméthylsiloxyméthyl-N'-2,2-diméthylpropyl-N-(4-méthoxy-3-chlorobenzoyl)-N'-benzoyl-hydrazine et la N-phénylthiométhyl-t-butyl-N-(4-toluoyl)-N'-(3,4-dichlorobenzoyl)-hydrazine, ou l'un de ses sels agronomiquement acceptables ; avec un diluant ou un support agronomiquement acceptables, pour préparer une composition insecticide.

**2.** Utilisation selon la revendication 1, dans laquelle:

X et X' représentent chacun O ou S ;

$R^1$ est un radical alkyle ramifié non substitué en $C_3$-$C_8$ ;

$R^2$ est un radical alkyle en $C_1$-$C_4$, (alcoxy en $C_1$-$C_4$)-(alkyle en $C_1$-$C_4$) ayant indépendamment l'un de l'autre le nombre d'atomes de carbone indiqué dans chaque groupe alkyle ; méthylthiométhyle ; alcényle en $C_2$-$C_5$ ; alcynyle en $C_2$-$C_5$ ; ou benzyle où le noyau phényle est non substitué, ou substitué par des substituants choisis parmi les radicaux halogéno, nitro, alkyle en $C_1$-$C_4$ ou alcoxy en $C_1$-$C_4$ ; et

A et B, qui sont identiques ou différents, sont choisis parmi le radical naphtyle non substitué ; ou le radical phényle non substitué, ou substitué par un à trois substituants, identiques ou différents, choisis parmi les radicaux halogéno ; nitro ; cyano ; alkyle en $C_1$-$C_4$ ; halogéno-(alkyle en $C_1$-$C_4$) ; cyano-(alkyle en $C_1$-$C_4$) ; alcoxy en $C_1$-$C_4$ ; halogéno-(alcoxy en $C_1$-$C_4$) ; un groupe -COD ; un radical carboxy ; (alcoxy en $C_1$-$C_4$)-carbonyle ; alcanoyloxy en $C_1$-$C_4$ ; alcényle en $C_2$-$C_6$ ; alcadiényle en $C_2$-$C_6$ ; alcynyle en $C_2$-$C_6$ ; un groupe -NDD' ; thiocyanato ; alkylthio en $C_1$-$C_4$ ; un groupe -CSD ; un groupe -$CS_2$D ; un groupe -SCOD ; tri-(alkyl en $C_1$-$C_4$)-silyle ayant, indépendamment les uns des autres, le nombre indiqué d'atomes de carbone dans chaque groupe alkyle ; phényle non substitué, ou phényle substitué ayant un ou deux substituants, identiques ou différents, choisis parmi les radicaux halogéno, nitro, alkyle en $C_1$-$C_4$,alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; phénoxy, où le noyau phényle est non substitué, ou substitué par un ou deux substituants, identiques ou différents, choisis parmi les radicaux halogéno, nitro, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; ou encore, quand deux positions contiguës du noyau phényle sont substituées par des groupes alcoxy, ces groupes peuvent être réunis pour former un noyau hétérocyclique dioxalanno ou dioxanno à 5 ou 6 chaînons ;

où D et D' représentent chacun un hydrogène ou un radical alkyle en $C_1$-$C_4$ ; et Z et Z' représentent chacun un radical alkyle en $C_1$-$C_4$.

**3.** Utilisation selon la revendication 2, dans laquelle:

$R^1$ est un radical alkyle ramifié en $C_4$-$C_7$ ;

$R^2$ est un radical méthyle ; éthyle ; (alcoxy en $C_1$-$C_2$)-alkyle ayant, indépendamment l'un de l'autre, le nombre indiqué d'atomes de carbone dans chaque groupe alkyle ; alcényle en $C_2$-$C_5$ ; alcynyle en $C_2$-$C_5$ ; ou benzyle où le noyau phényle est non substitué, ou substitué par des radicaux halogéno ; et

A et B, qui sont identiques ou différents, sont choisis parmi le radical naphtyle non substitué ; ou le radical phényle non substitué, ou substitué par un à trois substituants, identiques ou différents, choisis parmi les radicaux halogéno ; nitro ; cyano ; alkyle en $C_1$-$C_4$ ; halogéno-(alkyle en $C_1$-$C_4$) ; cyano-(alkyle en $C_1$-$C_4$) ; alcoxy en $C_1$-$C_4$ ; (alcoxy en $C_1$-$C_4$)-(alkyle en $C_1$-$C_4$) ayant, indépendamment l'un de l'autre, le nombre indiqué d'atomes de carbone dans chaque groupe alkyle;-COD; (alcoxy en $C_1$-$C_4$)-carbonyle; alcanoyloxy en $C_1$-$C_4$) ; thiocyanato ; phényle non substitué ou phényle substitué ayant un ou deux substituants, identiques ou différents, choisis parmi les radicaux halogéno, nitro, alkyle en $C_1$-$C_4$, alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; ou phénoxy, où le noyau phényle est non substitué, ou substitué par un ou deux substituants, identiques ou différents, choisis parmi les radicaux halogéno, nitro, alkyle en $C_1$-$C_4$,alcoxy en $C_1$-$C_4$, carboxy, (alcoxy en $C_1$-$C_4$)-carbonyle, alcanoyloxy en $C_1$-$C_4$, $NH_2$, NHZ ou NZZ' ; où D représente un hydrogène ou un radical alkyle en $C_1$-$C_4$ ; et Z et Z' représentent chacun un radical alkyle en $C_1$-$C_4$.

**4.** Utilisation selon la revendication 3, dans laquelle X et X' sont chacun O ;

$R^1$ est le radical tert-butyle ; néopentyle (2,2-diméthylpropyle) ou 1,2,2-triméthylpropyle ;

$R^2$ est le radical méthyle ; méthoxyméthyle ; un radical alcényle en $C_2$-$C_4$ ; alcynyle en $C_2$-$C_5$ ; benzyle ou 4-halogénobenzyle ; et

A et B, qui sont identiques ou différents, représentent chacun un radical phényle non substitué, ou substitué par un à trois substituants, identiques ou différents, pouvant être choisis parmi les radicaux halogéno, nitro, alkyle en $C_1$-$C_4$,alcoxy en $C_1$-$C_4$ ou halogéno-(alkyle en $C_1$-$C_4$).

**5.** Utilisation selon la revendication 4, dans laquelle

$R^1$ est le radical tert-butyle ;

$R^2$ est un radical alcynyle en $C_2$-$C_5$ ; et

A et B représentent chacun un radical phényle non substitué, ou substitué par un ou deux substituants, identiques ou différents, choisis parmi les radicaux chloro, fluoro, bromo, iodo, méthyle, éthyle, méthoxy ou trifluorométhyle.

**6.** Utilisation selon la revendication 5, dans laquelle X et X' sont chacun O ;
$R^1$ est le radical tert-butyle ; et
$R^2$ est le radical 2-propynyle ; et (a)
A est le radical 4-méthylphényle et
B est le radical 3-méthylphényle ; ou (b)
A est le radical phényle et
B est le radical phényle,
2,4-dichlorophényl ou 3,4-dichlorophényle.

**7.** Utilisation d'une matière active insecticide pour la maîtrise d'insectes, par mise en contact desdits insectes avec une quantité à effet insecticide d'une matière active insecticide telle que définie dans l'une quelconque des revendications 1 à 6, éventuellement dans une composition contenant aussi un diluant ou un support agronomiquement acceptables.

**8.** Procédé mécanique destiné à améliorer la valeur industrielle et/ou la rentabilité de cultures de rapport, correspondant à des plantes dont la croissance est affectée ou est susceptible d'être affectée par des insectes, procédé qui consiste (1) à introduire dans un récipient, un dispositif de fumigation ou un dispositif de dissémination mécanique un composé insecticide tel que défini dans l'une quelconque des revendications 1 à 6, éventuellement dans un mélange avec un diluant ou un support agronomiquement acceptables ; (2) à utiliser le récipient, le fumigateur ou le dispositif de dissémination mécanique pour appliquer le composé insecticide ou la composition, sous forme de granulés, de poudre , de fumée, de vapeur ou d'une préparation liquide contenant un tensioactif, sur des plantes en croissance ou sur un milieu de croissance dans lequel les plantes croissent ou doivent croître, ou sur les insectes eux-mêmes ; (3) à réguler la dose de la matière active pendant l'étape d'application de façon que la dose d'application de la matière active insecticide soit suffisante pour combattre les insectes mais soit insuffisante pour provoquer un effet indésirable inacceptable sur les plantes de culture qui croissent ou doivent croître dans la zone traitée.

**9.** Utilisation ou procédé selon l'une quelconque des revendications précédentes, dans lequel la composition contient de 0,001 à 90 et de préférence de 0,01 à 75 % en poids du composé insecticide.

**10.** Utilisation ou procédé selon l'une quelconque des revendications précédentes, dans lequel la composition (a) contient en outre un émulsifiant, ladite composition se présentant sous la forme d'un concentré émulsionnable ; (b) contient en outre un dispersant, ladite composition se présentant sous la forme d'une poudre mouillable ; (c) contient un support liquide agronomiquement acceptable et un dispersant, ladite composition se présentant sous la forme d'une bouillie ; (d) se présente sous la forme d'une poudre ou de granulés ou (e) contient en outre un attractif, ladite composition se présentant sous la forme d'un appât.

**11.** Utilisation ou procédé selon l'une quelconque des revendications 7 à 10, dans lequel la matière active insecticide est appliquée sur des plantes en croissance ou sur une zone dans laquelle les plantes doivent croître, à une dose de 10 grammes à environ 10 kilogrammes par hectare de ladite matière active, de préférence de 100 grammes à 5 kilogrammes par hectare.

**12.** Utilisation ou procédé selon l'une quelconque des revendications 7 à 11, pour la maîtrise des insectes appartenant aux ordres des Lépidoptères ou des Coléoptères.

**Patentansprüche**

**1.** Insektizid-wirksame Verbindung, nämlich ein substituiertes Hydrazin der Formel:

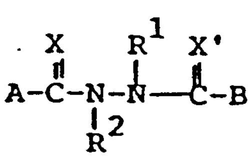

I

worin

X und X' gleich oder verschieden voneinander O, S oder NR sind,

$R^1$ ein unsubstituiertes, verzweigtes ($C_3$-$C_{10}$)Alkyl oder ein geradkettiges, mit ein oder zwei der gleichen oder verschiedenen von ($C_3$-$C_6$)Cycloalkyl substituiertes ($C_1$-$C_4$)Alkyl ist,

$R^2$ ($C_1$-$C_6$)Alkyl, Alkoxyalkyl mit, unabhängig davon, 1 bis 6 C-Atomen in jedem Alkyl-Rest, ($C_1$-$C_6$)Alkylthioalkyl mit, unabhängig davon, 1 bis 6 C-Atomen in jedem Alkyl-Rest, ($C_2$-$C_6$)Alkenyl, ($C_2$-$C_6$)Alkynyl oder Phen($C_1$-$C_4$)alkyl ist, wo der Phenylring unsubstituiert oder mit ein bis drei der gleichen oder verschiedenen von Halogen, Cyano, Nitro, Hydroxy, ($C_1$-$C_4$)Alkyl, Halogen($C_1$-$C_4$)alkyl ($C_1$-$C_4$)Alkoxy, Halogen($C_1$-$C_4$)alkoxy, Carboxy, ($C_1$-$C_4$)Alkoxycarbonyl, ($C_1$-$C_4$)Alkanoyloxy, $NH_2$, NHZ oder NZZ' substituiert ist,

A und B gleich oder verschieden voneinander ein unsubstituiertes oder substituiertes Naphthyl, wo die Substituenten ein bis fünf der gleichen oder verschiedenen von Halogen, Cyano, Nitro, Hydroxy, ($C_1$-$C_4$)- Alkoxy, $C_1$-$C_4$)Alkyl, Carboxy, ($C_1$-$C_4$)Alkoxy- carbonyl, ($C_1$-$C_4$)Alkanoyloxy, $NH_2$, NHZ oder NZZ' sein können,

ein unsubstituiertes oder substituiertes Phenyl, wo die Substituenten ein bis fünf der gleichen oder verschiedenen von Halogen, Nitroso, Nitro, Cyano, Hydroxy, ($C_1$-$C_6$)Alkyl, Halogen($C_1$-$C_6$)alkyl, Cyano($C_1$-$C_6$)alkyl, ($C_1$-$C_6$)Alkoxy, Halogen($C_1$-$C_6$)alkoxy, Alkoxyalkyl mit, unabhängig davon, 1 bis 6 C-Atomen in jedem Alkyl-Rest, Alk- oxyalkoxy mit, unabhängig davon, 1 bis 6 C-Atomen in jedem Alkyl-Rest, einem -$OCO_2$R-Rest, ($C_2$-$C_6$)- Alkenyl, gegebenenfalls substituiert mit Halogen, Cyano, ($C_1$-$C_4$)Alkyl, ($C_1$-$C_4$)-Alkoxy, Halogen($C_1$-$C_4$)alkoxy oder ($C_1$-$C_4$)Alkylthio, ($C_2$-$C_6$)Alkenylcarbonyl, ($C_2$-$C_6$)-Alkadienyl, ($C_2$-$C_6$)Alkynyl, gegebenenfalls substituiert mit Halogen, Cyano, Nitro, Hydroxy, ($C_1$-$C_4$)Alkyl, ($C_1$-$C_4$)Alkoxy, Halogen($C_1$-$C_4$)alkoxy oder ($C_1$-$C_4$)Alkylthio, ($C_2$-$C_6$)Alkynylcarbonyl, Carboxy, einem -$ZCO_2$R'-Rest, einem -COR-Rest, Halogen($C_1$-$C_6$)-alkylcarbonyl, Cyano($C_1$-$C_6$)alkylcarbonyl, Nitro($C_1$-$C_6$)alkylcarbonyl, ($C_1$-$C_6$)-Alkoxycarbonyl, Halogen($C_1$-$C_6$)alkoxycarbonyl, einem -OCOR-Rest, einem -NRR'-Rest, Amino, substituiert mit Hydroxy, ($C_1$-$C_4$)Alkoxy- oder ($C_1$-$C_4$)Alkylthio- Resten, Phenylamino, Diphenylamino, einem -CONRR'-Rest, einem -OCONRR'-Rest, einem -C(NR)-NR'R''-Rest, einem -N = NR-Rest, Phenylazo, einem -NRCOR'-Rest, einem -$NRCO_2$R'-Rest, einem -N(COR)COR'-Rest, einem -OCONRCOR'-Rest, Sulfhydryl, Halogenthio, Thiocyanato, ($C_1$-$C_6$)-Alkylthio, Halogen($C_1$-$C_6$)alkylthio, einem -SOR-Rest, einem -S $O_2$R-Rest, Phenylsulfonyl, einem -$OSO_2$R-Rest, ($C_1$-$C_6$)Halogenalkylsulfonyloxy, einem -$SO_2$NRR'-Rest, einem -NRSOR'-Rest, einem -$NRSO_2$R'-Rest, einem -CSR-Rest, einem -$CS_2$R-Rest, einem -NRCSR'-Rest, einem -SCOR-Rest, Tri($C_1$-$C_6$)alkylsilyl mit, unabhängig davon, der genannten Zahl von C-Atomen in jedem Alkyl-Rest, sein können, ein unsubstituiertes oder substituiertes Phenyl mit ein bis drei der gleichen oder verschiedenen von Halogen, Cyano, Nitro, Hydroxy, ($C_1$-$C_4$)Alkyl, ($C_1$- $C_4$)Alkoxy, Carboxy, ($C_1$-$C_4$)Alkoxy- carbonyl, ($C_1$-$C_4$)Alkanoyloxy, $NH_2$, NHZ oder NZZ', Phenoxy, wo der Phenylring unsubstituiert oder mit ein bis drei der gleichen oder verschiedenen von Halogen, Cyano, Nitro, Hydroxy, ($C_1$-$C_4$)Alkyl, ($C_1$-$C_4$)Alkoxy, Carboxy, ($C_1$-$C_4$)-Alkoxycarbon- yl, ($C_1$-$C_4$)Alkanoyloxy, $NH_2$, NHZ oder NZZ' substituiert ist, Benzoyl, wo der Phenylring unsubstituiert oder mit ein bis drei der gleichen oder verschiedenen von Halogen, Cyano, Nitro, Hydroxy, ($C_1$-$C_4$)Alkyl, ($C_1$-$C_4$)Alkoxy, Carboxy, ($C_1$-$C_4$)-Alkoxycarbonyl, ($C_1$-$C_4$)Alkanoyloxy, $NH_2$, NHZ oder NZZ' substituiert ist, Phenoxycarbonyl, wo der Phenylring unsubstituiert oder mit ein bis drei der gleichen oder verschiedenen von Halogen, Cyano, Nitro, Hydroxy, ($C_1$-$C_4$)Alkyl, ($C_1$-$C_4$)Alkoxy, Carboxy, ($C_1$- $C_4$)Alkoxycarbonyl, ($C_1$-$C_4$)Alkanoyloxy, $NH_2$, NHZ oder NZZ' substituiert ist, Phenylthio, wo der Phenylring unsubstituiert oder mit ein bis drei der gleichen oder verschiedenen von Halogen, Cyano, Nitro, Hydroxy, ($C_1$-$C_4$)Alkyl, ($C_1$-$C_4$)Alkoxy, Carboxy, ($C_1$-$C_4$)Alkoxycarbonyl, ($C_1$-$C_4$)Alkanoyloxy, $NH_2$, NHZ oder NZZ' substituiert ist, ein -CR = N-$R^2$-Rest, wo $R^2$ Hydroxy, ($C_1$-$C_4$)Alkyl, ($C_1$-$C_4$)Alkoxy, -NRR', Phenylamino, -COR, Carboxy, ($C_1$-$C_4$)Alkoxycarbonyl, ($C_1$-$C_4$)Alkanoyloxy, Benzoyl, Phenoxycarbonyl oder -CONRR' ist, sind, oder, wenn zwei benachbarte Positionen auf dem Phenylring mit Alkoxy-Resten substituiert sind, diese Reste verbunden sein können, um zusammen mit den C-Atomen, mit denen sie verknüpft sind, einen 5- oder 6-gliedrigen Dioxolano- oder Dioxano-heterocyclischen Ring auszubilden,

wo R, R' und R'' Wasserstoff oder ($C_1$-$C_6$)Alkyl sind, Z und Z' ($C_1$-$C_6$)Alkyl, plus N-

Trimethylsiloxymethyl-N'-2,2-dimethylpropyl-N-(4-methoxy-3-chlor-benzoyl)-N'-benzoylhydrazin und N-Phenylthiomethyl-t-butyl-N-(4-toluoyl)-N'-(3,4-dichlorbenzoyl)-hydrazin sind, wobei

$N(CHMe_2)COPh$
|
$N(CHMe_2)COPh$

und

$N(CH_2CHMe_2)COPh$
|
$N(CH_2CHMe_2)COPh$,

ausgenommen sind,

oder ein landwirtschaftlich verträgliches Salz davon.

2. Verbindung nach Anspruch 1, worin
X und X' O oder S sind,
$R^1$ ein unsubstituiertes, verzweigtes ($C_3$-$C_8$)Alkyl ist,
$R^2$ ($C_1$-$C_4$)Alkyl, ($C_1$-$C_4$)Alkoxy($C_1$-$C_4$)alkyl mit, unabhängig davon, der angegebenen Anzahl von C-Atomen in jedem Alkyl-Rest, Methylthiomethyl, ($C_2$-$C_5$)Alkenyl, ($C_2$-$C_5$)-Alkynyl oder Benzyl ist, wo der Phenylring unsubstituiert oder mit Halogen, Nitro, ($C_1$-$C_4$)Alkyl oder ($C_1$-$C_4$)Alkoxy substituiert ist, und
A und B gleich oder verschieden voneinander ein unsubstituiertes Naphthyl,

ein unsubstituiertes oder substituiertes Phenyl, wo die Substituenten ein bis drei der gleichen oder verschiedenen von Halogen, Nitro, Cyano, ($C_1$-$C_4$)Alkyl, Halogen($C_1$-$C_4$)-alkyl, Cyano($C_1$-$C_4$)alkyl, ($C_1$-$C_4$)Alkoxy, Halogen($C_1$-$C_4$)alkoxy, einem -COD-Rest, Carboxy, ($C_1$-$C_4$)Alkoxycarbonyl, ($C_1$-$C_4$)Alkanoyloxy, ($C_2$-$C_6$)Alkenyl, ($C_2$-$C_6$)Alkadienyl, ($C_2$-$C_6$)Alkynyl, einem -NDD'-Rest, Thiocyanato, ($C_1$-$C_4$)Alkylthio, einem -CSD-Rest, einem -$CS_2$D-Rest, einem -SCOD-Rest, Tri($C_1$-$C_4$)alkylsilyl mit, unabhängig davon, der angegebenen Anzahl von C-Atomen in jedem Alkyl-Rest sein können, ein unsubstituiertes oder substituiertes Phenyl mit ein bis zwei der gleichen oder verschiedenen von Halogen, Nitro, ($C_1$-$C_4$)Alkyl, ($C_1$-$C_4$)Alkoxy, Carboxy, ($C_1$-$C_4$)Alkoxycarbonyl, ($C_1$-$C_4$)-Alkanoyloxy, $NH_2$, NHZ oder NZZ', Phenoxy, wo der Phenylring unsubstituiert oder mit ein oder zwei der gleichen oder verschiedenen von Halogen, Nitro, ($C_1$-$C_4$)Alkyl, ($C_1$-$C_4$)Alkoxy, Carboxy, ($C_1$-$C_4$)Alkoxycarbonyl, ($C_1$-$C_4$)Alkanoyloxy, $NH_2$, NHZ oder NZZ' substituiert ist, sind, oder wenn zwei benachbarte Positionen auf dem Phenylring mit Alkoxy-Resten substituiert sind, diese Reste zur Ausbildung eines 5- oder 6-gliedrigen Dioxolano- oder Dioxano-heterocyclischen Ring verbunden sein können,

wo D und D' Wasserstoff oder ($C_1$-$C_4$)- Alkyl sind, und Z und Z' ($C_1$-$C_4$)Alkyl sind.

3. Verbindung nach Anspruch 2, worin
$R^1$ ein verzweigtes ($C_4$-$C_7$)Alkyl ist,
$R^2$ Methyl, Ethyl, ($C_1$-$C_2$)Alkoxyalkyl mit, unabhängig davon, der angegebenen Anzahl von C-Atomen in jedem Alkyl-Rest, ($C_2$-$C_5$)Alkenyl, ($C_2$-$C_5$)Alkynyl oder Benzyl ist, wo der Phenylring unsubstituiert oder mit Halogen substituiert ist, und
A und B gleich oder verschieden voneinander ein unsubstituiertes Naphthyl,

ein unsubstituiertes oder substituiertes Phenyl mit ein bis drei der gleichen oder verschiedenen von Halogen, Nitro, Cyano, ($C_1$-$C_4$)Alkyl, Halogen($C_1$-$C_4$)alkyl, Cyano($C_1$-$C_4$)alkyl, ($C_1$-$C_4$)Alkoxy, ($C_1$-$C_4$)Alkoxy-($C_1$-$C_4$)alkyl mit, unabhängig davon, der angegebenen Anzahl von C-Atomen in jedem Alkyl-Rest, -COD, ($C_1$-$C_4$)Alkoxycarbonyl, ($C_1$-$C_4$)Alkanoyloxy, Thiocyanato, ein unsubstituiertes oder substituiertes Phenyl mit ein oder zwei der gleichen oder verschiedenen von Halogen, Nitro, ($C_1$-$C_4$)Alkyl, ($C_1$-$C_4$)-

Alkoxy, Carboxy, ($C_1$-$C_4$)Alkoxycarbonyl, ($C_1$-$C_4$)Alkanoyloxy, $NH_2$, NHZ oder NZZ', oder Phenoxy, wo der Phenyl-Ring unsubstituiert oder mit ein oder zwei der gleichen oder verschiedenen von Halogen, Nitro, ($C_1$-$C_4$)Alkyl, ($C_1$-$C_4$)Alkoxy, Carboxy, ($C_1$-$C_4$)-Alkoxycarbonyl, ($C_1$-$C_4$)Alkanoyloxy, $NH_2$, NHZ, NZZ' substituiert ist, sind,

wo D Wasserstoff oder ($C_1$-$C_4$)Alkyl ist, und z und Z' ($C_1$-$C_4$)Alkyl sind.

**4.** Verbindung nach Anspruch 3, worin
X und X' 0 sind,
$R^1$ t-Butyl, Neopentyl(2,2-dimethylpropyl) oder 1,2,2,-Trimethylpropyl ist,
$R^2$ Methyl, Methoxymethyl, ($C_2$-$C_4$)Alkenyl, ($C_2$-$C_5$)Alkynyl, Benzyl oder 4-Halogenbenzyl ist, und
A und B gleich oder verschieden voneinander Phenyl oder ein substituiertes Phenyl sind, wo die Substituenten ein bis drei der gleichen oder verschiedenen von Halogen, Nitro, ($C_1$-$C_4$)-Alkyl, ($C_1$-$C_4$)Alkoxy oder Halogen($C_1$-$C_4$)alkyl sein können.

**5.** Verbindung nach Anspruch 4, worin
$R^1$ t-Butyl ist,
$R^2$ ($C_2$-$C_5$)Alkynyl ist, und
A und B ein unsubstituiertes oder substituiertes Phenyl sind, wo die Substituenten ein oder zwei der gleichen oder verschiedenen von Chlor, Fluor, Brom, Jod, Methyl, Ethyl, Methoxy oder Trifluormethyl sein können.

**6.** Verbindung nach Anspruch 5, worin
X und X' 0 sind,
$R^1$ t-Butyl ist, und
$R^2$ 2-Propynyl ist, und (a)
A 4-Methylphenyl ist, und
B 3-Methylphenyl ist, oder (b)
A Phenyl ist, und
B Phenyl, 2,4-Dichlorphenyl oder 3,4-Dichlorphenyl ist.

**7.** Insektizides Mittel, umfassend ein landwirtschaftlich verträgliches Verdünnungsmittel bzw. einen Träger und eine insektizide Verbindung nach einem der vorhergehenden Ansprüche.

**8.** Mittel nach Anspruch 7, enthaltend 0,001 bis 90 Gew.-%, vorzugsweise 0,01 bis 75 Gew.-% der insektiziden Verbindung.

**9.** Mittel nach Anspruch 7 oder 8, jedoch mit

$N(CHMe_2)COPh$ | $N(CHMe_2)COPh$ und $N(CH_2CHMe_2)COPh$ | $N(CH_2CHMe_2)COPh$,

zusätzlich enthaltend ein Emulgierungsmittel (a), wobei das Mittel in Form eines emulgierbaren Konzentrates vorliegt, zusätzlich enthaltend ein Dispersionsmittel (b), wobei das Mittel in Form eines benetzbaren Pulvers vorliegt, enthaltend einen flüssigen landwirtschaftlich verträglichen Träger und ein Dispersionsmittel (c), wobei das Mittel in fließbarer Form vorliegt, in Form von Staub oder Granulat (d) oder zusätzlich enthaltend einen Lockstoff (e), wobei das Mittel in Form eines Köders vorliegt.

**10.** Verfahren zur Kontrolle von Insekten, bei dem man die Insekten mit einer Insektizid-wirksamen Menge einer Insektizid-wirksamen Verbindung nach einem der Ansprüche 1 bis 6, jedoch mit

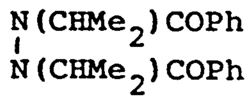 und 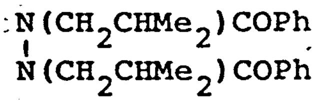

gegebenenfalls in einem Mittel nach Anspruch 7, 8 oder 9 in Kontakt bringt.

11. Verfahren nach Anspruch 10, worin die Insektizid-wirksame Verbindung wachsenden Pflanzen oder einem Bereich, wo Pflanzen wachsen sollen, mit einer Rate von 10 g bis etwa 10 kg der wirksamen Verbindung pro Hektar, vorzugsweise 100 g bis 5 kg pro Hektar verabreicht wird.

12. Verfahren nach Anspruch 10 oder 11 zur Kontrolle von Insekten der Ordnung Lepidoptera oder Coleoptera.

**Patentansprüche für folgenden Vertragsstaat : AT**

1. Insektizides Mittel, enthaltend (a) eine Insektizid-wirksamen Verbindung, nämlich eines substituierten Hydrazins der Formel:

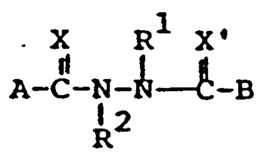 I,

worin

X und X' gleich oder verschieden voneinander O, S oder NR sind,

$R^1$ ein unsubstituiertes, verzweigtes ($C_3$-$C_{10}$)Alkyl oder ein geradkettiges, mit ein oder zwei der gleichen oder verschiedenen von ($C_3$-$C_6$)Cycloalkyl substituiertes ($C_1$-$C_4$)Alkyl ist,

$R^2$ ($C_1$-$C_6$)Alkyl, Alkoxyalkyl mit, unabhängig davon, 1 bis 6 C-Atomen in jedem Alkyl-Rest, ($C_1$-$C_6$)Alkylthioalkyl mit, unabhängig davon, 1 bis 6 C-Atomen in jedem Alkyl-Rest, ($C_2$-$C_6$)Alkenyl, ($C_2$-$C_6$)Alkynyl oder Phen($C_1$-$C_4$)alkyl ist, wo der Phenylring unsubstituiert oder mit ein bis drei der gleichen oder verschiedenen von Halogen, Cyano, Nitro, Hydroxy, ($C_1$-$C_4$)Alkyl, Halogen($C_1$-$C_4$)alkyl ($C_1$-$C_4$)Alkoxy, Halogen($C_1$-$C_4$)alkoxy, Carboxy, ($C_1$-$C_4$)Alkoxycarbonyl, ($C_1$-$C_4$)Alkanoyloxy, $NH_2$, NHZ oder NZZ' substituiert ist,

A und B gleich oder verschieden voneinander ein unsubstituiertes oder substituiertes Naphthyl, wo die Substituenten ein bis fünf der gleichen oder verschiedenen von Halogen, Cyano, Nitro, Hydroxy, ($C_1$-$C_4$)- Alkoxy, $C_1$-$C_4$)Alkyl, Carboxy, ($C_1$-$C_4$)Alkoxy- carbonyl, ($C_1$-$C_4$)Alkanoyloxy, $NH_2$, NHZ oder NZZ' sein können,

ein unsubstituiertes oder substituiertes Phenyl, wo die Substituenten ein bis fünf der gleichen oder verschiedenen von Halogen, Nitroso, Nitro, Cyano, Hydroxy, ($C_1$-$C_6$)Alkyl, Halogen($C_1$-$C_6$)alkyl, Cyano($C_1$-$C_6$)alkyl, ($C_1$-$C_6$)Alkoxy, Halogen($C_1$-$C_6$)alkoxy, Alkoxyalkyl mit, unabhängig davon, 1 bis 6 C-Atomen in jedem Alkyl-Rest, Alk- oxyalkoxy mit, unabhängig davon, 1 bis 6 C-Atomen in jedem Alkyl-Rest, einem -$OCO_2$R-Rest, ($C_2$-$C_6$)- Alkenyl, gegebenenfalls substituiert mit Halogen, Cyano, ($C_1$-$C_4$)Alkyl, ($C_1$-$C_4$)-Alkoxy, Halogen($C_1$-$C_4$)alkoxy oder ($C_1$-$C_4$)Alkylthio, ($C_2$-$C_6$)Alkenylcarbonyl, ($C_2$-$C_6$)-Alkadienyl, ($C_2$-$C_6$)Alkynyl, gegebenenfalls substituiert mit Halogen, Cyano, Nitro, Hydroxy, ($C_1$-$C_4$)Alkyl, ($C_1$-$C_4$)Alkoxy, Halogen($C_1$-$C_4$)alkoxy oder ($C_1$-$C_4$)Alkylthio, ($C_2$-$C_6$)Alkynylcarbonyl, Carboxy, einem -$ZCO_2$R'-Rest, einem -COR-Rest, Halogen($C_1$-$C_6$)-alkylcarbonyl, Cyano($C_1$-$C_6$)alkylcarbonyl, Nitro($C_1$-$C_6$)alkylcarbonyl, ($C_1$-$C_6$)-Alkoxycarbonyl, Halogen($C_1$-$C_6$)alkoxycarbonyl, einem -OCOR-Rest, einem -NRR'-Rest, Amino, substituiert mit Hydroxy, ($C_1$-$C_4$)Alkoxy- oder ($C_1$-$C_4$)Alkylthio- Resten, Phenylamino, Diphenylamino, einem -CONRR'-Rest, einem -OCONRR'-Rest, einem -C(NR)-NR'R''-Rest, einem -N=NR-Rest, Phenylazo, einem -NRCOR'-Rest, einem -$NRCO_2$R'-Rest, einem -N(COR)COR'-Rest, einem -OCONRCOR'-Rest, Sulfhydryl, Halogenthio, Thiocyanato, ($C_1$-$C_6$)Alkylthio,Halogen($C_1$-$C_6$)alkylthio, einem -SOR-Rest, einem -$SO_2$R-

Rest, Phenylsulfonyl, einem $-OSO_2R$-Rest, $(C_1-C_6)$Halogenalkylsulfonyloxy, einem $-SO_2NRR'$-Rest, einem -NRSOR'-Rest, einem $-NRSO_2R'$-Rest, einem -CSR-Rest, einem $-CS_2R$-Rest, einem -NRCSR'-Rest, einem -SCOR-Rest, Tri$(C_1-C_6)$alkylsilyl mit, unabhängig davon, der genannten Zahl von C-Atomen in jedem Alkyl-Rest, sein können, ein unsubstituiertes oder substituiertes Phenyl mit ein bis drei der gleichen oder verschiedenen von Halogen, Cyano, Nitro, Hydroxy, $(C_1-C_4)$Alkyl, $(C_1- C_4)$Alkoxy, Carboxy, $(C_1-C_4)$Alkoxy- carbonyl, $(C_1-C_4)$Alkanoyloxy, $NH_2$, NHZ oder NZZ', Phenoxy, wo der Phenylring unsubstituiert oder mit ein bis drei der gleichen oder verschiedenen von Halogen, Cyano, Nitro, Hydroxy, $(C_1-C_4)$Alkyl, $(C_1-C_4)$Alkoxy, Carboxy, $(C_1-C_4)$-Alkoxycarbon- yl, $(C_1-C_4)$Alkanoyloxy, $NH_2$, NHZ oder NZZ' substituiert ist, Benzoyl, wo der Phenylring unsubstituiert oder mit ein bis drei der gleichen oder verschiedenen von Halogen, Cyano, Nitro, Hydroxy, $(C_1-C_4)$Alkyl, $(C_1-C_4)$Alkoxy, Carboxy, $(C_1-C_4)$-Alkoxycarbonyl, $(C_1-C_4)$Alkanoyloxy, $NH_2$, NHZ oder NZZ' substituiert ist, Phenoxycarbonyl, wo der Phenylring unsubstituiert oder mit ein bis drei der gleichen oder verschiedenen von Halogen, Cyano, Nitro, Hydroxy, $(C_1-C_4)$Alkyl, $(C_1-C_4)$Alkoxy, Carboxy, $(C_1- C_4)$Alkoxycarbonyl, $(C_1-C_4)$Alkanoyloxy, $NH_2$, NHZ oder NZZ' substituiert ist, Phenylthio, wo der Phenylring unsubstituiert oder mit ein bis drei der gleichen oder verschiedenen von Halogen, Cyano, Nitro, Hydroxy, $(C_1-C_4)$Alkyl, $(C_1-C_4)$Alkoxy, Carboxy, $(C_1-C_4)$Alkoxycarbonyl, $(C_1-C_4)$Alkanoyloxy, $NH_2$, NHZ oder NZZ' substituiert ist, ein $-CR=N-R^2$-Rest, wo $R^2$ Hydroxy, $(C_1-C_4)$Alkyl, $(C_1-C_4)$Alkoxy, -NRR', Phenylamino, -COR, Carboxy, $(C_1-C_4)$Alkoxycarbonyl, $(C_1-C_4)$Alkanoyloxy, Benzoyl, Phenoxycarbonyl oder -CONRR' ist, sind, oder, wenn zwei benachbarte Positionen auf dem Phenylring mit Alkoxy-Resten substituiert sind, diese Reste verbunden sein können, um zusammen mit den C-Atomen, mit denen sie verknüpft sind, einen 5- oder 6-gliedrigen Dioxolano- oder Dioxano-heterocyclischen Ring auszubilden,

wo R, R' und R'' Wasserstoff oder $(C_1-C_6)$Alkyl sind, Z und Z' $(C_1-C_6)$Alkyl, plus N-Trimethylsiloxymethyl-N'-2,2-dimethylpropyl-N-(4-methoxy-3-chlorbenzoyl)-N'-benzoylhydrazin und N-Phenylthiomethyl-t- butyl-N-(4-toluoyl)- N'-(3,4-dichlorbenzoyl)-hydrazin sind,

wobei

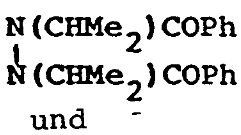

und

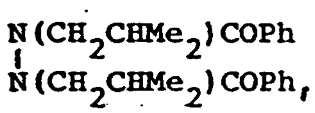

ausgenommen sind,

oder ein landwirtschaftlich verträgliches Salz davon, und (b) ein landwirtschaftlich verträgliches Verdünnungsmittel oder einen Träger.

**2.** Mittel nach Anspruch 1, worin

| | |
|---|---|
| X und X' | O oder S sind, |
| $R^1$ | ein unsubstituiertes, verzweigtes $(C_3-C_8)$Alkyl ist, |
| $R^2$ | $(C_1-C_4)$Alkyl, $(C_1-C_4)$Alkoxy$(C_1-C_4)$alkyl mit, unabhängig davon, der angegebenen Anzahl von C-Atomen in jedem Alkyl-Rest, Methylthiomethyl, $(C_2-C_5)$Alkenyl, $(C_2-C_5)$-Alkynyl oder Benzyl ist, wo der Phenylring unsubstituiert oder mit Halogen, Nitro, $(C_1-C_4)$Alkyl oder $(C_1-C_4)$Alkoxy substituiert ist, und |
| A und B | gleich oder verschieden voneinander ein unsubstituiertes Naphthyl, |

ein unsubstituiertes oder substituiertes Phenyl, wo die Substituenten ein bis drei der gleichen oder verschiedenen von Halogen, Nitro, Cyano, $(C_1-C_4)$Alkyl, Halogen$(C_1-C_4)$-alkyl, Cyano$(C_1-C_4)$alkyl, $(C_1-C_4)$Alkoxy, Halogen$(C_1-C_4)$alkoxy, einem -COD-Rest, Car-

boxy, $(C_1-C_4)$Alkoxycarbonyl, $(C_1-C_4)$Alkanoyloxy, $(C_2-C_6)$Alkenyl, $(C_2-C_6)$Alkadienyl, $(C_2-C_6)$Alkynyl, einem -NDD'-Rest, Thiocyanato, $(C_1-C_4)$Alkylthio, einem -CSD-Rest, einem -$CS_2$D-Rest, einem -SCOD-Rest, Tri$(C_1-C_4)$alkylsilyl mit, unabhängig davon, der angegebenen Anzahl von C-Atomen in jedem Alkyl-Rest sein können, ein unsubstituiertes oder substituiertes Phenyl mit ein bis zwei der gleichen oder verschiedenen von Halogen, Nitro, $(C_1-C_4)$Alkyl, $(C_1-C_4)$Alkoxy, Carboxy, $(C_1-C_4)$Alkoxycarbonyl, $(C_1-C_4)$-Alkanoyloxy, $NH_2$, NHZ oder NZZ', Phenoxy, wo der Phenylring unsubstituiert oder mit ein oder zwei der gleichen oder verschiedenen von Halogen, Nitro, $(C_1-C_4)$Alkyl, $(C_1-C_4)$Alkoxy, Carboxy, $(C_1-C_4)$Alkoxycarbonyl, $(C_1-C_4)$Alkanoyloxy, $NH_2$, NHZ oder NZZ' substituiert ist, sind, oder wenn zwei benachbarte Positionen auf dem Phenylring mit Alkoxy-Resten substituiert sind, diese Reste zur Ausbildung eines 5- oder 6-gliedrigen Dioxolano- oder Dioxano-heterocyclischen Ring verbunden sein können,

wo D und D' Wasserstoff oder $(C_1-C_4)$- Alkyl sind, und Z und Z' $(C_1-C_4)$Alkyl sind.

**3.** Mittel nach Anspruch 2, worin

$R^1$ ein verzweigtes $(C_4-C_7)$Alkyl ist,

$R^2$ Methyl, Ethyl, $(C_1-C_2)$Alkoxyalkyl mit, unabhängig davon, der angegebenen Anzahl von C-Atomen in jedem Alkyl-Rest, $(C_2-C_5)$Alkenyl, $(C_2-C_5)$Alkynyl oder Benzyl ist, wo der Phenylring unsubstituiert oder mit Halogen substituiert ist, und

A und B gleich oder verschieden voneinander ein unsubstituiertes Naphthyl,

ein unsubstituiertes oder substituiertes Phenyl mit ein bis drei der gleichen oder verschiedenen von Halogen, Nitro, Cyano, $(C_1-C_4)$Alkyl, Halogen$(C_1-C_4)$alkyl, Cyano$(C_1-C_4)$alkyl, $(C_1-C_4)$Alkoxy, $(C_1-C_4)$Alkoxy-$(C_1-C_4)$alkyl mit, unabhängig davon, der angegebenen Anzahl von C-Atomen in jedem Alkyl-Rest, -COD, $(C_1-C_4)$Alkoxycarbonyl, $(C_1-C_4)$Alkanoyloxy, Thiocyanato, ein unsubstituiertes oder substituiertes Phenyl mit ein oder zwei der gleichen oder verschiedenen von Halogen, Nitro, $(C_1-C_4)$Alkyl, $(C_1-C_4)$-Alkoxy, Carboxy, $(C_1-C_4)$Alkoxycarbonyl, $(C_1-C_4)$Alkanoyloxy, $NH_2$, NHZ oder NZZ', oder Phenoxy, wo der Phenyl-Ring unsubstituiert oder mit ein oder zwei der gleichen oder verschiedenen von Halogen, Nitro, $(C_1-C_4)$Alkyl, $(C_1-C_4)$Alkoxy, Carboxy, $(C_1-C_4)$-Alkoxycarbonyl, $(C_1-C_4)$Alkanoyloxy, $NH_2$, NHZ, NZZ' substituiert ist, sind,

wo D Wasserstoff oder $(C_1-C_4)$Alkyl ist, und Z und Z' $(C_1-C_4)$Alkyl sind.

**4.** Mittel nach Anspruch 3, worin

X und X' 0 sind,

$R^1$ t-Butyl, Neopentyl(2,2-dimethylpropyl) oder 1,2,2,-Trimethylpropyl ist,

$R^2$ Methyl, Methoxymethyl, $(C_2-C_4)$Alkenyl, $(C_2-C_5)$Alkynyl, Benzyl oder 4-Halogenbenzyl ist, und

A und B gleich oder verschieden voneinander Phenyl oder ein substituiertes Phenyl sind, wo die Substituenten ein bis drei der gleichen oder verschiedenen von Halogen, Nitro, $(C_1-C_4)$-Alkyl, $(C_1-C_4)$Alkoxy oder Halogen$(C_1-C_4)$alkyl sein können.

**5.** Mittel nach Anspruch 4, worin

$R^1$ t-Butyl ist,

$R^2$ $(C_2-C_5)$Alkynyl ist, und

A und B ein unsubstituiertes oder substituiertes Phenyl sind, wo die Substituenten ein oder zwei der gleichen oder verschiedenen von Chlor, Fluor, Brom, Jod, Methyl, Ethyl, Methoxy oder Trifluormethyl sein können.

**6.** Mittel nach Anspruch 5, worin

X und X' 0 sind,

$R^1$ t-Butyl ist, und

$R^2$ 2-Propynyl ist, und (a)

A 4-Methylphenyl ist, und

B 3-Methylphenyl ist, oder (b)

A Phenyl ist, und

B Phenyl, 2,4-Dichlorphenyl oder 3,4-Dichlorphenyl ist.

**7.** Mittel nach einem der vorhergehenden Ansprüche, enthaltend 0,001 bis 90 Gew.-%, vorzugsweise 0,01 bis 75 Gew.-% der insektiziden Verbindung.

**8.** Mittel nach einem der vorhergehenden Ansprüche, jedoch mit

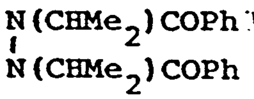 und 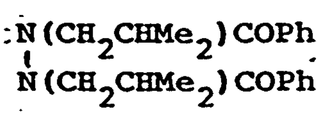

zusätzlich enthaltend ein Emulgierungsmittel enthält (a), wobei das Mittel in Form eines emulgierbaren Konzentrates vorliegt, zusätzlich enthaltend ein Dispersionsmittel (b), wobei das Mittel in Form eines benetzbaren Pulvers vorliegt, einen flüssigen landwirtschaftlich vertraglichen Träger und ein Dispersionsmittel enthält (c), wobei das Mittel in fließbarer Form vorliegt, in Form von Staub oder Granulat vorliegt (d) oder zusätzlich enthaltend einen Lockstoff (e), wobei das Mittel in Form eines Köders vorliegt.

**9.** Verfahren zur Kontrolle von Insekten, bei dem man die Insekten mit einer Insektizid-wirksamen Menge eines Insektizid-wirksamen Mittels nach einem der Ansprüche 1 bis 8.

**10.** verfahren nach Anspruch 9, worin das Insektizidwirksame Mittel wachsenden Pflanzen oder einem Bereich, wo Pflanzen wachsen sollen, mit einer Rate von 10 g bis etwa 10 kg der wirksamen Verbindung pro Hektar, vorzugsweise 100 g bis 5 kg pro Hektar verabreicht wird.

**11.** Verfahren nach Anspruch 9 oder 10 zur Kontrolle von Insekten der Ordnung Lepidoptera oder Coleoptera.

**Patentansprüche für folgenden Vertragsstaat : ES**

**1.** Verwendung einer Insektizid-wirksamen Verbindung, nämlich eines substituierten Hydrazins der Formel:

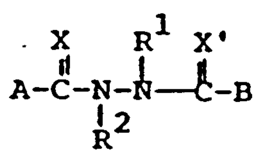

I

worin

X und X' gleich oder verschieden voneinander O, S oder NR sind,

$R^1$ ein unsubstituiertes, verzweigtes ($C_3$-$C_{10}$)Alkyl oder ein geradkettiges, mit ein oder zwei der gleichen oder verschiedenen von ($C_3$-$C_6$)Cycloalkyl substituiertes ($C_1$-$C_4$)Alkyl ist,

$R^2$ ($C_1$-$C_6$)Alkyl, Alkoxyalkyl mit, unabhängig davon, 1 bis 6 C-Atomen in jedem Alkyl-Rest, ($C_1$-$C_6$)Alkylthioalkyl mit, unabhängig davon, 1 bis 6 C-Atomen in jedem Alkyl-Rest, ($C_2$-$C_6$)Alkenyl, ($C_2$-$C_6$)Alkynyl oder Phen($C_1$-$C_4$)alkyl ist, wo der Phenylring unsubstituiert oder mit ein bis drei der gleichen oder verschiedenen von Halogen, Cyano, Nitro, Hydroxy, ($C_1$-$C_4$)Alkyl, Halogen($C_1$-$C_4$)alkyl ($C_1$-$C_4$)Alkoxy, Halogen($C_1$-$C_4$)alkoxy, Carboxy, ($C_1$-$C_4$)Alkoxycarbonyl, ($C_1$-$C_4$)Alkanoyloxy, $NH_2$, NHZ oder NZZ' substituiert ist,

A und B gleich oder verschieden voneinander ein unsubstituiertes oder substituiertes Naphthyl, wo die Substituenten ein bis fünf der gleichen oder verschiedenen von Halogen, Cyano, Nitro, Hydroxy, ($C_1$-$C_4$)- Alkoxy, $C_1$-$C_4$)Alkyl, Carboxy, ($C_1$-$C_4$)Alkoxy- carbonyl, ($C_1$-$C_4$)Alkanoyloxy, $NH_2$, NHZ oder NZZ' sein können,

ein unsubstituiertes oder substituiertes Phenyl, wo die Substituenten ein bis fünf der gleichen oder verschiedenen von Halogen, Nitroso, Nitro, Cyano, Hydroxy, ($C_1$-$C_6$)Alkyl, Halogen($C_1$-$C_6$)alkyl, Cyano($C_1$-$C_6$)alkyl, ($C_1$-$C_6$)Alkoxy, Halogen($C_1$-$C_6$)alkoxy, Alkoxyalkyl mit, unabhängig davon, 1 bis 6 C-Atomen in jedem Alkyl-Rest, Alk- oxyalkoxy mit,

unabhängig davon, 1 bis 6 C-Atomen in jedem Alkyl-Rest, einem $-OCO_2R$-Rest, ($C_2$-$C_6$)- Alkenyl, gegebenenfalls substituiert mit Halogen, Cyano, ($C_1$-$C_4$)Alkyl, ($C_1$-$C_4$)-Alkoxy, Halogen($C_1$-$C_4$)alkoxy oder ($C_1$-$C_4$)Alkylthio, ($C_2$-$C_6$)Alkenylcarbonyl, ($C_2$-$C_6$)-Alkadienyl, ($C_2$-$C_6$)Alkynyl, gegebenenfalls substituiert mit Halogen, Cyano, Nitro, Hydroxy, ($C_1$-$C_4$)Alkyl, ($C_1$-$C_4$)Alkoxy, Halogen($C_1$-$C_4$)alkoxy oder ($C_1$-$C_4$)Alkylthio, ($C_2$-$C_6$)Alkynylcarbonyl, Carboxy, einem $-ZCO_2R'$-Rest, einem -COR-Rest, Halogen($C_1$-$C_6$)-alkylcarbonyl, Cyano($C_1$-$C_6$)alkylcarbonyl, Nitro($C_1$-$C_6$)alkylcarbonyl, ($C_1$-$C_6$)-Alkoxycarbonyl, Halogen($C_1$-$C_6$)alkoxycarbonyl, einem -OCOR-Rest, einem -NRR'-Rest, Amino, substituiert mit Hydroxy, ($C_1$-$C_4$)Alkoxy- oder ($C_1$-$C_4$)Alkylthio- Resten, Phenylamino, Diphenylamino, einem -CONRR'-Rest, einem -OCONRR'-Rest, einem -C(NR)-NR'R''-Rest, einem -N = NR-Rest, Phenylazo, einem -NRCOR'-Rest, einem $-NRCO_2R'$-Rest, einem -N(COR)COR'-Rest, einem -OCONRCOR'-Rest, Sulfhydryl, Halogenthio, Thiocyanato, ($C_1$-$C_6$)Alkylthio,Halogen($C_1$-$C_6$)alkylthio, einem -SOR-Rest, einem $-SO_2R$-Rest, Phenylsulfonyl, einem $-OSO_2R$-Rest, ($C_1$-$C_6$)Halogenalkylsulfonyloxy, einem $-SO_2NRR'$-Rest, einem -NRSOR'-Rest, einem $-NRSO_2R'$-Rest, einem -CSR-Rest, einem $-CS_2R$-Rest, einem -NRCSR'-Rest, einem -SCOR-Rest, Tri($C_1$-$C_6$)alkylsilyl mit, unabhängig davon, der genannten Zahl von C-Atomen in jedem Alkyl-Rest, sein können, ein unsubstituiertes oder substituiertes Phenyl mit ein bis drei der gleichen oder verschiedenen von Halogen, Cyano, Nitro, Hydroxy, ($C_1$-$C_4$)Alkyl, ($C_1$- $C_4$)Alkoxy, Carboxy, ($C_1$-$C_4$)Alkoxy- carbonyl, ($C_1$-$C_4$)Alkanoyloxy, $NH_2$, NHZ oder NZZ', Phenoxy, wo der Phenylring unsubstituiert oder mit ein bis drei der gleichen oder verschiedenen von Halogen, Cyano, Nitro, Hydroxy, ($C_1$-$C_4$)Alkyl, ($C_1$-$C_4$)Alkoxy, Carboxy, ($C_1$-$C_4$)-Alkoxycarbon- yl, ($C_1$-$C_4$)Alkanoyloxy, $NH_2$, NHZ oder NZZ' substituiert ist, Benzoyl, wo der Phenylring unsubstituiert oder mit ein bis drei der gleichen oder verschiedenen von Halogen, Cyano, Nitro, Hydroxy, ($C_1$-$C_4$)Alkyl, ($C_1$-$C_4$)Alkoxy, Carboxy, ($C_1$-$C_4$)-Alkoxycarbonyl, ($C_1$-$C_4$)Alkanoyloxy, $NH_2$, NHZ oder NZZ' substituiert ist, Phenoxycarbonyl, wo der Phenylring unsubstituiert oder mit ein bis drei der gleichen oder verschiedenen von Halogen, Cyano, Nitro, Hydroxy, ($C_1$-$C_4$)Alkyl, ($C_1$-$C_4$)Alkoxy, Carboxy, ($C_1$- $C_4$)Alkoxycarbonyl, ($C_1$-$C_4$)Alkanoyloxy, $NH_2$, NHZ oder NZZ' substituiert ist, Phenylthio, wo der Phenylring unsubstituiert oder mit ein bis drei der gleichen oder verschiedenen von Halogen, Cyano, Nitro, Hydroxy, ($C_1$-$C_4$)Alkyl, ($C_1$-$C_4$)Alkoxy, Carboxy, ($C_1$-$C_4$)Alkoxycarbonyl, ($C_1$-$C_4$)Alkanoyloxy, $NH_2$, NHZ oder NZZ' substituiert ist, ein -CR = N-$R^2$-Rest, wo $R^2$ Hydroxy, ($C_1$-$C_4$)Alkyl, ($C_1$-$C_4$)Alkoxy, -NRR', Phenylamino, -COR, Carboxy, ($C_1$-$C_4$)Alkoxycarbonyl, ($C_1$-$C_4$)Alkanoyloxy, Benzoyl, Phenoxycarbonyl oder -CONRR' ist, sind, oder, wenn zwei benachbarte Positionen auf dem Phenylring mit Alkoxy-Resten substituiert sind, diese Reste verbunden sein können, um zusammen mit den C-Atomen, mit denen sie verknüpft sind, einen 5- oder 6-gliedrigen Dioxolano- oder Dioxano-heterocyclischen Ring auszubilden,

wo R, R' und R'' Wasserstoff oder ($C_1$-$C_6$)Alkyl sind, Z und Z' ($C_1$-$C_6$)Alkyl, plus N-Trimethylsiloxymethyl-N'-2,2-dimethylpropyl-N-(4-methoxy-3-chlorbenzoyl)-N'-benzoylhydrazin und N-Phenylthiomethyl-t-butyl-N-(4-toluoyl)-N'-(3,4-dichlorbenzoyl)-hydrazin sind,

oder eines landwirtschaftlich verträglichen Salzes davon, zusammen mit einem landwirtschaftlich verträglichen Verdünnungsmittel oder einem Träger zur Herstellung eines insektiziden Mittels.

**2.** Verwendung nach Anspruch 1, worin

X und X' O oder S sind,

$R^1$ ein unsubstituiertes, verzweigtes ($C_3$-$C_8$)Alkyl ist,

$R^2$ ($C_1$-$C_4$)Alkyl, ($C_1$-$C_4$)Alkoxy($C_1$-$C_4$)alkyl mit, unabhängig davon, der angegebenen Anzahl von C-Atomen in jedem Alkyl-Rest, Methylthiomethyl, ($C_2$-$C_5$)Alkenyl, ($C_2$-$C_5$)-Alkynyl oder Benzyl ist, wo der Phenylring unsubstituiert oder mit Halogen, Nitro, ($C_1$-$C_4$)Alkyl oder ($C_1$-$C_4$)Alkoxy substituiert ist, und

A und B gleich oder verschieden voneinander ein unsubstituiertes Naphthyl,

ein unsubstituiertes oder substituiertes Phenyl, wo die Substituenten ein bis drei der

gleichen oder verschiedenen von Halogen, Nitro, Cyano, $(C_1-C_4)$Alkyl, Halogen$(C_1-C_4)$-alkyl, Cyano$(C_1-C_4)$alkyl, $(C_1-C_4)$Alkoxy, Halogen$(C_1-C_4)$alkoxy, einem -COD-Rest, Carboxy, $(C_1-C_4)$Alkoxycarbonyl, $(C_1-C_4)$Alkanoyloxy, $(C_2-C_6)$Alkenyl, $(C_2-C_6)$Alkadienyl, $(C_2-C_6)$Alkynyl, einem -NDD'-Rest, Thiocyanato, $(C_1-C_4)$Alkylthio, einem -CSD-Rest, einem -$CS_2$D-Aest, einem -SCOD-Rest, Tri$(C_1-C_4)$alkylsilyl mit, unabhängig davon, der angegebenen Anzahl von C-Atomen in jedem Alkyl-Rest sein können, ein unsubstituiertes oder substituiertes Phenyl mit ein bis zwei der gleichen oder verschiedenen von Halogen, Nitro, $(C_1-C_4)$Alkyl, $(C_1-C_4)$Alkoxy, Carboxy, $(C_1-C_4)$Alkoxycarbonyl, $(C_1-C_4)$-Alkanoyloxy, $NH_2$, NHZ oder NZZ', Phenoxy, wo der phenylring unsubstituiert oder mit ein oder zwei der gleichen oder verschiedenen von Halogen, Nitro, $(C_1-C_4)$Alkyl, $(C_1-C_4)$Alkoxy, Carboxy, $(C_1-C_4)$Alkoxycarbonyl, $(C_1-C_4)$Alkanoyloxy, $NH_2$, NHZ oder NZZ' substituiert ist, sind, oder wenn zwei benachbarte Positionen auf dem Phenylring mit Alkoxy-Resten substituiert sind, diese Reste zur Ausbildung eines 5- oder 6-gliedrigen Dioxolano- oder Dioxano-heterocyclischen Ring verbunden sein können,

wo D und D' Wasserstoff oder $(C_1-C_4)$- Alkyl sind, und Z und Z'- $(C_1-C_4)$Alkyl sind.

**3.** Verwendung nach Anspruch 2, worin

- $R^1$ ein verzweigtes $(C_4-C_7)$Alkyl ist,
- $R^2$ Methyl, Ethyl, $(C_1-C_2)$Alkoxyalkyl mit, unabhängig davon, der angegebenen Anzahl von C-Atomen in jedem Alkyl-Aest, $(C_2-C_5)$Alkenyl, $(C_2-C_5)$Alkynyl oder Benzyl ist, wo der Phenylring unsubstituiert oder mit Halogen substituiert ist, und
- A und B gleich oder verschieden voneinander ein unsubstituiertes Naphthyl,

ein unsubstituiertes oder substituiertes Phenyl mit ein bis drei der gleichen oder verschiedenen von Halogen, Nitro, Cyano, $(C_1-C_4)$Alkyl, Halogen$(C_1-C_4)$alkyl, Cyano$(C_1-C_4)$alkyl, $(C_1-C_4)$Alkoxy, $(C_1-C_4)$Alkoxy-$(C_1-C_4)$alkyl mit, unabhängig davon, der angegebenen Anzahl von C-Atomen in jedem Alkyl-Rest, -COD, $(C_1-C_4)$Alkoxycarbonyl, $(C_1-C_4)$Alkanoyloxy, Thiocyanato, ein unsubstituiertes oder substituiertes Phenyl mit ein oder zwei der gleichen oder verschiedenen von Halogen, Nitro, $(C_1-C_4)$Alkyl, $(C_1-C_4)$-Alkoxy, Carboxy, $(C_1-C_4)$Alkoxycarbonyl, $(C_1-C_4)$Alkanoyloxy, $NH_2$, NHZ oder NZZ', oder Phenoxy, wo der Phenyl-Ring unsubstituiert oder mit ein oder zwei der gleichen oder verschiedenen von Halogen, Nitro, $(C_1-C_4)$Alkyl, $(C_1-C_4)$Alkoxy, Carboxy, $(C_1-C_4)$-Alkoxycarbonyl, $(C_1-C_4)$Alkanoyloxy, $NH_2$, NHZ, NZZ' substituiert ist, sind,

wo D Wasserstoff oder $(C_1-C_4)$Alkyl ist, und Z und Z' $(C_1-C_4)$Alkyl sind.

**4.** Verwendung nach Anspruch 3, worin

- X und X' 0 sind,
- $R^1$ t-Butyl, Neopentyl(2,2-dimethylpropyl) oder 1,2,2,-Trimethylpropyl ist,
- $R^2$ Methyl, Methoxymethyl, $(C_2-C_4)$Alkenyl, $(C_2-C_5)$Alkynyl, Benzyl oder 4-Halogenbenzyl ist, und
- A und B gleich oder verschieden voneinander Phenyl oder ein substituiertes Phenyl sind, wo die Substituenten ein bis drei der gleichen oder verschiedenen von Halogen, Nitro, $(C_1-C_4)$-Alkyl, $(C_1-C_4)$Alkoxy oder Halogen$(C_1-C_4)$alkyl sein können.

**5.** Verwendung nach Anspruch 4, worin

- $R^1$ t-Butyl ist,
- $R^2$ $(C_2-C_5)$Alkynyl ist, und
- A und B ein unsubstituiertes oder substituiertes Phenyl sind, wo die Substituenten ein oder zwei der gleichen oder verschiedenen von Chlor, Fluor, Brom, Jod, Methyl, Ethyl, Methoxy oder Trifluormethyl sein können.

**6.** Verwendung nach Anspruch 5, worin

- X und X' 0 sind,
- $R^1$ t-Butyl ist, und
- $R^2$ 2-Propynyl ist, und (a)
- A 4-Methylphenyl ist, und

B 3-Methylphenyl ist, oder (b)
A Phenyl ist, und
B Phenyl, 2,4-Dichlorphenyl oder 3,4-Dichlorphenyl ist.

**7.** Verwendung einer Insektizid-wirksamen Verbindung zur Insektenkontrolle, bei der die Insekten mit einer Insektizid-wirksamen Menge einer Insektizid-wirksamen Verbindung und einem der Ansprüche 1 bis 6, gegebenenfalls in einem auch ein landwirtschaftlich verträgliches Verdünnungsmittel oder einen Träger enthaltenden Mittel in Kontakt gebracht werden.

**8.** Mechanisches Verfahren zur Steigerung des kommerziellen Werts und/oder Nutzens verkäuflicher Ernten von Pflanzen, deren Wachstum beeinträchtigt oder wahrscheinlich durch Insekten beeinträchtigt wird, bei dem ein Behälter, ein Räucherapparat oder eine mechanische Streuvorrichtung mit einer insektiziden Verbindung nach einem der Ansprüche 1 bis 6, gegebenenfalls in einem Gemisch mit einem landwirtschaftlich verträglichen Verdünnungsmittel oder einem Träger, beladen (1), der Behälter, der Räucherapparat oder die mechanische Streuvorrichtung zur Verabreichung der insektiziden Verbindung oder des Mittels in Form von Granulat, Staub, Rauch, Dampf oder einer flüßigen, ein Tensid enthaltenden Präparation an wachsende Pflanzen oder an ein Wachstumsmedium, in dem die Pflanzen wachsen sollen, oder an die Insekten selbst verwendet (2) und die Dosis des wirksamen Bestandteils während dieses Verfahrensschrittes kontrolliert werden, so daß die Verabreichungsrate der Insektizid-wirksamen Verbindung zur Kontrolle der Insekten ausreichend, nicht jedoch zur Verursachung eines nicht-annehmbaren nachteiligen Effekts auf die wachsenden Erntepflanzen oder auf die, die auf dem behandelten Bereich noch wachsen sollen, ausreichend ist.

**9.** Verwendung oder Verfahren nach einem der vorhergehenden Ansprüche, worin das Mittel 0,001 bis 90 Gew.-%, vorzugsweise 0,01 bis 75 Gew.-% der insektiziden Verbindungen enthält.

**10.** Verwendung oder Verfahren nach einem der vorhergehenden Ansprüche, worin das Mittel zusätzlich ein Emulgierungsmittel enthält (a), wobei das Mittel in Form eines emulgierbaren Konzentrates vorliegt, zusätzlich ein Dispersionsmittel enthält (b), wobei das Mittel in Form eines benetzbaren Pulvers vorliegt, einen flüssigen landwirtschaftlich verträglichen Träger und ein Dispersionsmittel enthält (c), wobei das Mittel in fließbarer Form vorliegt, in Form von Staub oder Granulat vorliegt (d) oder zusätzlich einen Lockstoff enthält (e), wobei das Mittel in Form eines Köders vorliegt.

**11.** Verwendunmg oder Verfahren nach einem der Ansprüche 7 bis 10, worin die Insektizid- wirksame Verbindung wachsenden Pflanzen oder einem Bereich, wo Pflanzen wachsen sollen, mit einer Rate von 10 g bis etwa 10 kg der wirksamen Verbindung pro Hektar, vorzugsweise 100 g bis 5 kg pro Hektar verabreicht wird.

**12.** Verwendung oder Verfahren nach einem der Ansprüche 7 bis 10 zur Kontrolle von Insekten der Ordnung Lepidoptera oder Coleoptera.